# EUROPEAN PATENT SPECIFICATION

(11) **EP 2 203 565 B1**
(45) Date of publication and mention of the grant of the patent: **26.08.2015**
(21) Application number: 08836084.7
(22) Date of filing: 03.10.2008
(51) Int. Cl.: C12N 9/88, C12N 15/82

(54) **MUTATED ACETOHYDROXYACID SYNTHASE GENES IN BRASSICA**
MUTIERTE ACETOHYDROXYSÄURE-SYNTHASE-GENE IN BRASSICA
GÈNES D'ACÉTOHYDROXYACIDE SYNTHASE MUTÉS CHEZ BRASSICA

(30) Priority: 05.10.2007 US 977944 P
(43) Date of publication of application: 07.07.2010
(60) Divisional application: 13175389.9
(73) Proprietor: Cibus Europe B.V., 4421 Ad Kapelle (NL)
(72) Inventor: SCHOPKE, Christian, San Diego California 92130 (US); GOCAL, Greg F. W., San Diego California 92129 (US); WALKER, Keith, San Diego California 92130 (US); BEETHAM, Peter R., Carlsbad California 92009 (US)
(74) Representative: Zimmermann & Partner Patentanwälte mbB
(86) International application number: PCT/US2008/078798
(87) International publication number: WO 2009/046334

(56) References cited:
- WO-A1-2005/020673
- WO-A1-2006/024351
- WO-A1-2008/071715
- WO-A2-01/85970
- WO-A2-2006/060634
- WO-A2-2007/005581
- WO-A2-2008/124495
- US-A- 5 795 972
- US-A1- 2003 097 692
- US-A1- 2007 118 920
- US-B1- 6 576 455
- US-B1- 6 870 075
- JOSE MARIA BRUNIARD: "Inheritance of imidazolinone resistance, characterization of cross-resistance pattern, and identification of molecular markers in sunflower (Helianthus annuus L.)" DISSERTATION,, 1 September 2001 (2001-09-01), page 89PP, XP009138420
- KOLKMAN J M ET AL: "Acetohydroxyacid synthase mutations conferring resistance to imidazolinone or sulfonylurea herbicides in sunflower" THEORETICAL AND APPLIED GENETICS, SPRINGER, BERLIN, DE LNKD- DOI:10.1007/S00122-004-1716-7, vol. 109, no. 6, 1 October 2004 (2004-10-01), pages 1147-1159, XP002417954 ISSN: 0040-5752
- WHITE A D ET AL: "ISOLATION OF ACETOLACTATET SYNTHASE HOMOLOGS IN COMMON SUNFLOWERS" WEED SCIENCE, WEED SCIENCE SOCIETY OF AMERICA, CHAMPAIGN, IL, US, vol. 51, no. 6, 1 November 2003 (2003-11-01), pages 845-853, XP009058786 ISSN: 0043-1745

## Description

### FIELD OF THE INVENTION

This invention relates to the field of herbicide resistant plants and seeds and more specifically to mutations in the acetohydroxyacid synthase (AHAS) gene and protein.

### BACKGROUND OF THE INVENTION

The following description is provided simply as an aid in understanding the invention and is not admitted to describe or constitute prior art to the invention.

Benefits of herbicide-tolerant plants are known. For example, herbicide-tolerant plants may reduce the need for tillage to control weeds thereby effectively reducing soil erosion.

The introduction of exogenous mutant genes into plants is well documented. For example, U.S. Pat. No. 4,545,060 relates to increasing a plant's resistance to glyphosate by introducing into the plant's genome a gene coding for an EPSPS variant having at least one mutation that renders the enzyme more resistant to its competitive inhibitor, i.e., glyphosate.

Examples of some of the mutations in AHAS genes are known. *See e.g.* U.S. Patent No. 7,094,606; US 2003/0097692 A1; "Inheritance of Imidazolinone Resistance, Characterization of cross-resistance pattern, and Identification of Molecular Markers in Sunflower" by Josè Maria Bruniard, doctoral thesis at the North Dakota State University; US 2007/0118920 A1; "Acetohydroxyacid synthase mutations conferring resistance to imidazolinone or sulfonylurea herbicides in sunflower" by Judith Kolkman et al. (Theor. Appl. Genet (2004) 109: 1147-1159); "Isolation of acetolactate synthase homologs in common sunflower" by Anthony White et al. (Weed Science, 51:845-853 (2003)); WO 2008/071715 A1; and WO 2008/124495 A2.

Through chemical mutagenesis, a mutation was found in the lower expressed AHAS I gene. This mutation is known as PM-1 (a mutation at an equivalent position known as 653 based on the acetolactate synthase (ALS) amino acid sequence of *Arabidopsis,* a serine to asparagine amino acid change, respectively encoded as follows - AGT to AAT). Another mutation was found in the higher expressed gene AHAS III, known as PM-2 (a mutation at an equivalent position known as 574, a tryptophan to leucine amino acid change, respectively encoded as follows - TGG to TTG). These two mutations, PM-1 and PM-2, are combined in a commercial variety of Canola known as Clearfield Canola (Tan *et al.,* 2005).

### SUMMARY OF THE INVENTION

The disclosure relates in part to mutated acetohydroxyacid synthase (AHAS) nucleic acids and the proteins encoded by the mutated nucleic acids. The disclosure also relates in part to canola plants, cells, and seeds comprising these mutated nucleic acids and proteins.

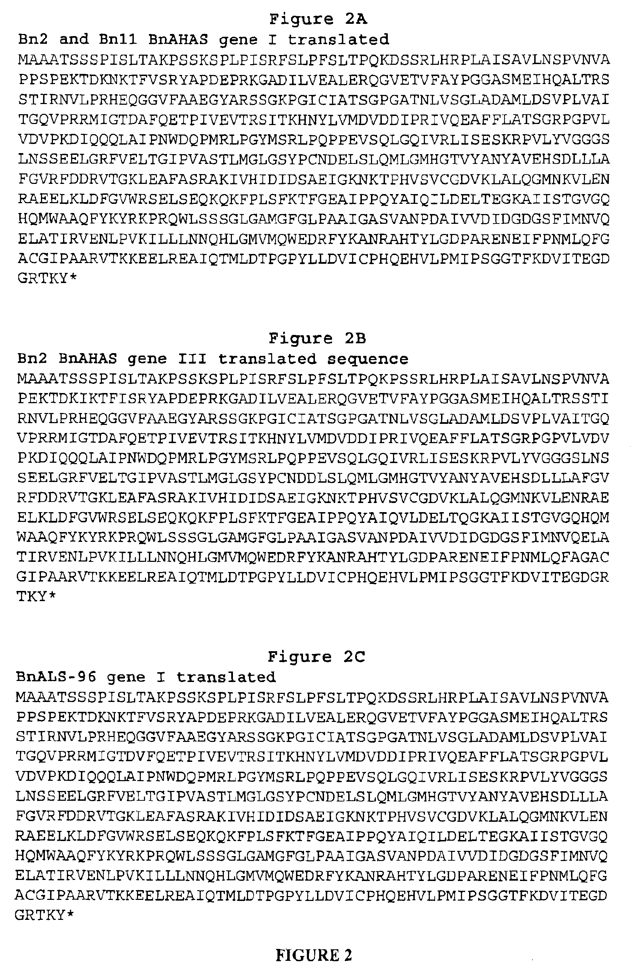

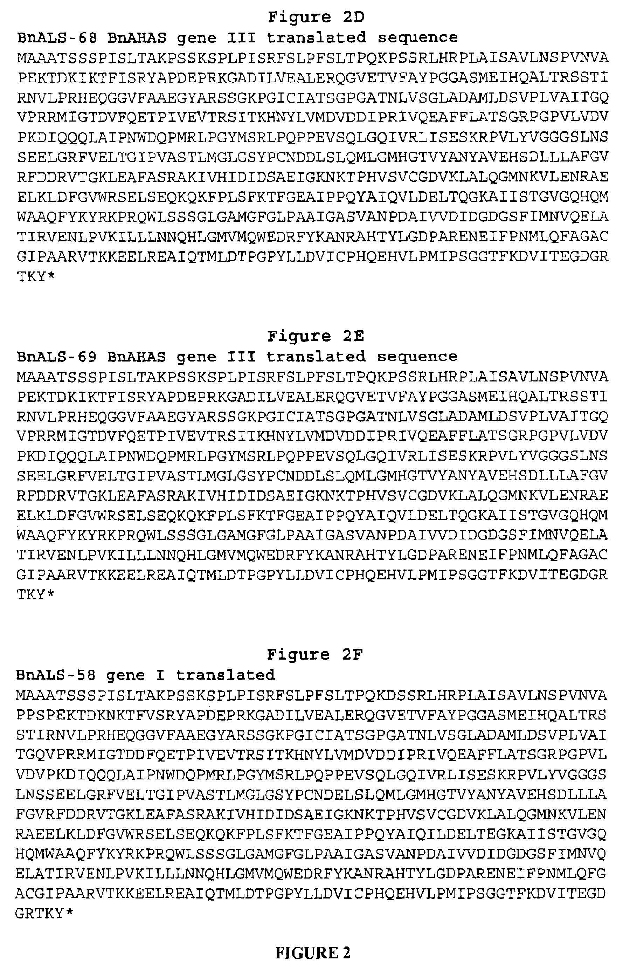

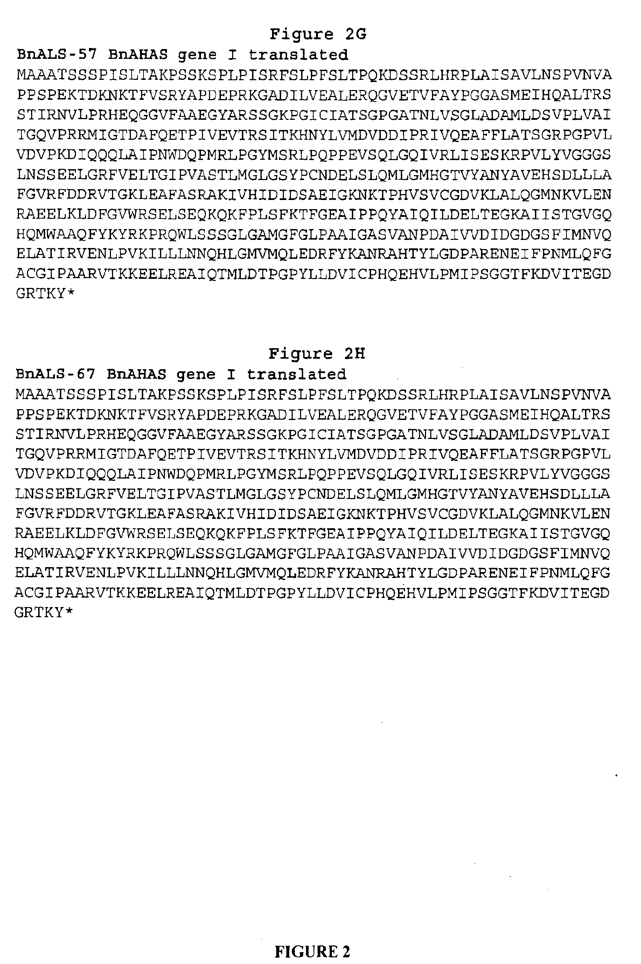

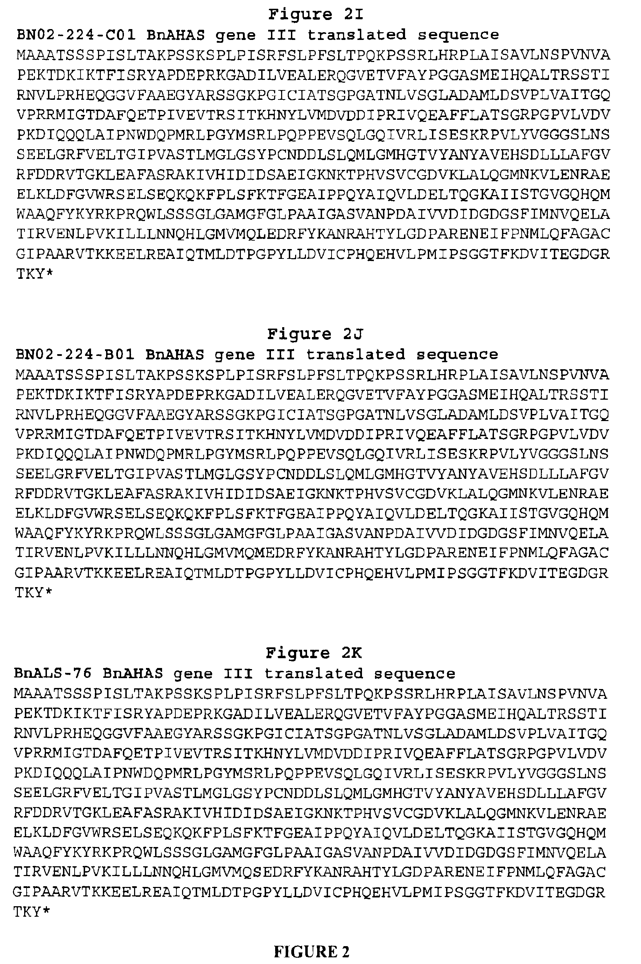

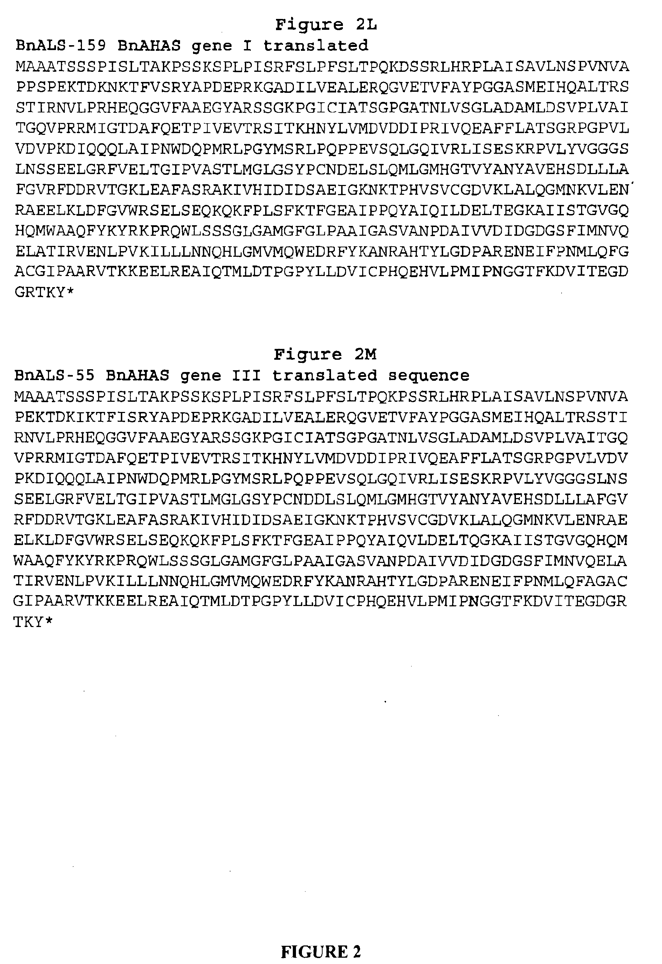

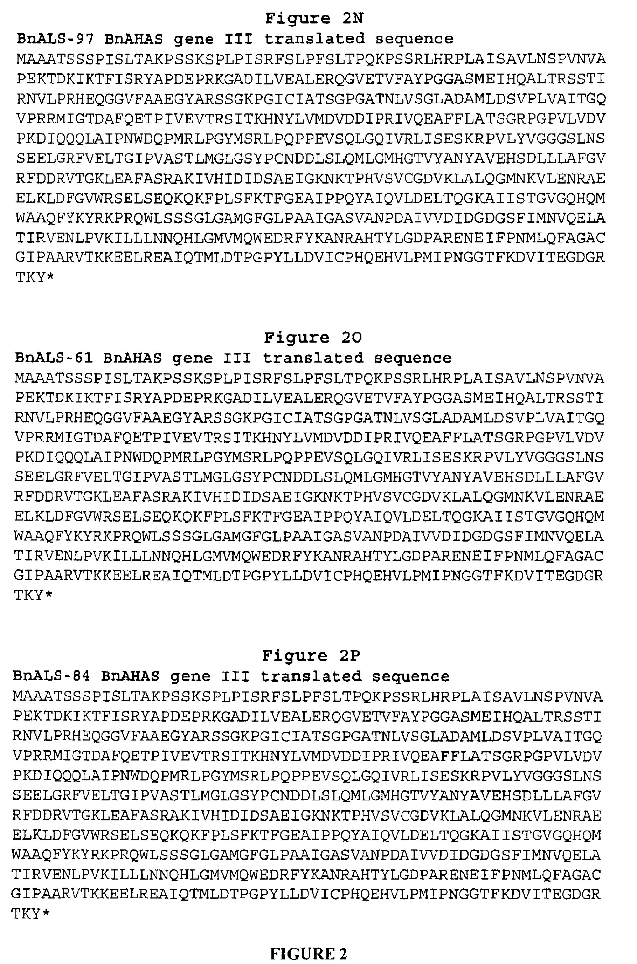

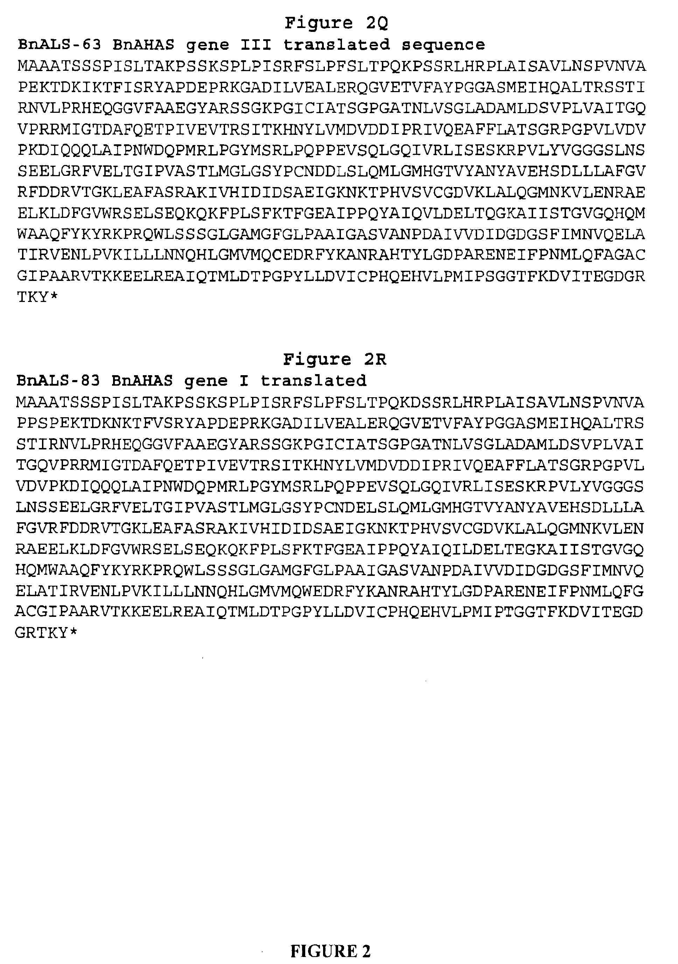

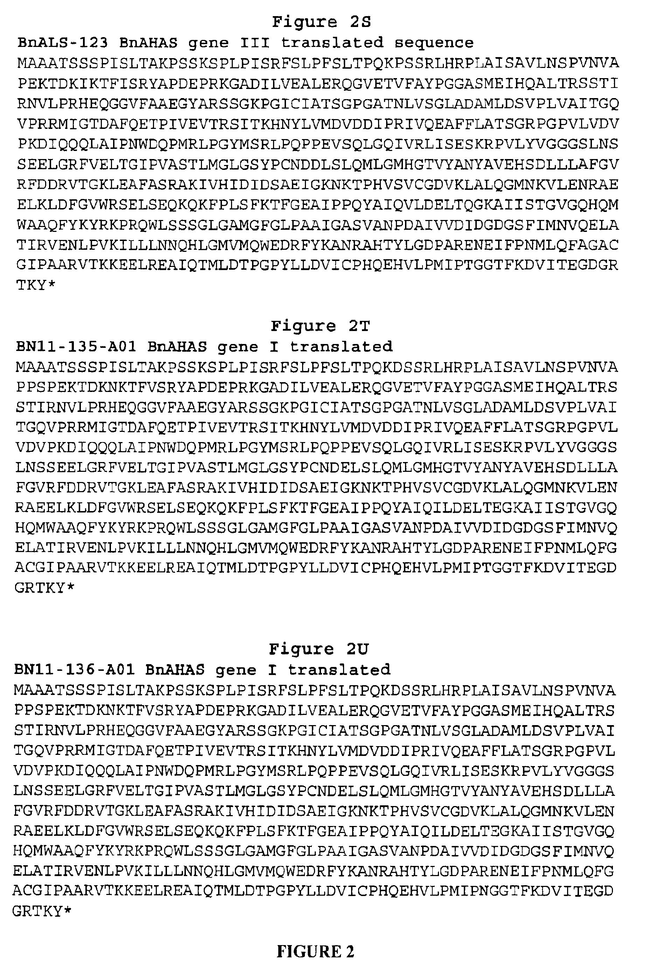

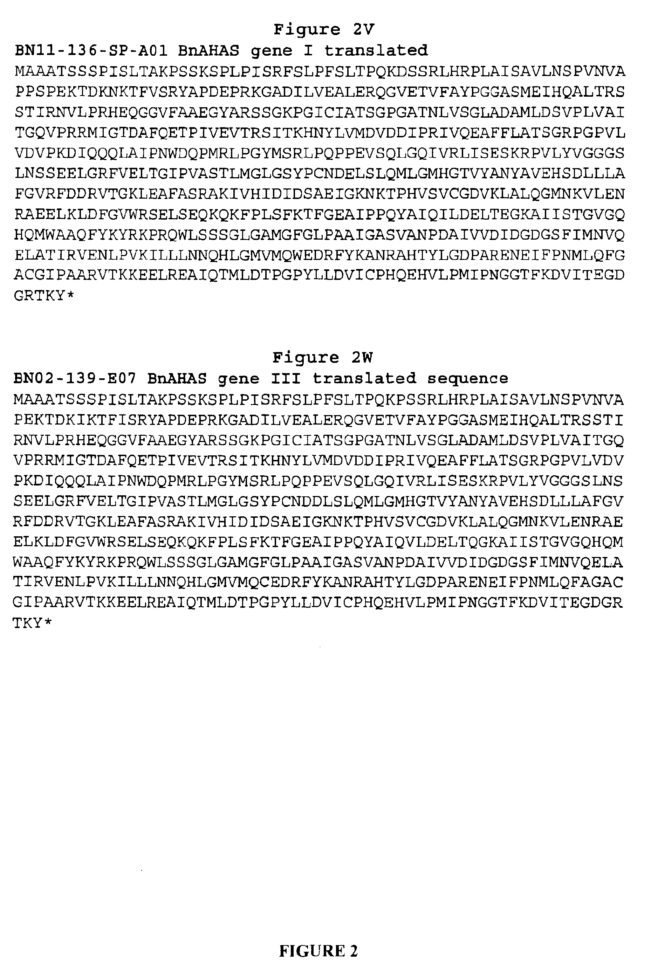

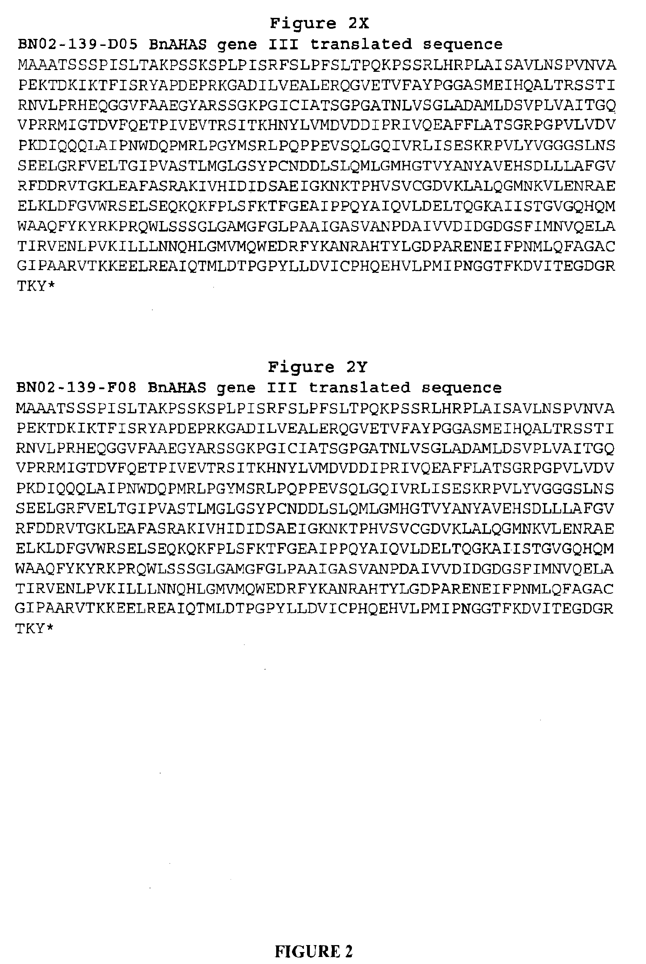

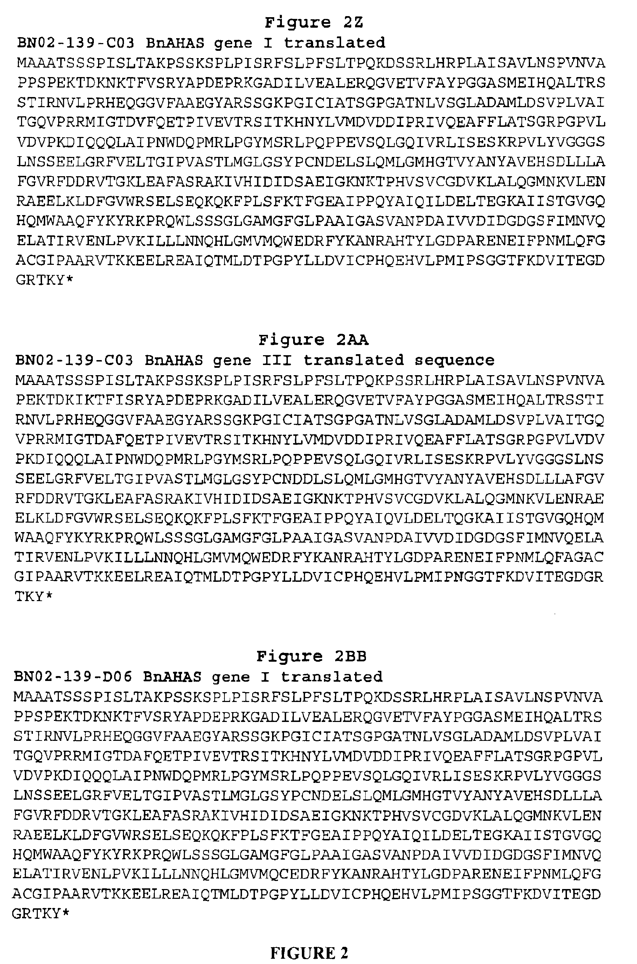

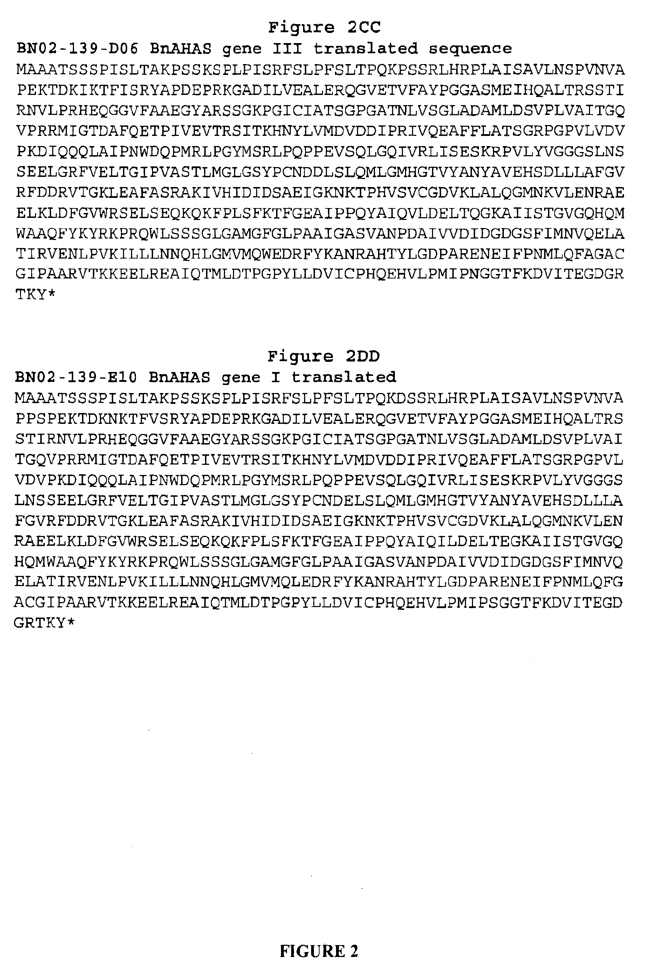

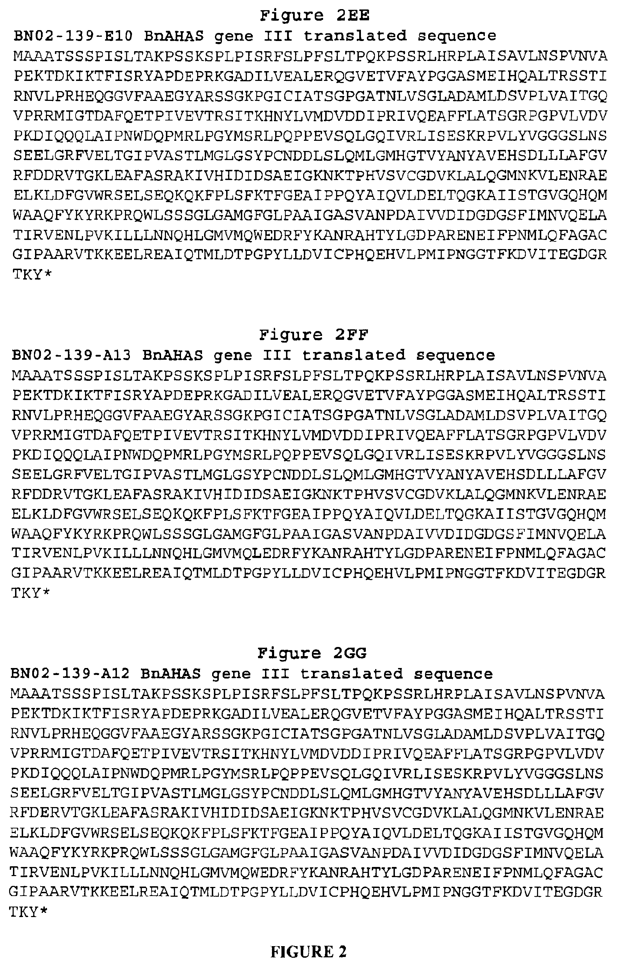

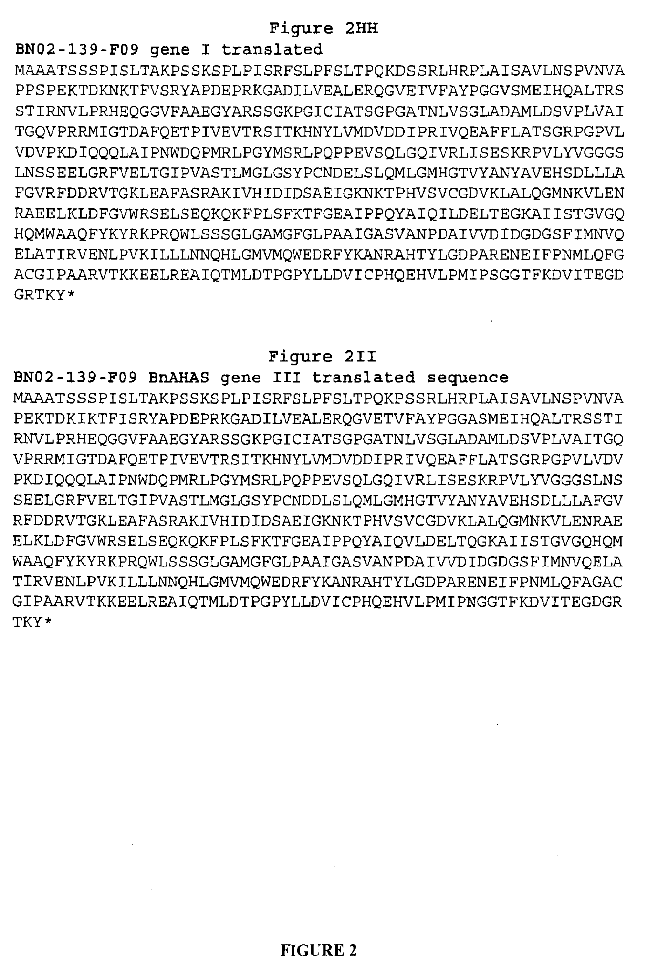

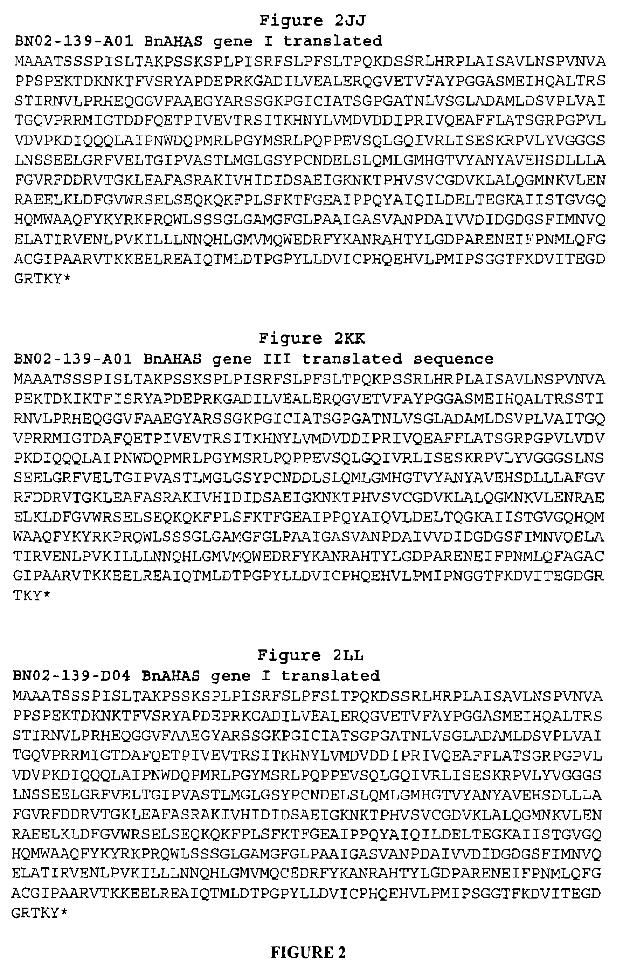

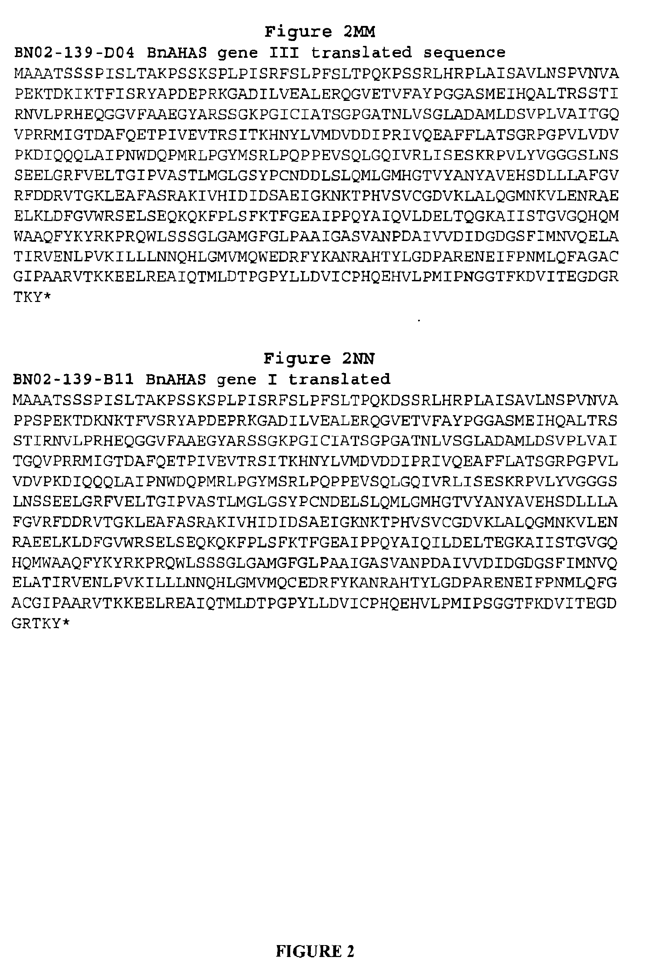

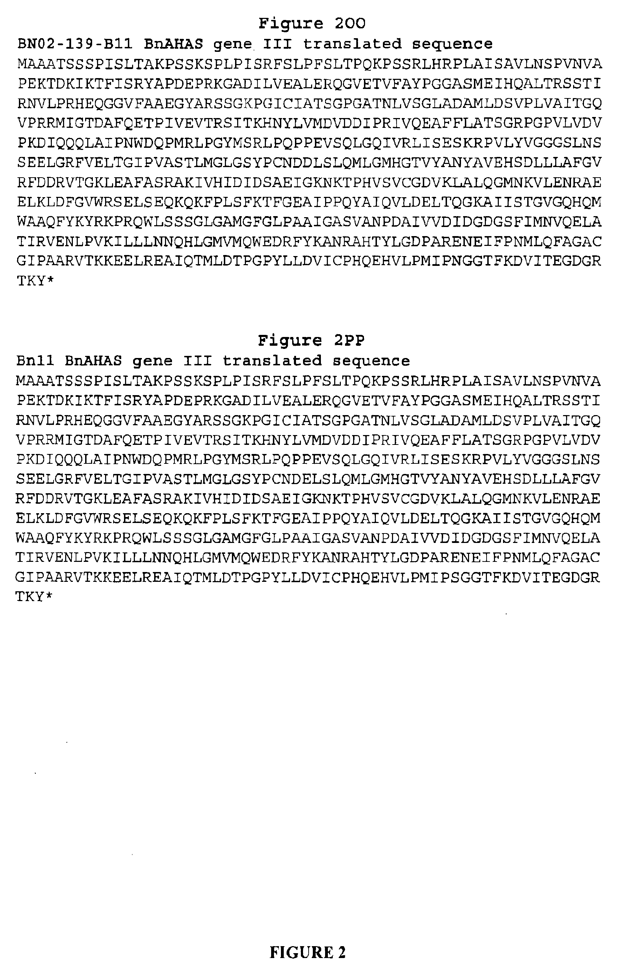

Described herein is an isolated nucleic acid encoding a *Brassica* acetohydroxyacid synthase protein having a mutation at one or more amino acid positions corresponding to a position selected from the group consisting of: A205, D376, W574, R577, and S653 of SEQ ID NO: 1. In some examples, the isolated nucleic acid encodes a protein having one or more mutations selected from the group consisting of: an alanine to valine substitution at a position corresponding to position 205 of SEQ ID NO: 1, an alanine to aspartic acid substitution at a position corresponding to position 205 of SEQ ID NO: 1, an aspartic acid to glutamic acid substitution at a position corresponding to position 376 of SEQ ID NO: 1, a tryptophan to cysteine substitution at a position corresponding to position 574 of SEQ ID NO: 1, a tryptophan to leucine substitution at a position corresponding to position 574 of SEQ ID NO: I, a tryptophan to methionine substitution at a position corresponding to position 574 of SEQ ID NO: 1, a tryptophan to serine substitution at a position corresponding to position 574 of SEQ ID NO: 1, an arginine to tryptophan substitution at a position corresponding to position 577 of SEQ ID NO: 1, a serine to asparagine at a position corresponding to position 653 of SEQ ID NO: 1, and a serine to threonine at a position corresponding to position 653 of SEQ ID NO: 1. In some examples, the mutation is a serine to asparagine substitution at a position corresponding to position 653 of SEQ ID NO: 1 in which the mutation is not S653N in *Brassica* AHAS I gene. 4. In some examples, the mutation is a serine to threonine substitution at a position corresponding to position 653 of SEQ ID NO: 1. In some examples, the mutation is a tryptophan to leucine substitution at a position corresponding to position 574 of SEQ ID NO: 1, in which the mutation is not W574L in *Brassica* AHAS III gene. In some examples, the mutation is an alanine to valine substitution at a position corresponding to position 205 of SEQ ID NO: 1. In other examples the mutation is an alanine to aspartic acid substitution at a position corresponding to position 205 of SEQ ID NO: 1. In other examples, the isolated nucleic acid encodes a protein having a mutation selected from the mutations shown in Table 2. In certain examples, the isolated nucleic acid encodes a protein having two or more mutations. In some examples, the two or more mutations are selected from Table 2. In some examples, the isolated nucleic acid encodes a protein having a mutation at a position corresponding to S653 of SEQ ID NO: 1 and a mutation at one or more amino acid positions corresponding to a position selected from the group consisting of: A205, D376, W574, and R577 of SEQ ID NO: 1. In other examples, the isolated nucleic acid encodes a protein having a mutation at a position corresponding to W574 of SEQ ID NO: 1 and a mutation at a position corresponding to R577 of SEQ ID NO: 1. In some examples, the isolated nucleic acid encodes an acetohydroxyacid synthase (AHAS) protein that is resistant to inhibition by an AHAS-inhibiting herbicide. In some examples, the AHAS-inhibiting herbicide is selected from the group consisting of herbicides of: imidazolinone, sulfonylurea, triazolopyrimidine, pyrimidinylthiobenzoate, sulfonylamino-carbonyltriazolinone, and mixtures thereof. In some examples, the herbicide is an imidazolinone herbicide. In some examples, the herbicide is a sulfonylurea herbicide. In some examples the isolated nucleic acid encodes an AHAS protein comprising 70% or more identity to one or more of the amino acid sequences in Figure 2. In some examples, the isolated nucleic acid encodes a *Brassica napus* AHAS protein. In other examples, the isolated nucleic acid encodes a *Brassica napus* AHAS I protein. In certain examples, the isolated nucleic acid encodes a *Brassica napus* AHAS III protein.

Also disclosed herein is an expression vector containing an isolated nucleic acid encoding a *Brassica* acetohydroxyacid synthase protein having a mutation at one or more amino acid positions corresponding to a position selected from the group consisting of: A205, D376, W574, R577, and S653 of SEQ ID NO: 1. In some examples, the expression vector contains an isolated nucleic acid encoding a protein having one or more mutations selected from the group consisting of: an alanine to valine substitution at a position corresponding to position 205 of SEQ ID NO: 1, an alanine to aspartic acid substitution at a position corresponding to position 205 of SEQ ID NO: 1, an aspartic acid to glutamic acid substitution at a position corresponding to position 376 of SEQ ID NO: 1, a tryptophan to cysteine substitution at a position corresponding to position 574 of SEQ ID NO: 1, a tryptophan to leucine substitution at a position corresponding to position 574 of SEQ ID NO: 1, a tryptophan to methionine substitution at a position corresponding to position 574 of SEQ ID NO: 1, a tryptophan to serine substitution at a position corresponding to position 574 of SEQ ID NO: 1, an arginine to tryptophan substitution at a position corresponding to position 577 of SEQ ID NO: 1, a serine to asparagine at a position corresponding to position 653 of SEQ ID NO: 1, and a serine to threonine at a position corresponding to position 653 of SEQ ID NO: 1. In some examples, the mutation is a serine to asparagine substitution at a position corresponding to position 653 of SEQ ID NO: 1 in which the mutation is not S653N in *Brassica* AHAS I gene. 4. In some examples, the mutation is a serine to threonine substitution at a position corresponding to position 653 of SEQ ID NO: 1. In some examples, the mutation is a tryptophan to leucine substitution at a position corresponding to position 574 of SEQ ID NO: 1, in which the mutation is not W574L in *Brassica* AHAS III gene. In some examples, the mutation is an alanine to valine substitution at a position corresponding to position 205 of SEQ ID NO: 1. In other examples the mutation is an alanine to aspartic acid substitution at a position corresponding to position 205 of SEQ ID NO: 1.

Further disclosed herein is a plant having a *Brassica* acetohydroxyacid synthase (AHAS) gene, in which the gene encodes a protein having a mutation at one or more amino acid positions corresponding to a position selected from the group consisting of: A205, D376, W574, R577, and S653 of SEQ ID NO: 1. Further disclosed is a plant having a *Brassica* acetohydroxyacid synthase (AHAS) gene, in which the plant is resistant to an AHAS-inhibiting herbicide, in which the gene encodes a protein having a mutation at one or more amino acid positions corresponding to a position selected from the group consisting of: A205, D376, W574, R577, and S653 of SEQ ID NO: 1. In some examples of the above aspects, the plant has an AHAS gene which encodes a protein having one or more mutations selected from the group consisting of: an alanine to valine substitution at a position corresponding to position 205 of SEQ ID NO: 1, an alanine to aspartic acid substitution at a position corresponding to position 205 of SEQ ID NO: 1, an aspartic acid to glutamic acid substitution at a position corresponding to position 376 of SEQ ID NO: 1, a tryptophan to cysteine substitution at a position corresponding to position 574 of SEQ ID NO: 1, a tryptophan to leucine substitution at a position corresponding to position 574 of SEQ ID NO: 1, a tryptophan to methionine substitution at a position corresponding to position 574 of SEQ ID NO: 1, a tryptophan to serine substitution at a position corresponding to position 574 of SEQ ID NO: 1, an arginine to tryptophan substitution at a position corresponding to position 577 of SEQ ID NO: 1, a serine to asparagine at a position corresponding to position 653 of SEQ ID NO: 1, and a serine to threonine at a position corresponding to position 653 of SEQ ID NO: 1. In some examples, the mutation is a serine to asparagine substitution at a position corresponding to position 653 of SEQ ID NO: 1 in which the mutation is not S653N in *Brassica* AHAS I gene. In some examples, the mutation is a serine to threonine substitution at a position corresponding to position 653 of SEQ ID NO: 1. In some examples, the mutation is a tryptophan to leucine substitution at a position corresponding to position 574 of SEQ ID NO: 1, in which the mutation is not W574L in *Brassica* AHAS III gene. In some examples, the mutation is an alanine to valine substitution at a position corresponding to position 205 of SEQ ID NO: 1. In other examples the mutation is an alanine to aspartic acid substitution at a position corresponding to position 205 of SEQ ID NO: 1. In other examples, the mutation selected from the mutations shown in Table 2. In certain examples, the plant has an AHAS gene which encodes a protein having two or more mutations. In some examples, the two or more mutations are selected from Table 2. In some examples, the plant has an AHAS gene which encodes a protein having a mutation at a position corresponding to S653 of SEQ ID NO: 1 and a mutation at one or more amino acid positions corresponding to a position selected from the group consisting of: A205, D376, W574, and R577 of SEQ ID NO: 1. In other examples, the plant has an AHAS gene which encodes a protein having a mutation at a position corresponding to W574 of SEQ ID NO: 1 and a mutation at a position corresponding to R577 of SEQ ID NO: 1. In some examples, the plant has an AHAS gene which encodes a protein that is resistant to inhibition by an AHAS-inhibiting herbicide. In some examples the plant has an AHAS gene which encodes a protein comprising 70% or more identity to one or more of the amino acid sequences in Figure 2. In some examples, the plant is resistant to the application of at least one AHAS-inhibiting herbicide. In some examples, the AHAS-inhibiting herbicide is selected from the group consisting of herbicides of: imidazolinone, sulfonylurea, triazolopyrimidine, pyrimidinylthiobenzoate, sulfonylamino-carbonyltriazolinone, and mixtures thereof. In some embodiments, the herbicide is an imidazolinone herbicide. In some examples, the herbicide is a sulfonylurea herbicide. In some examples, the plant is a *Brassica* plant produced by growing a seed of a line selected from the lines listed in Table 2. In some examples, the plant is a *Brassica* species. In other examples, the plant is *Brassica napus.* In some examples, the plant is selected from Spring Oilseed Rape and Winter Oilseed Rape. In some examples, the plant has an AHAS gene which encodes a *Brassica napus* AHAS protein. In other examples, the plant has an AHAS gene which encodes a *Brassica napus* AHAS I protein. In certain examples, the plant has an AHAS gene which encodes a *Brassica napus* AHAS III protein. In some examples, the plant is non-transgenic.

Further disclosed herein is a seed having a *Brassica* acetohydroxyacid synthase (AHAS) gene encoding a protein having a mutation at one or more amino acid positions corresponding to a position selected from the group consisting of: A205, D376, W574, R577, and S653 of SEQ ID NO: 1. In some examples, the seed has an AHAS gene which encodes a protein having one or more mutations selected from the group consisting of: an alanine to valine substitution at a position corresponding to position 205 of SEQ ID NO: 1, an alanine to aspartic acid substitution at a position corresponding to position 205 of SEQ ID NO: I, an aspartic acid to glutamic acid substitution at a position corresponding to position 376 of SEQ ID NO: 1, a tryptophan to cysteine substitution at a position corresponding to position 574 of SEQ ID NO: 1, a tryptophan to leucine substitution at a position corresponding to position 574 of SEQ ID NO: 1, a tryptophan to methionine substitution at a position corresponding to position 574 of SEQ ID NO: 1, a tryptophan to serine substitution at a position corresponding to position 574 of SEQ ID NO: 1, an arginine to tryptophan substitution at a position corresponding to position 577 of SEQ ID NO: 1, a serine to asparagine at a position corresponding to position 653 of SEQ ID NO: 1, and a serine to threonine at a position corresponding to position 653 of SEQ ID NO: 1. In some examples, the mutation is a serine to asparagine substitution at a position corresponding to position 653 of SEQ ID NO: 1 in which the mutation is not S653N in *Brassica* AHAS I gene. 4. In some examples, the mutation is a serine to threonine substitution at a position corresponding to position 653 of SEQ ID NO: 1. In some examples, the mutation is a tryptophan to leucine substitution at a position corresponding to position 574 of SEQ ID NO: 1, in which the mutation is not W574L in *Brassica* AHAS III gene. In some examples, the mutation is an alanine to valine substitution at a position corresponding to position 205 of SEQ ID NO: 1. In other examples the mutation is an alanine to aspartic acid substitution at a position corresponding to position 205 of SEQ ID NO: 1. In other examples, the mutation selected from the mutations shown in Table 2. In certain examples, the seed has an AHAS gene which encodes a protein having two or more mutations. In some examples, the two or more mutations are selected from Table 2. In some examples, the seed has an AHAS gene which encodes a protein having a mutation at a position corresponding to S653 of SEQ ID NO: 1 and a mutation at one or more amino acid positions corresponding to a position selected from the group consisting of: A205, D376, W574, and R577 of SEQ ID NO: 1. In other examples, the seed has an AHAS gene which encodes a protein having a mutation at a position corresponding to W574 of SEQ ID NO: 1 and a mutation at a position corresponding to R577 of SEQ ID NO: 1. In some examples, the seed has an AHAS gene which encodes a protein that is resistant to inhibition by an AHAS-inhibiting herbicide. In some examples the seed has an AHAS gene which encodes a protein comprising 70% or more identity to one or more of the amino acid sequences in Figure 2. In some examples, the seed is resistant to the application of at least one AHAS-inhibiting herbicide. In some examples, the AHAS-inhibiting herbicide is selected from the group consisting of herbicides of: imidazolinone, sulfonylurea, triazolopyrimidine, pyrimidinylthiobenzoate, sulfonylamino-carbonyltriazolinone, and mixtures thereof. In some examples, the herbicide is an imidazolinone herbicide. In some examples, the herbicide is a sulfonylurea herbicide. In some examples, the seed is a *Brassica* seed. In some examples, the seed has an AHAS gene which encodes a *Brassica napus* AHAS protein. In other examples, the seed has an AHAS gene which encodes a *Brassica napus* AHAS I protein. In certain examples, the seed has an AHAS gene which encodes a *Brassica napus* AHAS III protein. In some examples, the seed is a seed of a *Brassica* plant line in which the line is selected from the lines listed in Table 2. In some examples, the seed is non-transgenic. In some examples, there is provided a seed produced by a plant of the methods disclosed herein. In other examples, the seed is a canola seed.

According to the present invention, there is provided a method for producing an herbicide-resistant plant as defined in claim 1.

Further disclosed is a method for increasing the herbicide-resistance of a plant by: (a) crossing a first *Brassica* plant to a second *Brassica* plant, in which the first plant comprises a *Brassica* acetohydroxyacid synthase (AHAS) gene, in which the gene encodes a protein having a mutation at one or more amino acid positions corresponding to a position selected from the group consisting of: A205, D376, W574, R577, and S653 of SEQ ID NO: 1; (b) screening a population resulting from the cross for increased AHAS herbicide-resistance; (c) selecting a member resulting from the cross having increased AHAS herbicide-resistance; and (d) producing seeds resulting from the cross. In some examples, a hybrid seed is produced by any of the above methods. In some examples, plants are grown from seeds produced by any of the above methods. Further disclosed is a method of controlling weeds in a field containing plants by applying an effective amount of at least one AHAS-inhibiting herbicide to a field containing said weeds and plants, the plant having a *Brassica* acetohydroxyacid synthase (AHAS) gene, in which the gene encodes a protein having a mutation at one or more amino acid positions corresponding to a position selected from the group consisting of: A205, D376, W574, R577, and S653 of SEQ ID NO: 1. 134. In some embodiments, the AHAS-inhibiting herbicide is selected from the group consisting of herbicides of: imidazolinone, sulfonylurea, triazolopyrimidine, pyrimidinylthiobenzoate, sulfonylamino-carbonyltriazolinone, and mixtures thereof. In other embodiments, the AHAS-inhibiting herbicide is an imidazolinone herbicide. In other embodiments, the AHAS-inhibiting herbicide is a sulfonylurea herbicide.

The term "nucleic acid" or "nucleic acid sequence" refers to an oligonucleotide, nucleotide or polynucleotide, and fragments or portions thereof, which may be single or double stranded, and represent the sense or antisense strand. A nucleic acid may include DNA or RNA, and may be of natural or synthetic origin. For example, a nucleic acid may include mRNA or cDNA. Nucleic acid may include nucleic acid that has been amplified (e.g., using polymerase chain reaction). The convention "NTwt###NTmut" is used to indicate a mutation that results in the wild-type nucleotide NTwt at position ### in the nucleic acid being replaced with mutant NTmut. The single letter code for nucleotides is as described in the U.S. Patent Office Manual of Patent Examining Procedure, section 2422, table 1. In this regard, the nucleotide designation "R" means purine such as guanine or adenine, "Y" means pyrimidine such as cytosine or thymine (uracil if RNA); "M" means adenine or cytosine; "K" means guanine or thymine; and "W" means adenine or thymine.

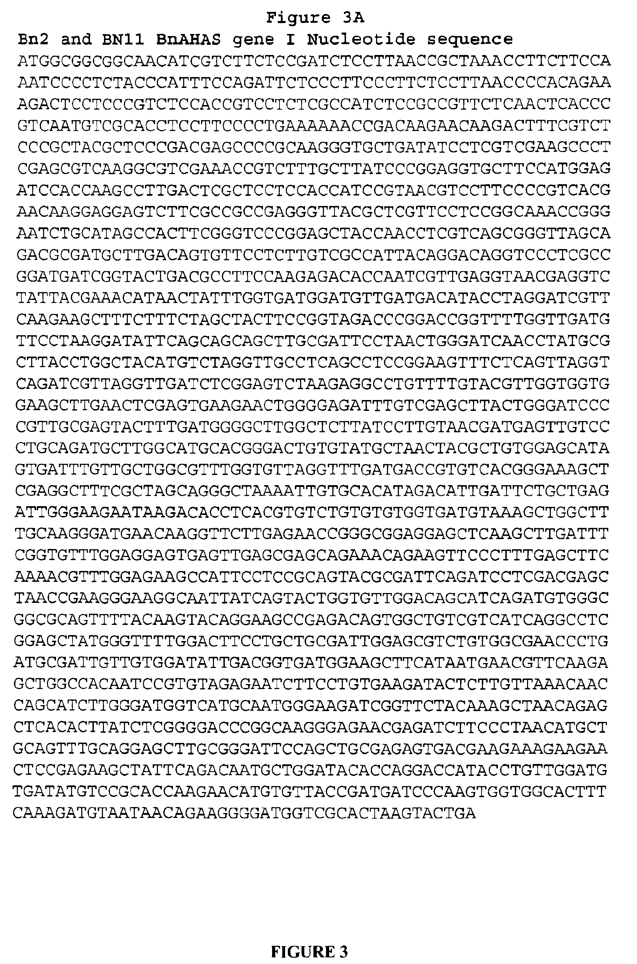

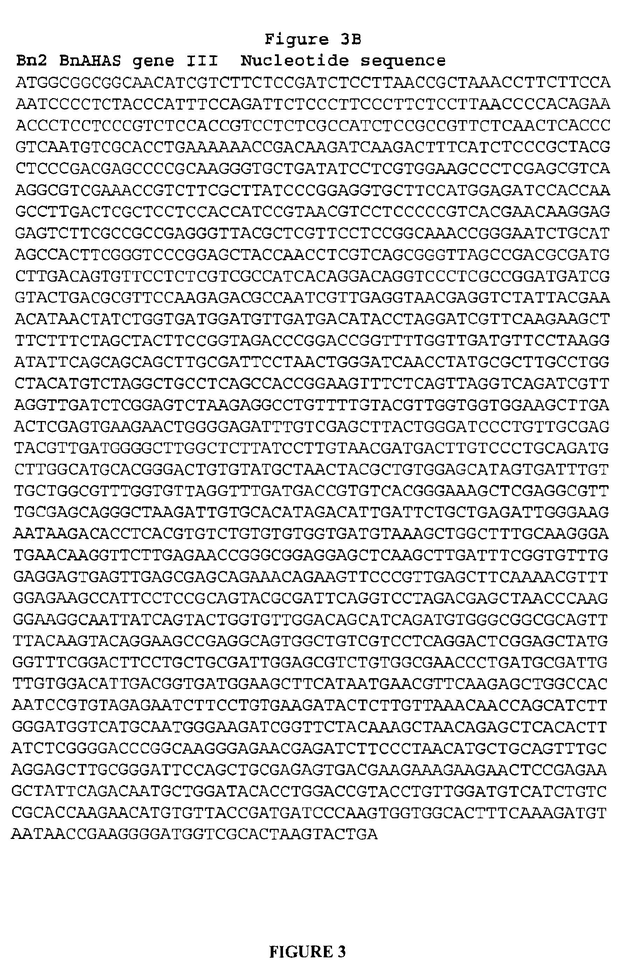

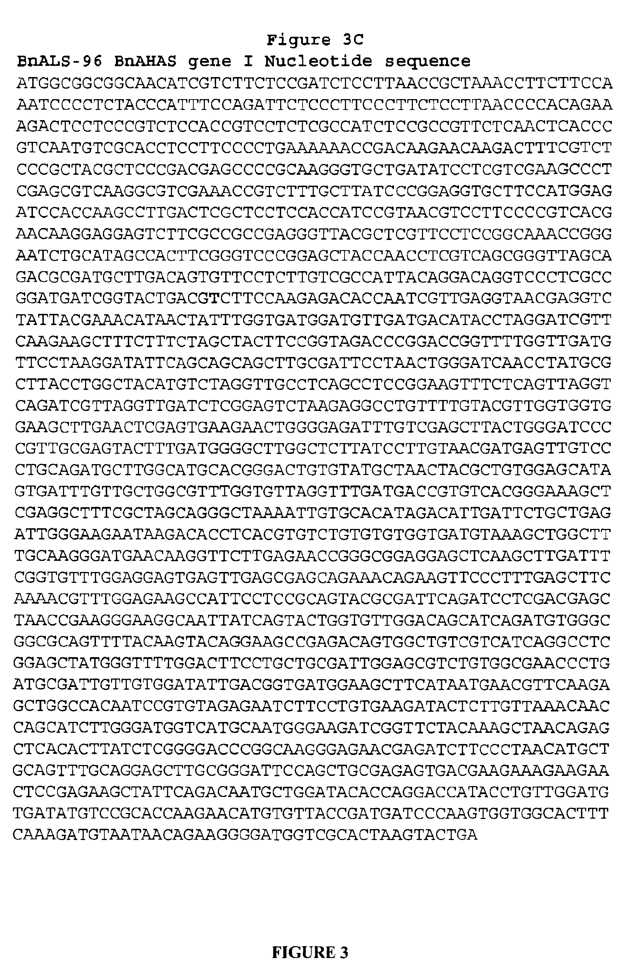

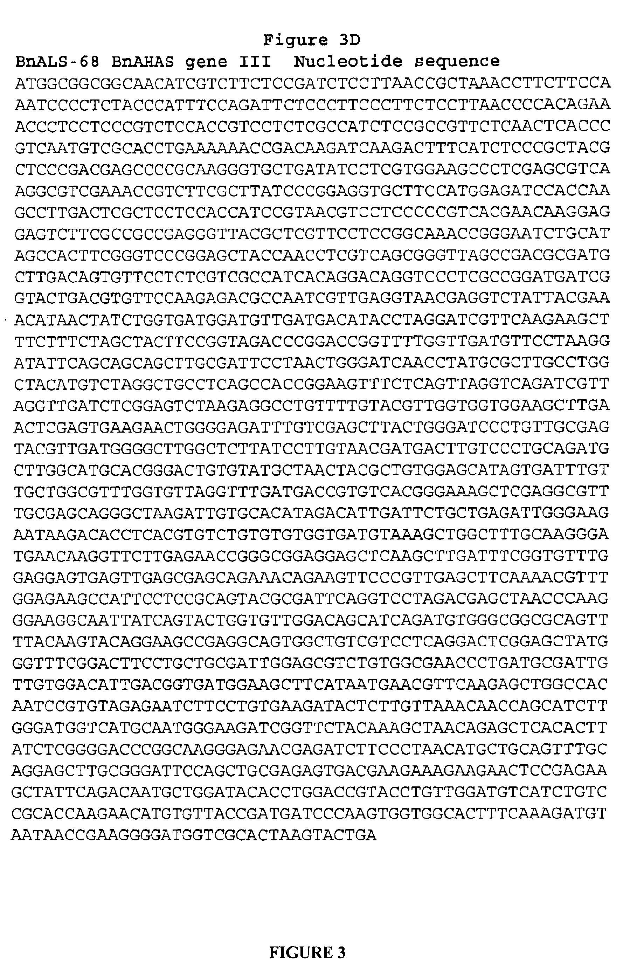

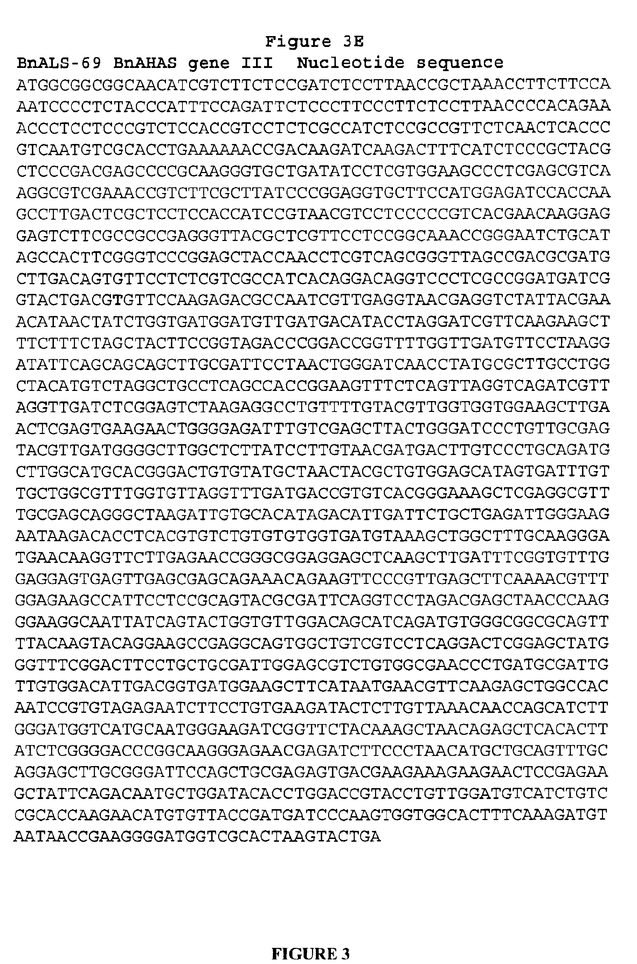

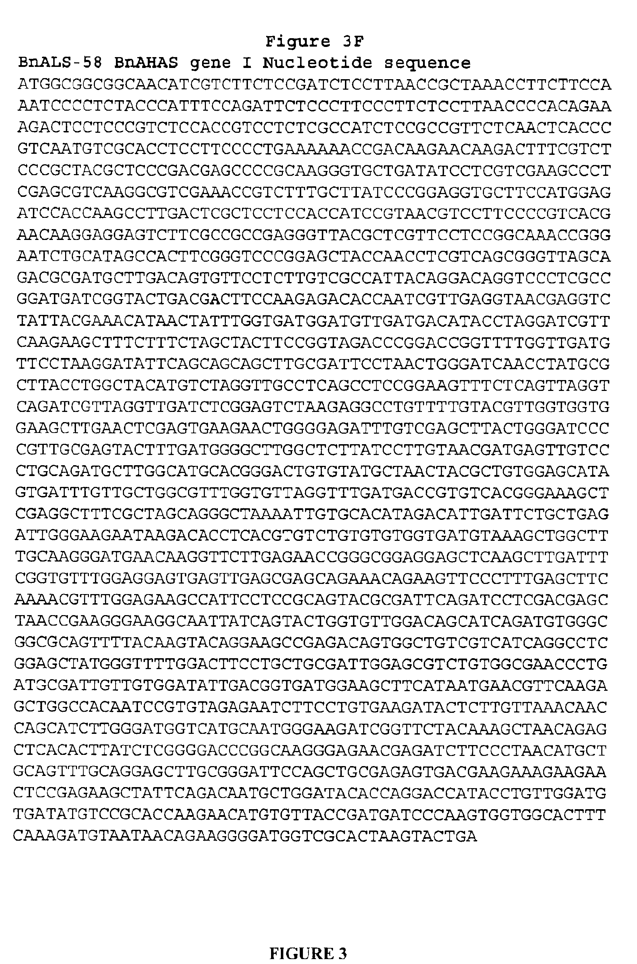

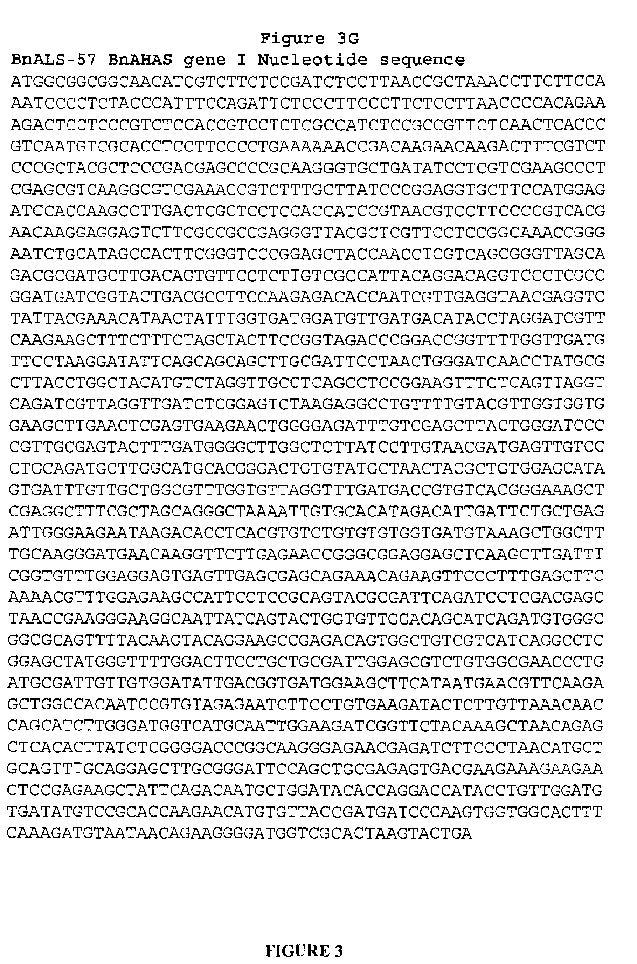

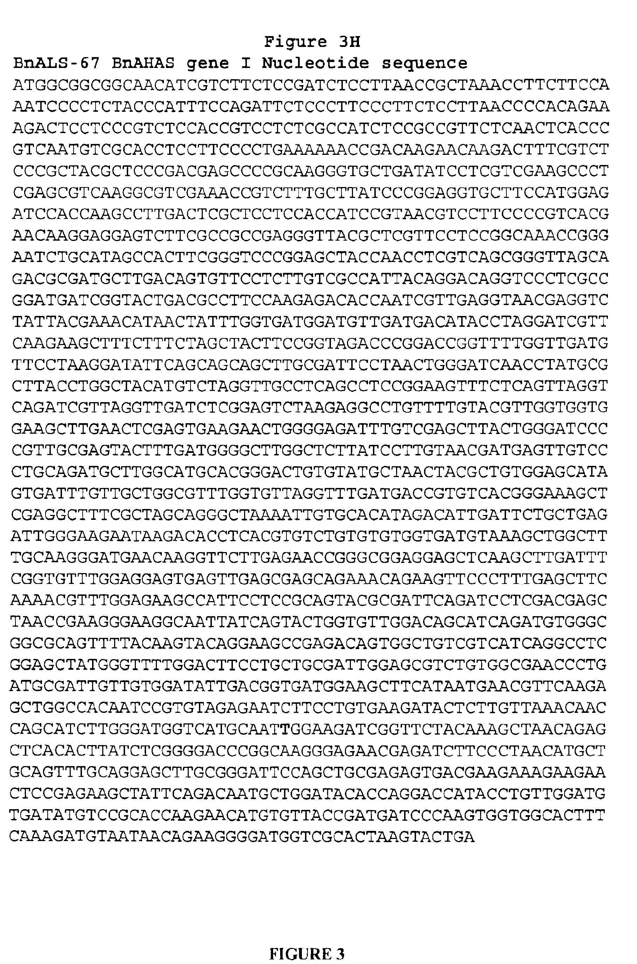

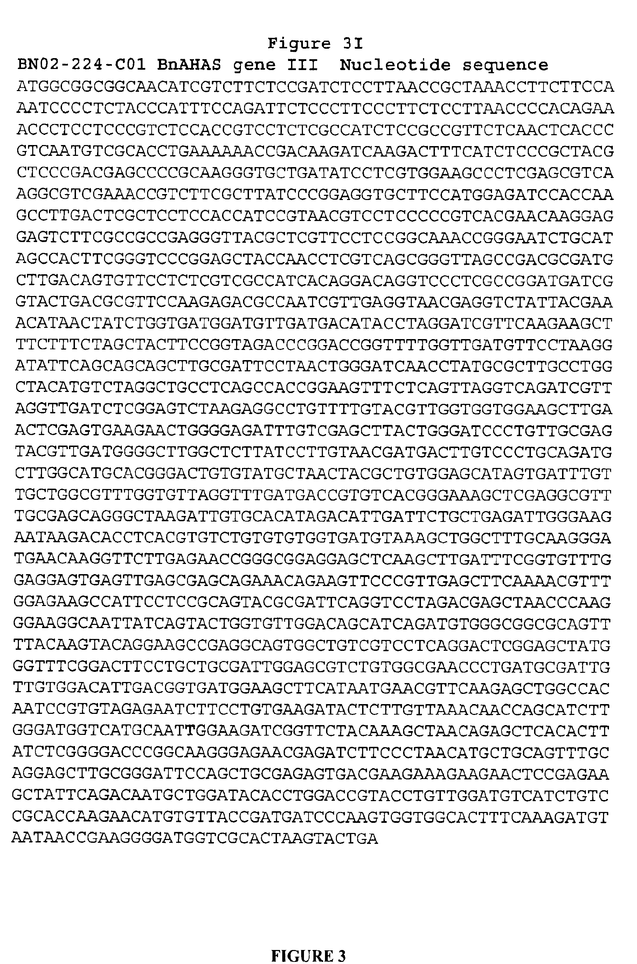

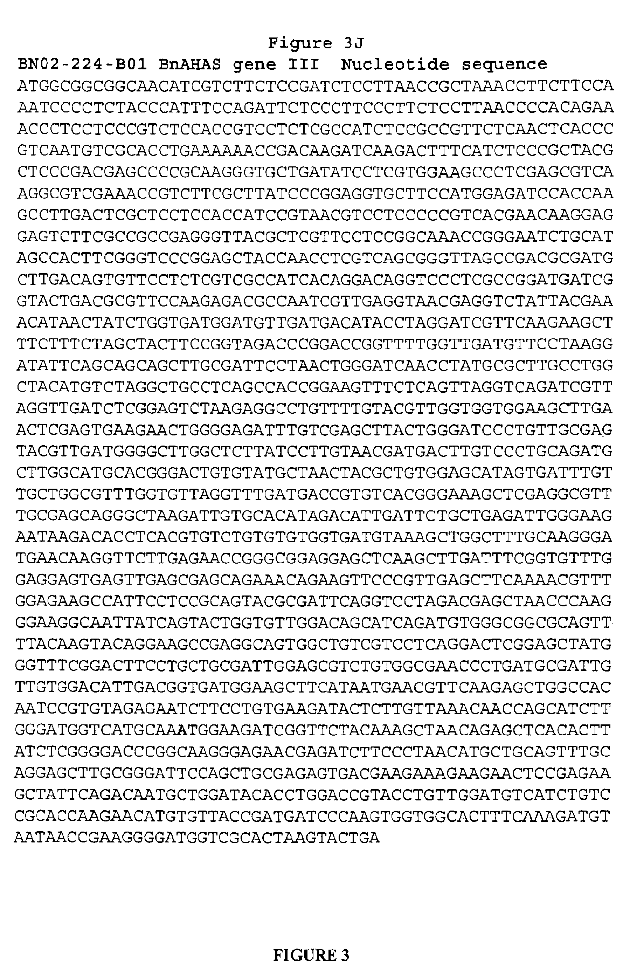

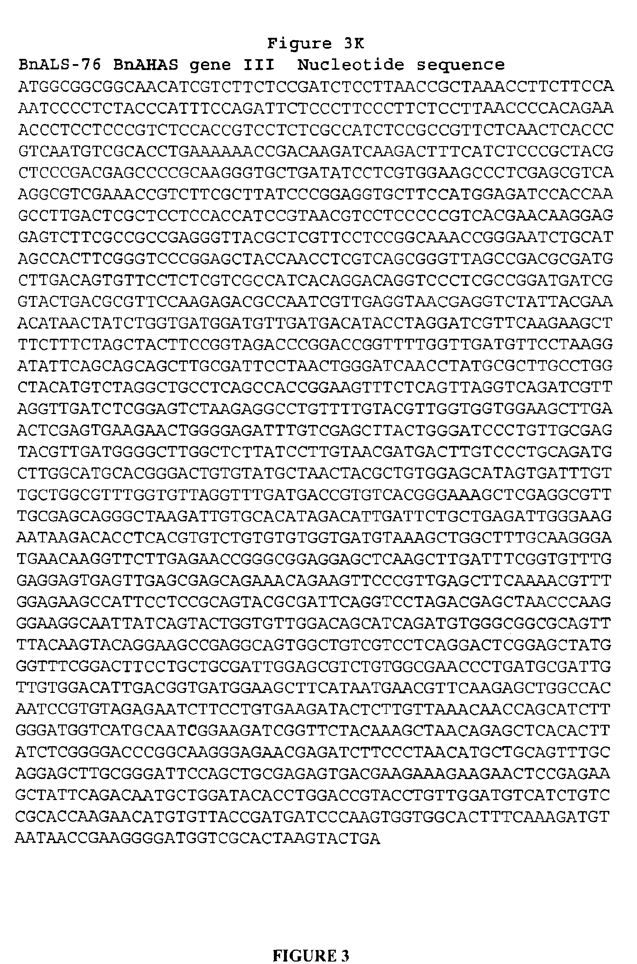

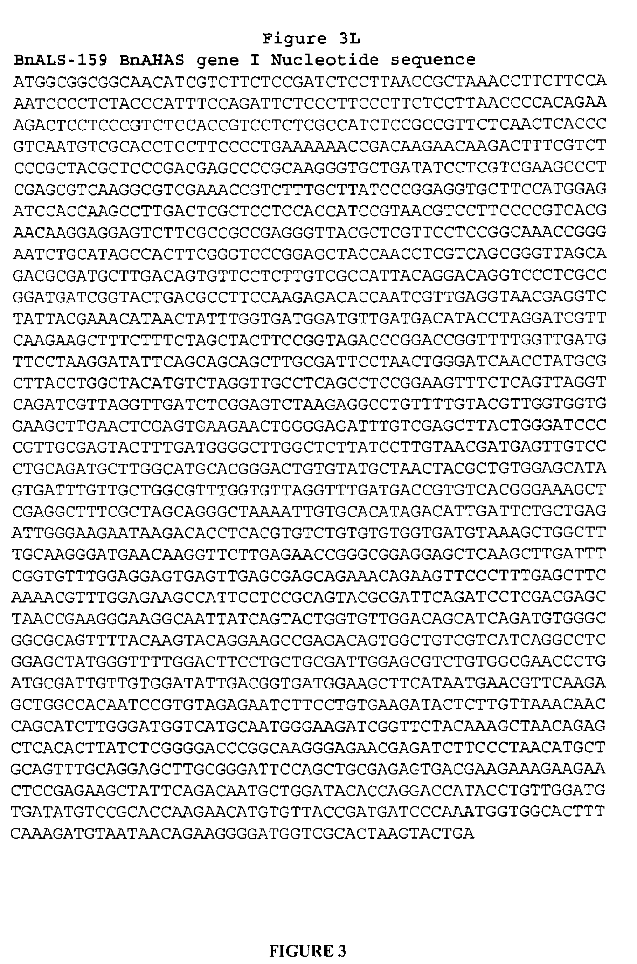

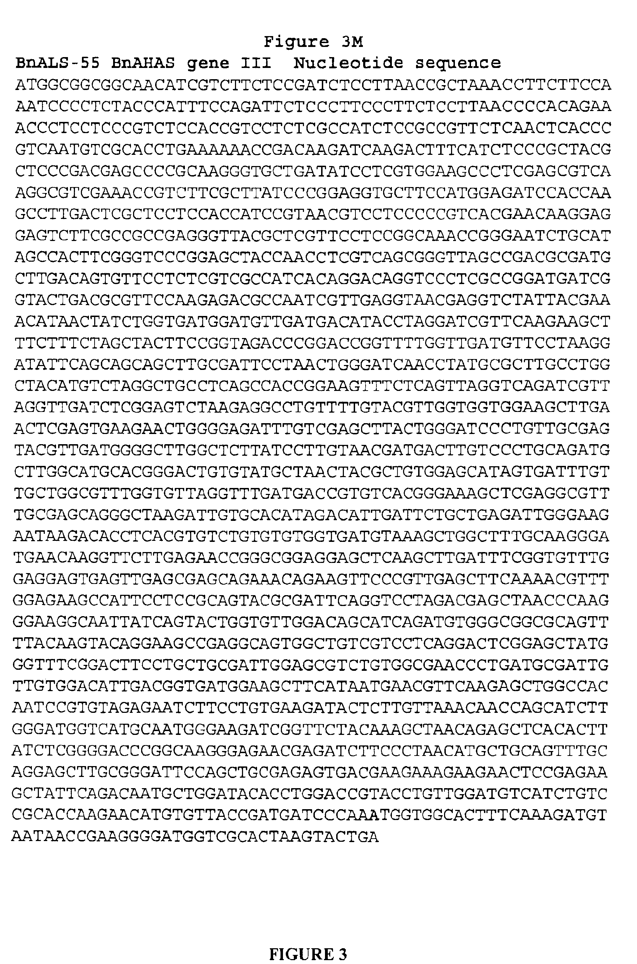

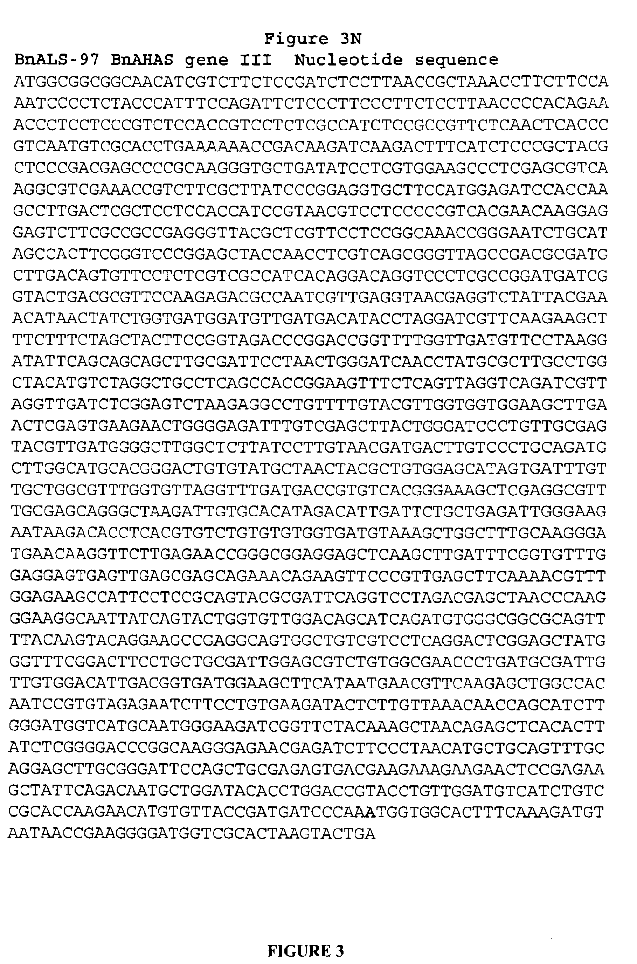

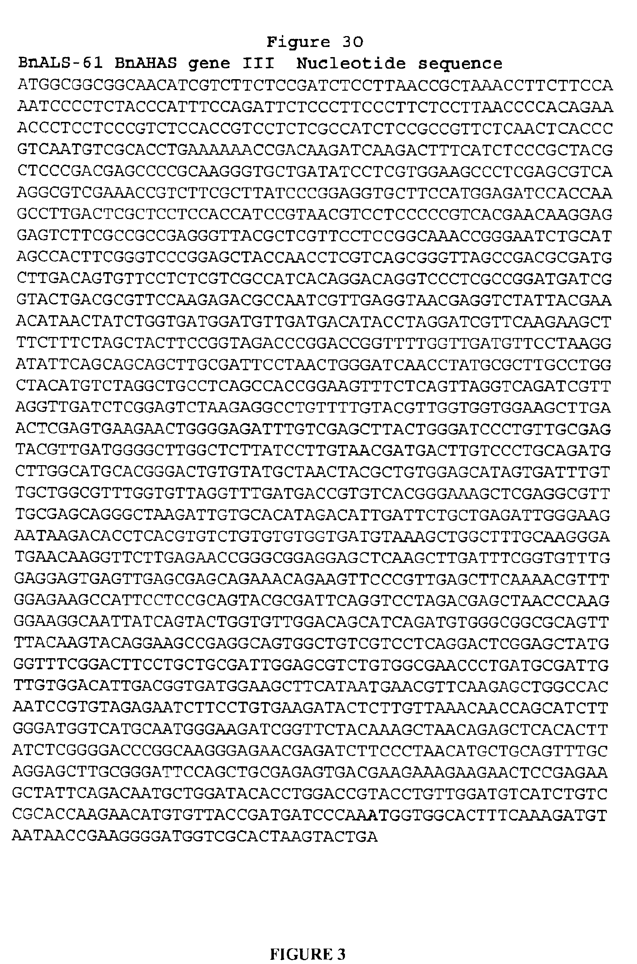

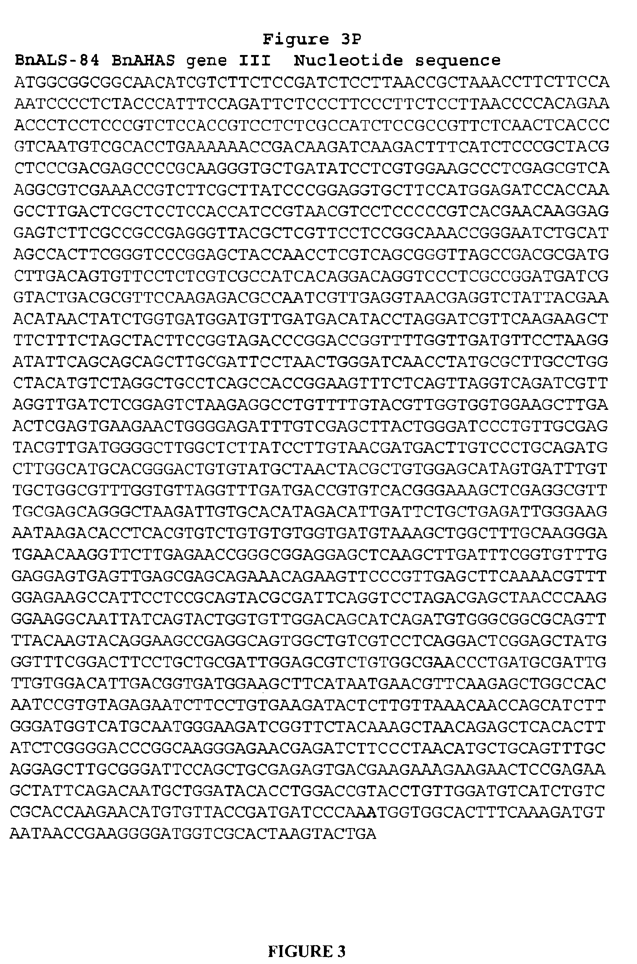

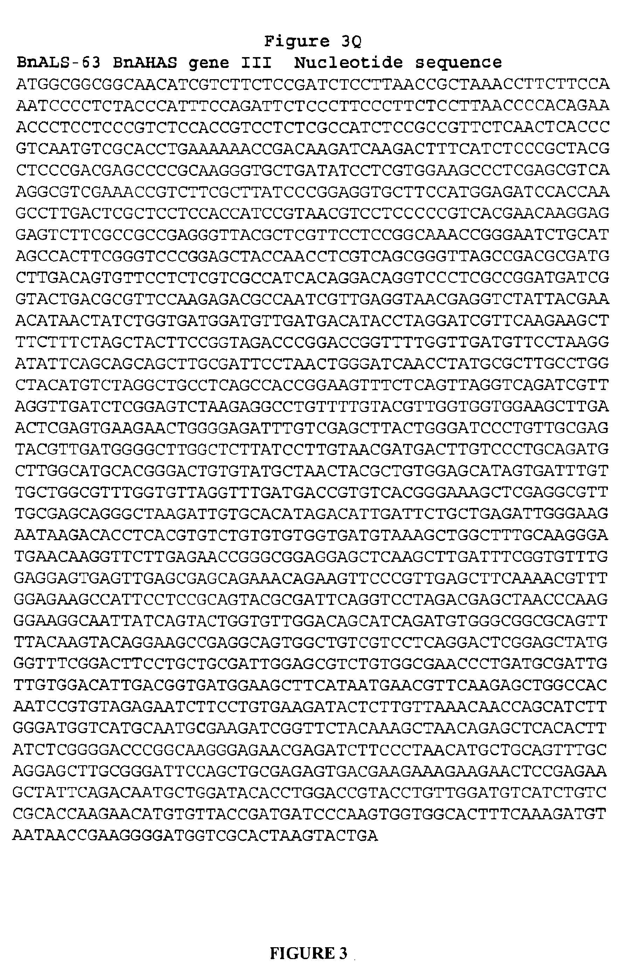

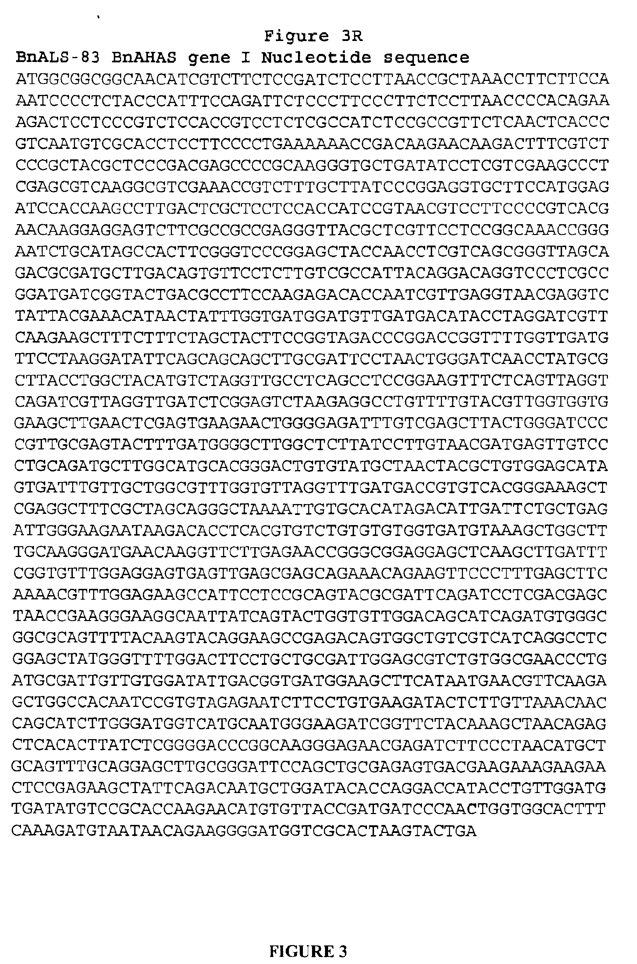

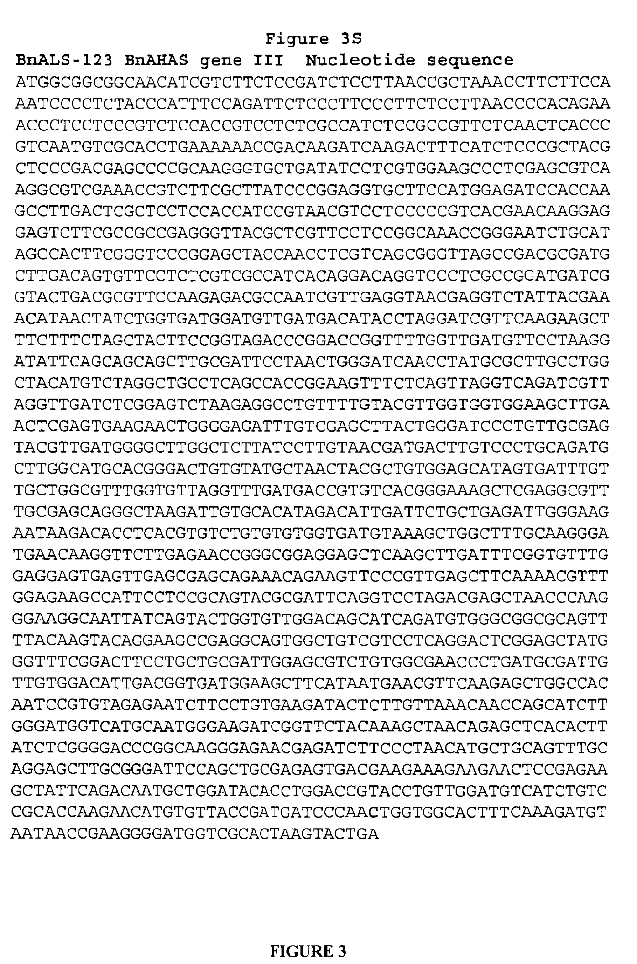

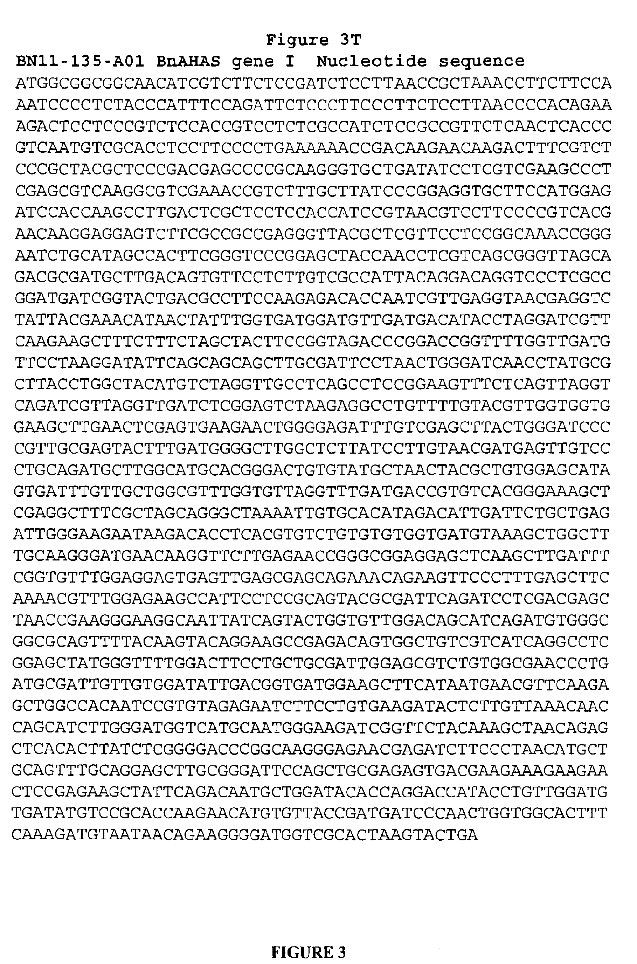

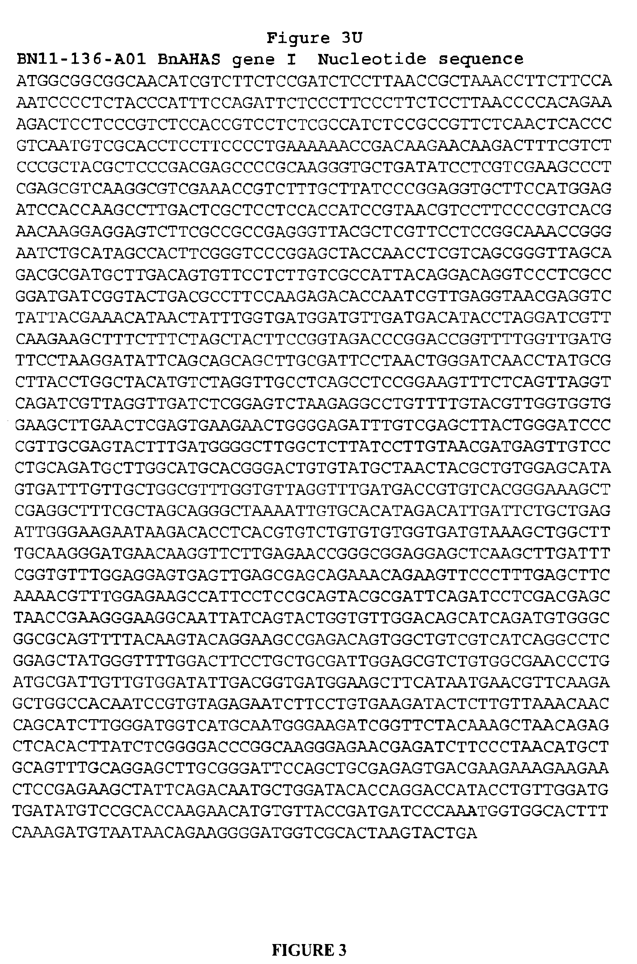

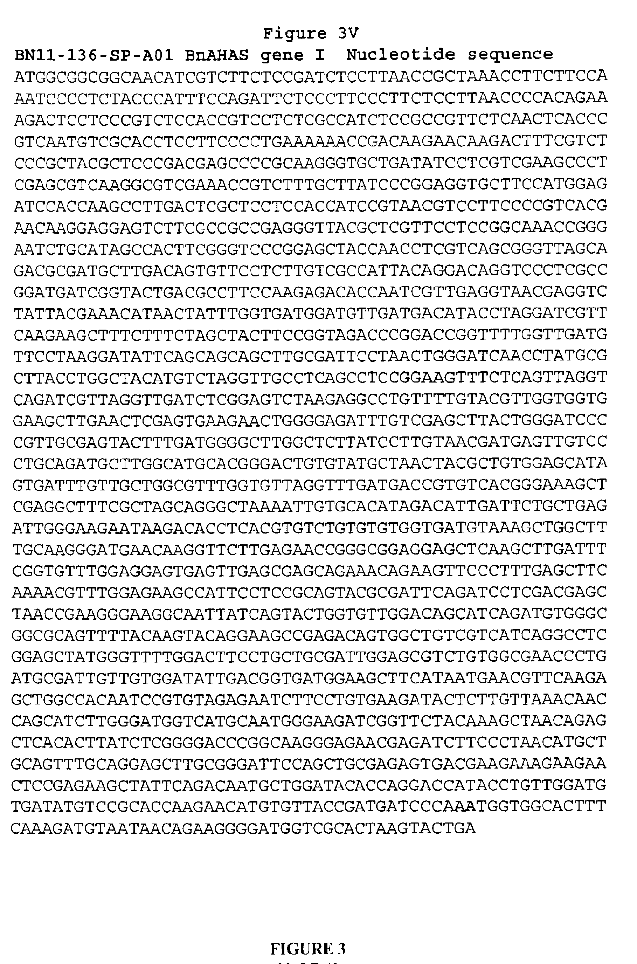

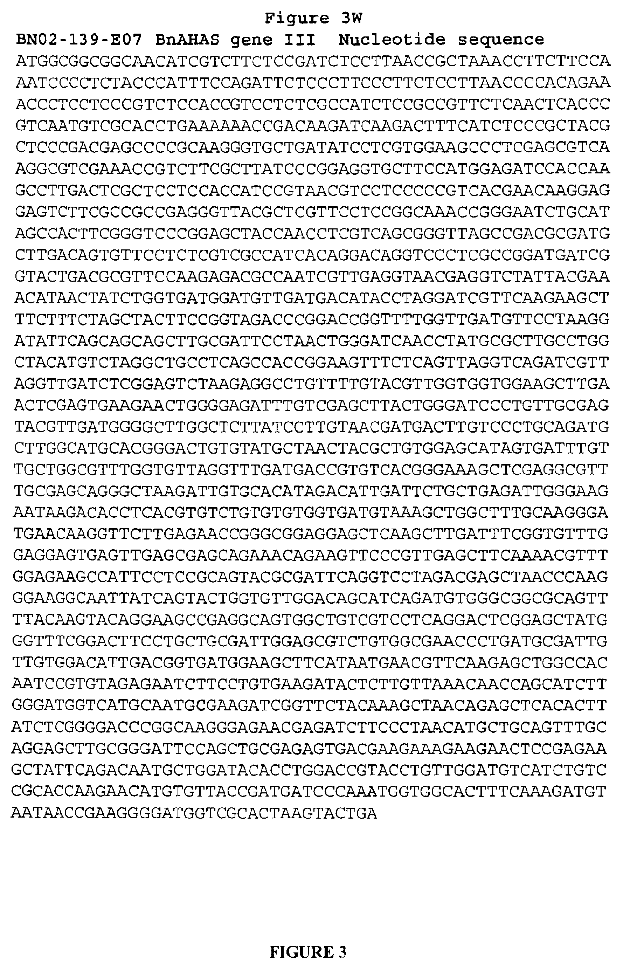

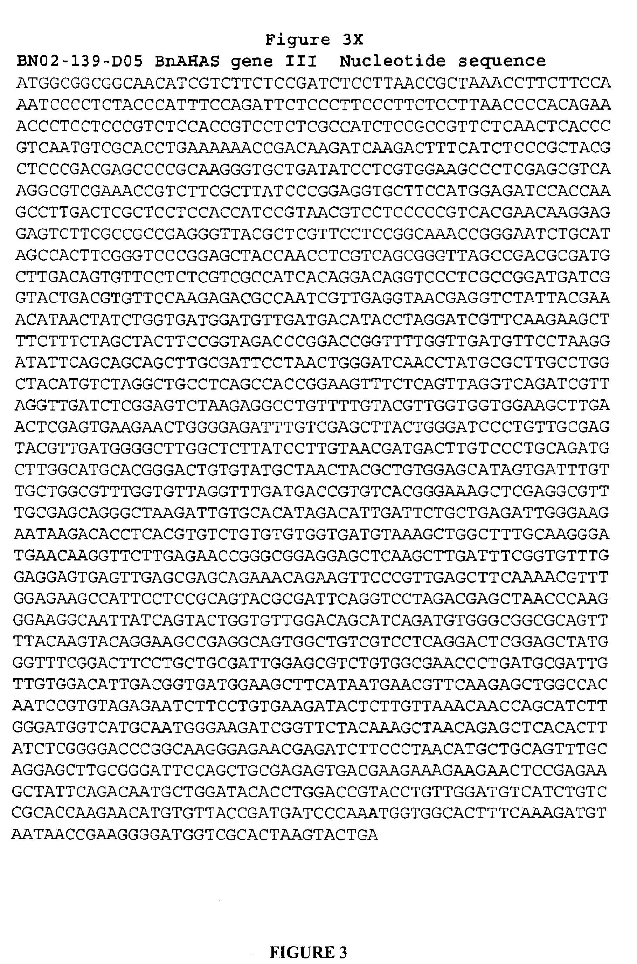

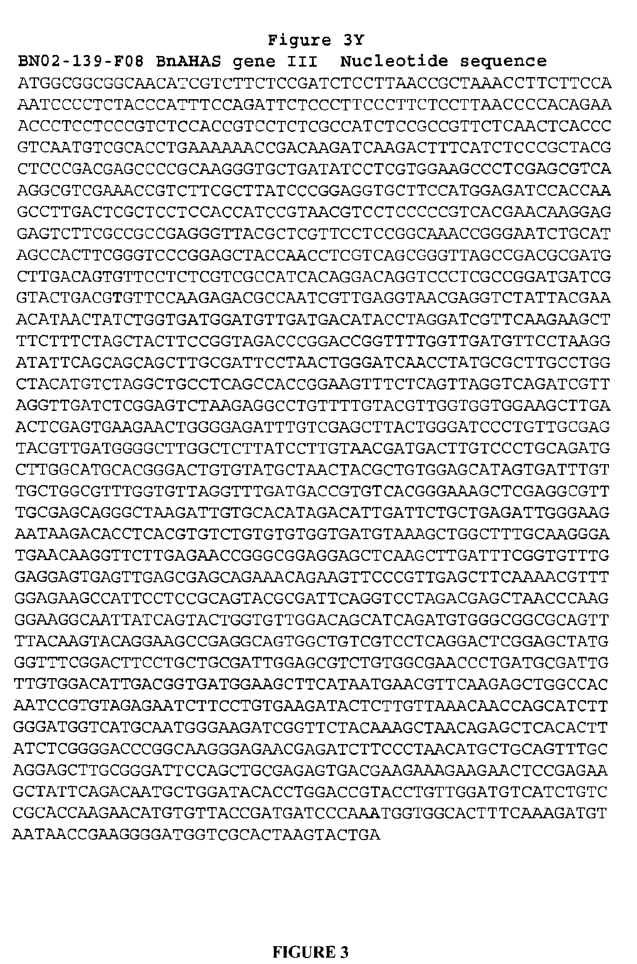

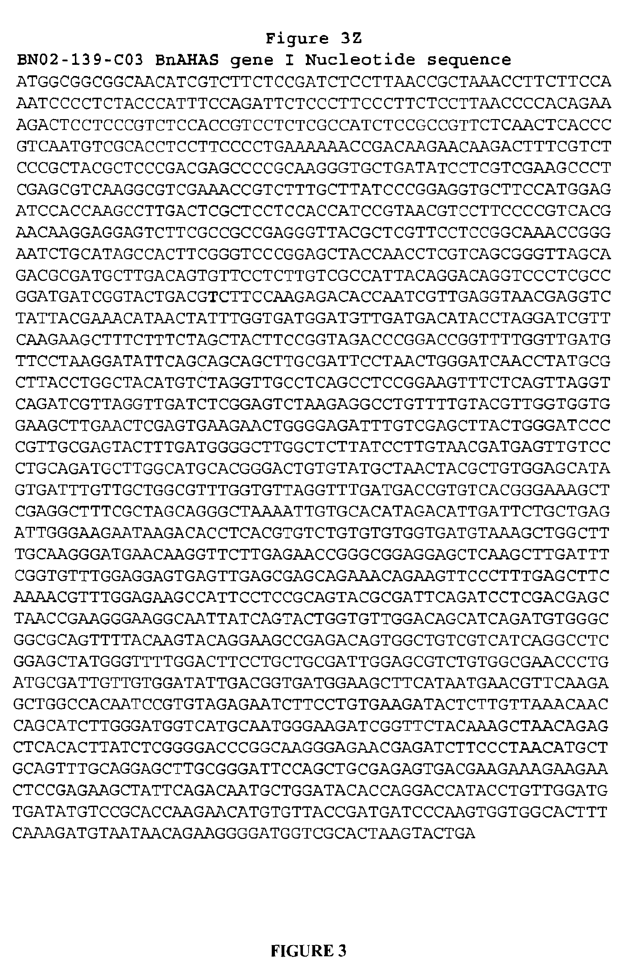

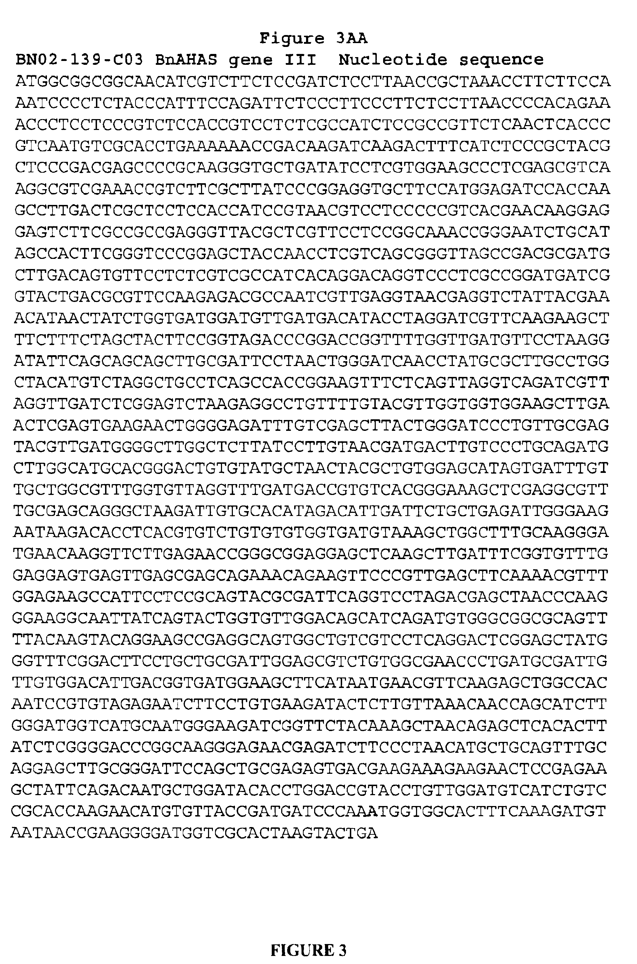

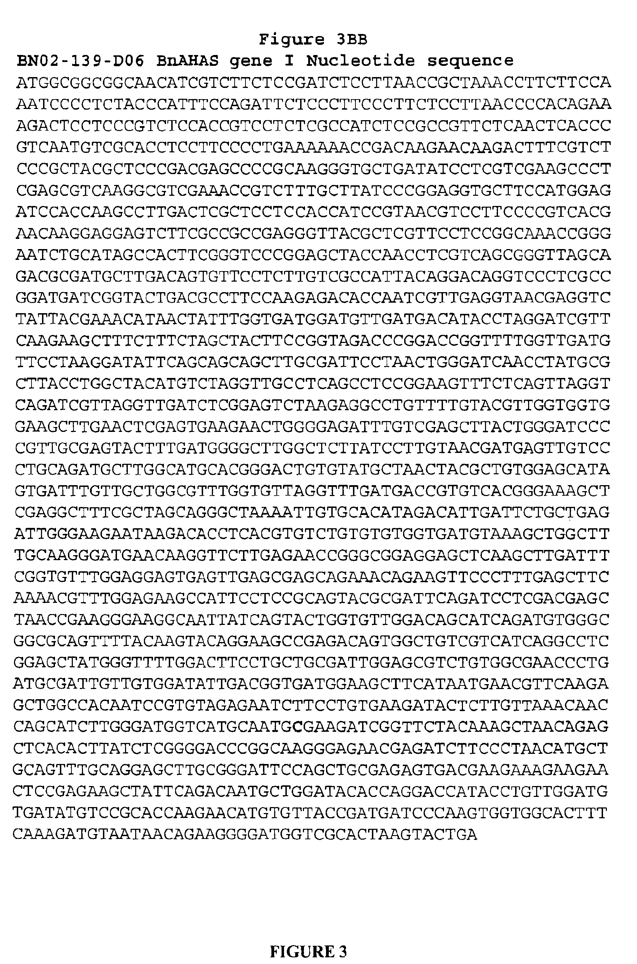

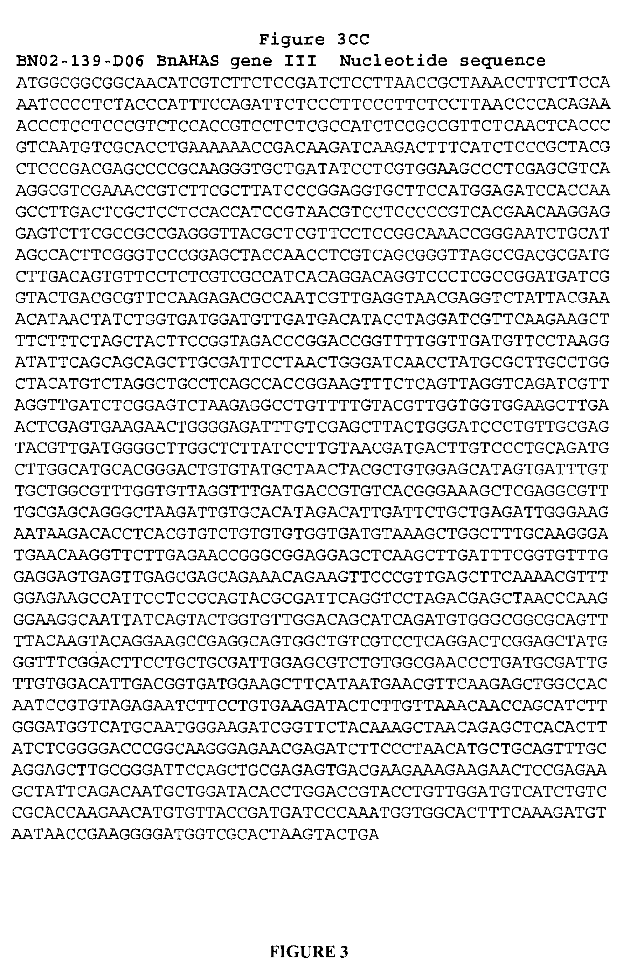

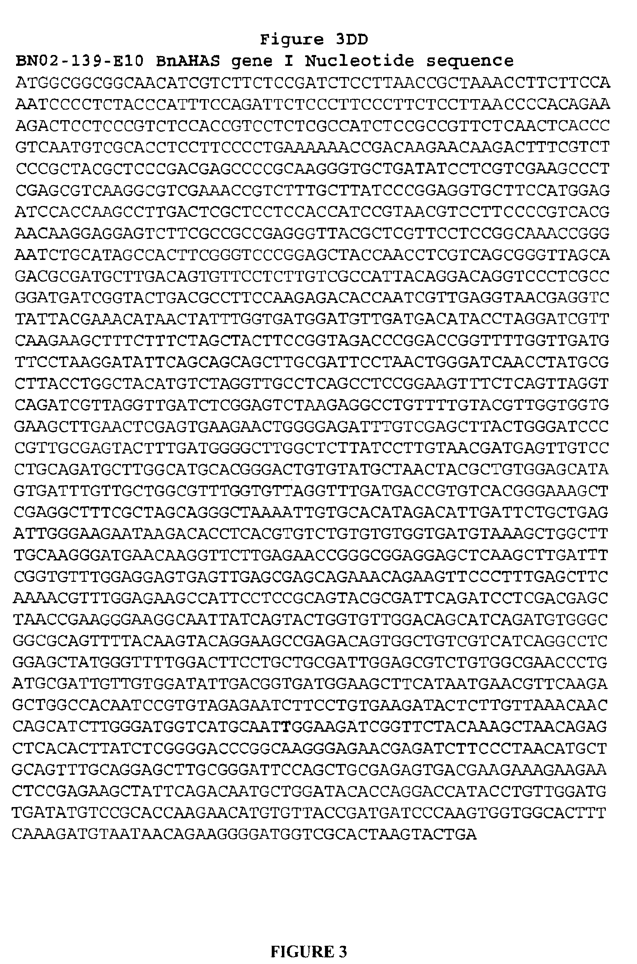

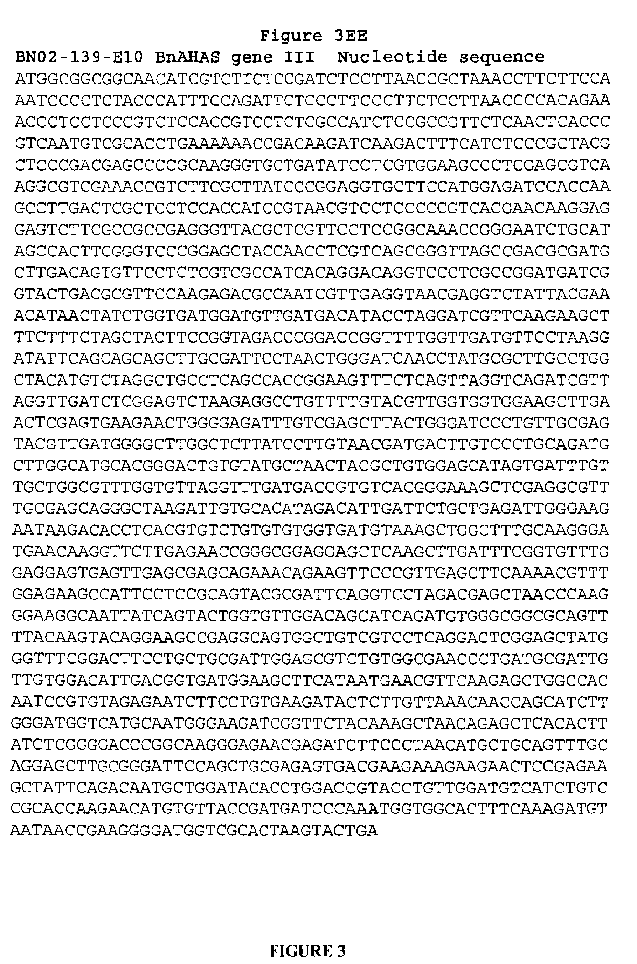

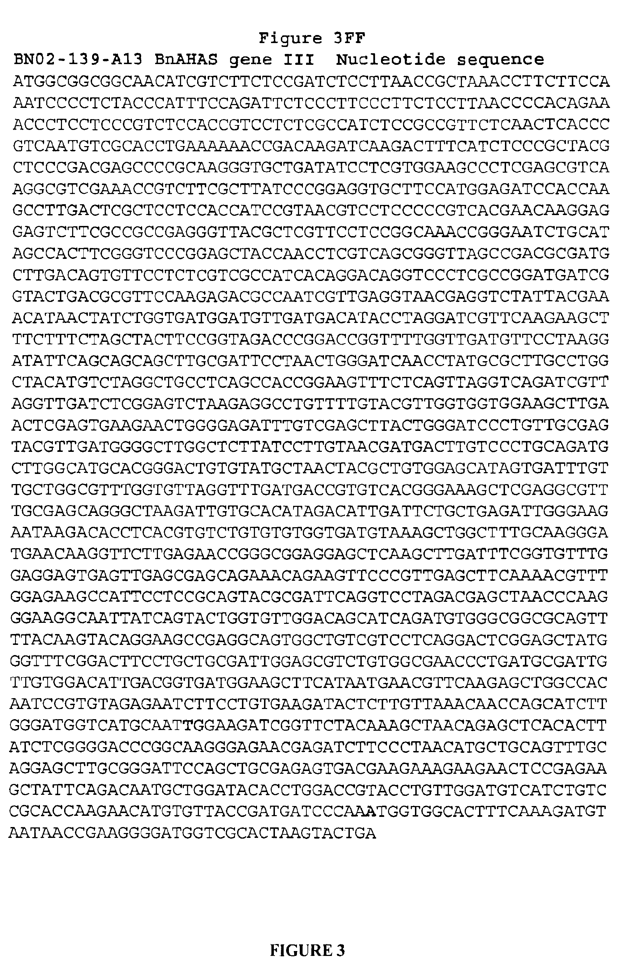

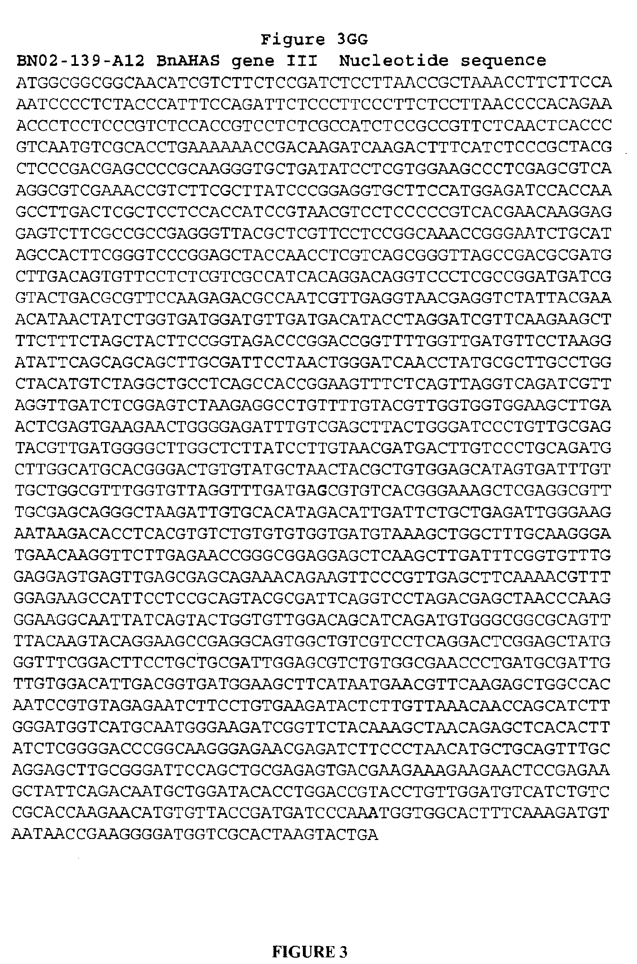

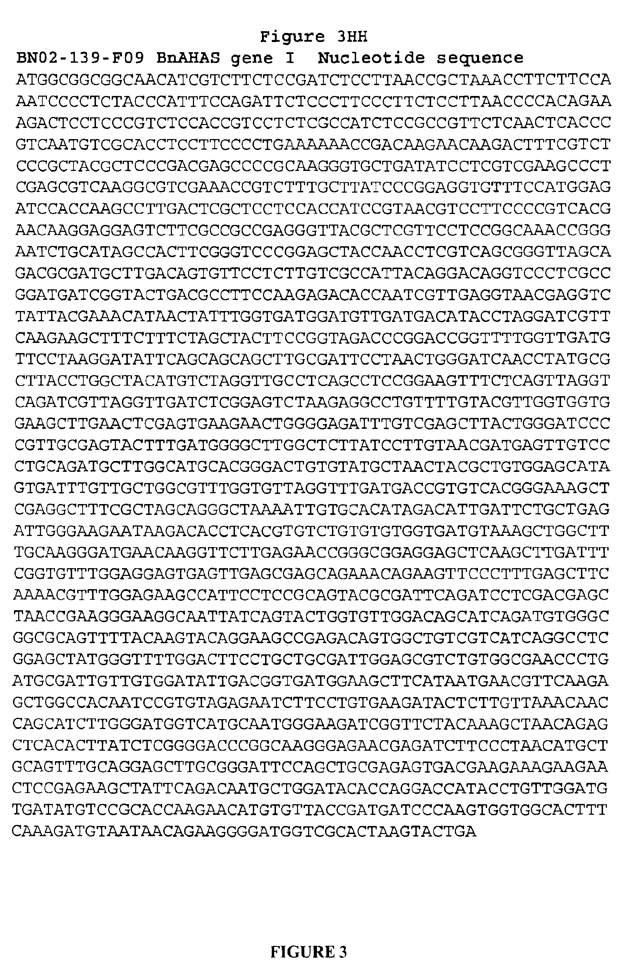

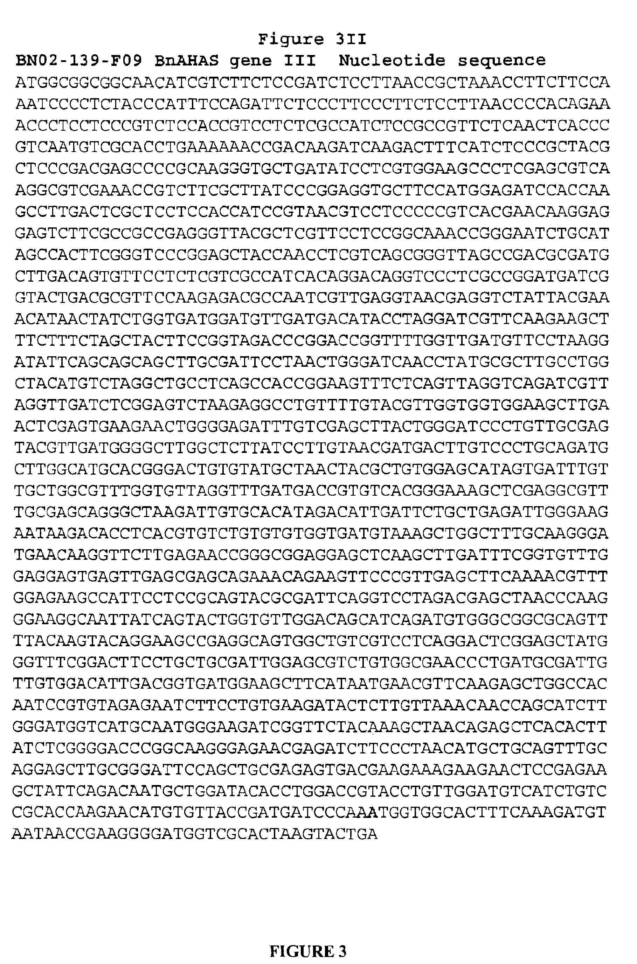

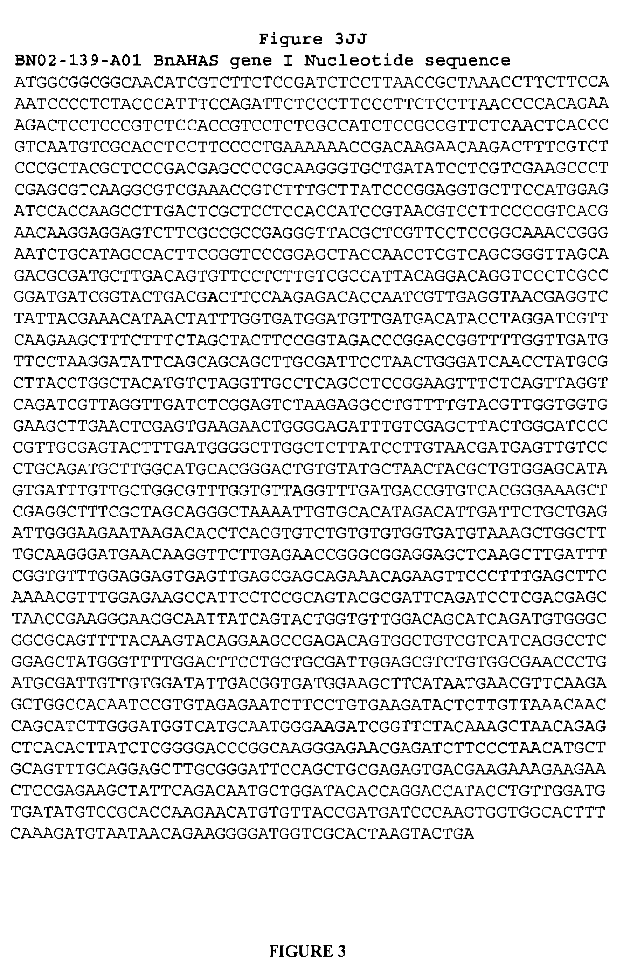

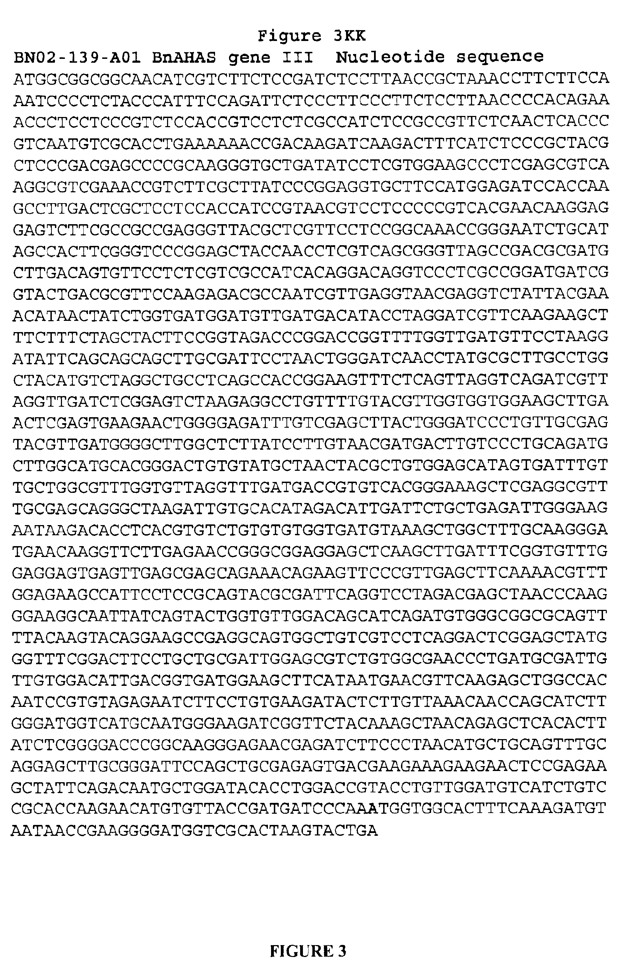

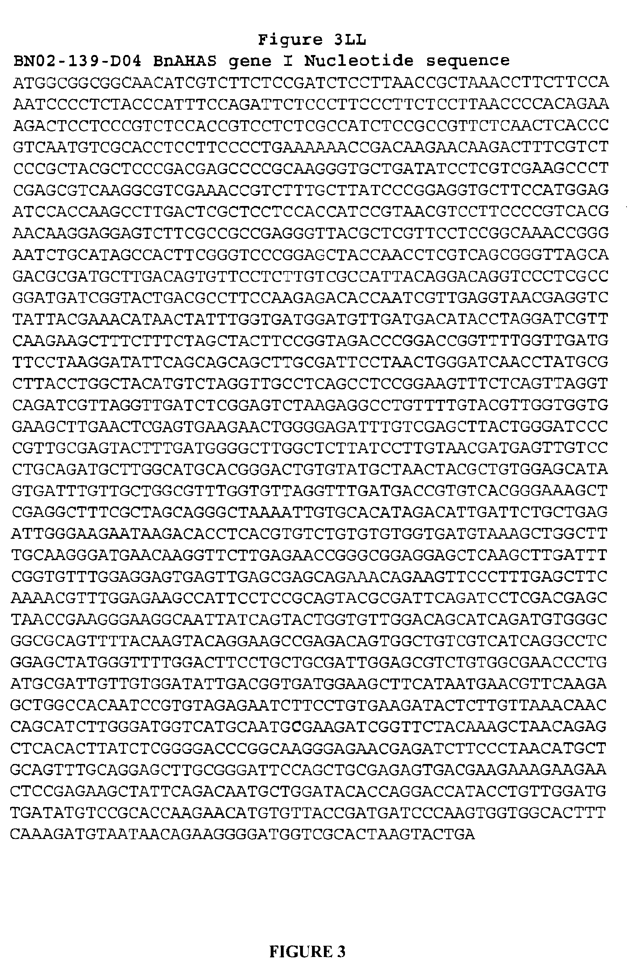

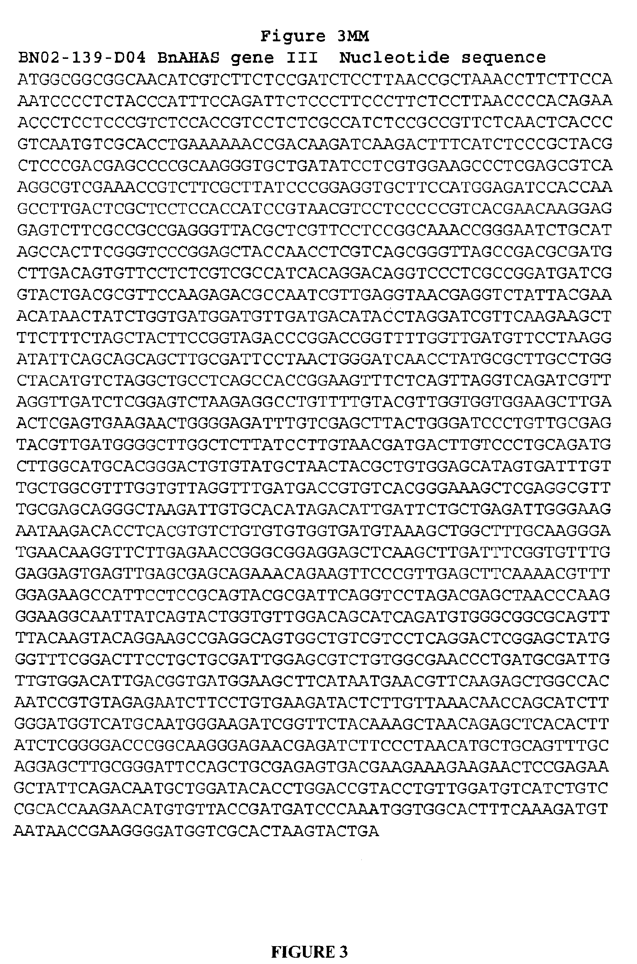

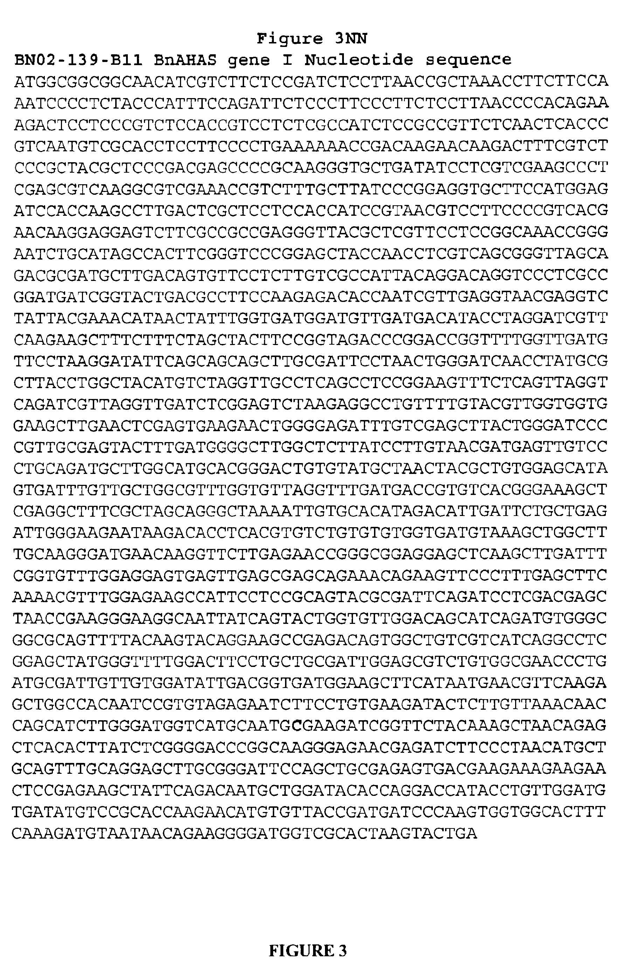

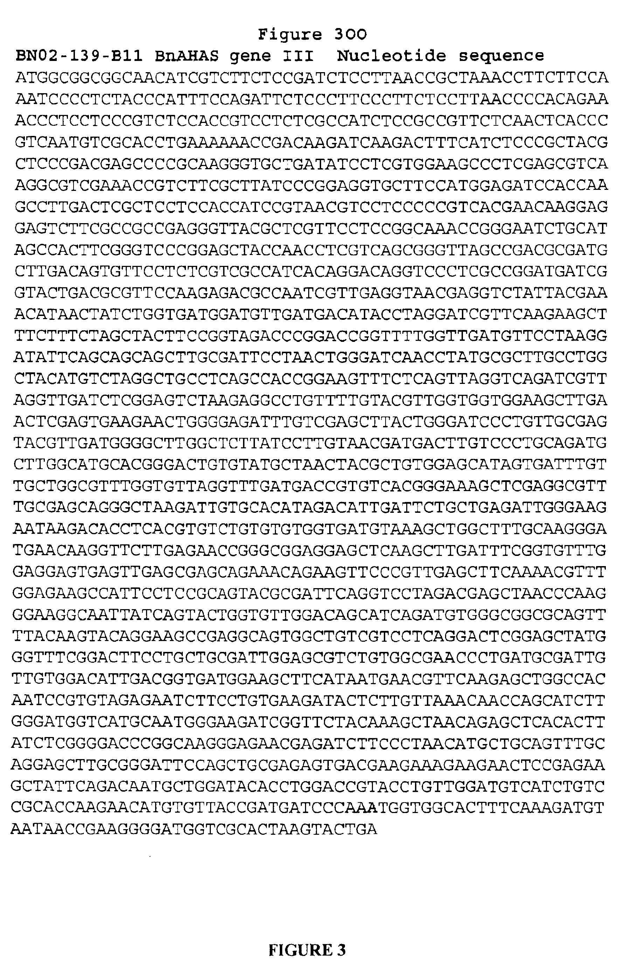

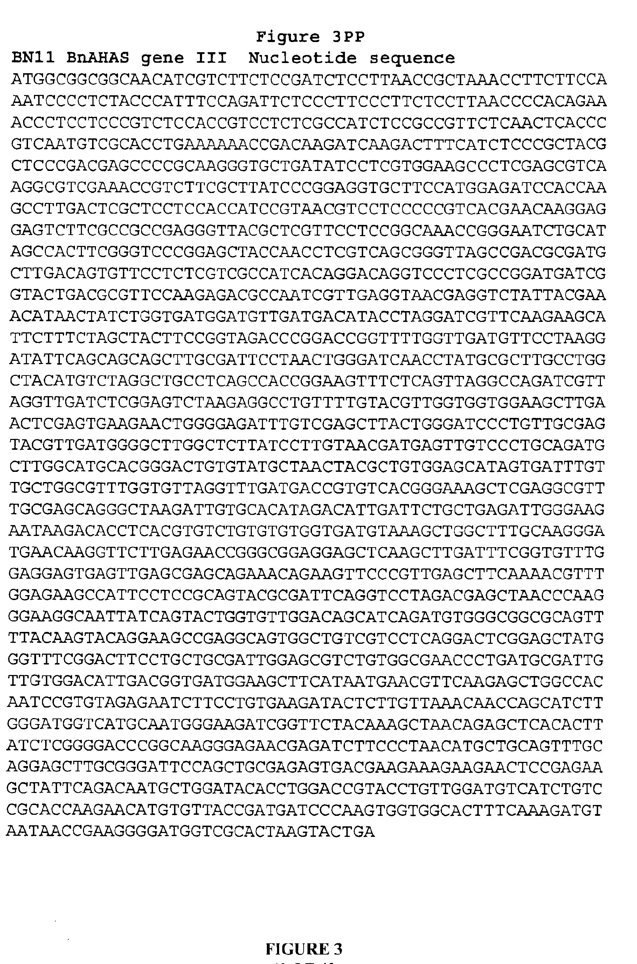

A "gene" refers to a DNA sequence that comprises control and coding sequences necessary for the production of an RNA, which may have a non-coding function (*e.g.,* a ribosomal or transfer RNA) or which may include a polypeptide or a polypeptide precursor. The RNA or polypeptide may be encoded by a full length coding sequence or by any portion of the coding sequence so long as the desired activity or function is retained. As used herein, the term "AHAS Gene" refers to a gene that has homology to a *Brassica* AHAS gene. In certain embodiments, the AHAS gene has 70%; 75%; 80%; 85%; 90%; 95%; 96%; 97%; 98%; 99%; or 100% identity to a specific *Brassica* AHAS gene, for example the *Brassica napus* AHAS gene I or the *Brassica napus* AHAS gene III. In certain embodiments, the AHAS gene has 60%; 70%; 75%; 80%; 85%; 90%; 95%; 96%; 97%; 98%; 99%; or 100% identity to a sequence selected from the sequences in Figure 3.

The AHAS gene is modified with at least two mutations.

The AHAS gene is modified with at least two mutations shown in Table 2.

By "coding sequence" is meant a sequence of a nucleic acid or its complement, or a part thereof, that can be transcribed and/or translated to produce the mRNA for and/or the polypeptide or a fragment thereof. Coding sequences include exons in a genomic DNA or immature primary RNA transcripts, which are joined together by the cell's biochemical machinery to provide a mature mRNA. The anti-sense strand is the complement of such a nucleic acid, and the encoding sequence can be deduced therefrom.

By "non-coding sequence" is meant a sequence of a nucleic acid or its complement, or a part thereof, that is not transcribed into amino acid *in vivo,* or where tRNA does not interact to place or attempt to place an amino acid. Non-coding sequences include both intron sequences in genomic DNA or immature primary RNA transcripts, and gene-associated sequences such as promoters, enhancers, silencers, *etc.*

A nucleobase is a base, which in certain preferred embodiments is a purine, pyrimidine, or a derivative or analog thereof. Nucleosides are nucleobases that contain a pentosefuranosyl moiety, e.g., an optionally substituted riboside or 2'-deoxyriboside. Nucleosides can be linked by one of several linkage moieties, which may or may not contain phosphorus. Nucleosides that are linked by unsubstituted phosphodiester linkages are termed nucleotides. The term "nucleobase" as used herein includes peptide nucleobases, the subunits of peptide nucleic acids, and morpholine nucleobases as well as nucleosides and nucleotides.

An oligonucleobase is a polymer comprising nucleobases; preferably at least a portion of which can hybridize by Watson-Crick base pairing to a DNA having the complementary sequence. An oligonucleobase chain may have a single 5' and 3' terminus, which are the ultimate nucleobases of the polymer. A particular oligonucleobase chain can contain nucleobases of all types. An oligonucleobase compound is a compound comprising one or more oligonucleobase chains that may be complementary and hybridized by Watson-Crick base pairing. Ribo-type nucleobases include pentosefuranosyl containing nucleobases wherein the 2' carbon is a methylene substituted with a hydroxyl, alkyloxy or halogen. Deoxyribo-type nucleobases are nucleobases other than ribo-type nucleobases and include all nucleobases that do not contain a pentosefuranosyl moiety.

In certain embodiments, an oligonucleobase strand may include both oligonucleobase chains and segments or regions of oligonucleobase chains. An oligonucleobase strand may have a 3' end and a 5' end, and when an oligonucleobase strand is coextensive with a chain, the 3' and 5' ends of the strand are also 3' and 5' termini of the chain.

The term "gene repair oligonucleobase" as used herein denotes oligonucleobases, including mixed duplex oligonucleotides, non-nucleotide containing molecules, single stranded oligodeoxynucleotides and other gene repair molecules.

By "isolated", when referring to a nucleic acid (e.g., an oligonucleotide such as RNA, DNA, or a mixed polymer) is meant a nucleic acid that is apart from a substantial portion of the genome in which it naturally occurs and/or is substantially separated from other cellular components which naturally accompany such nucleic acid. For example, any nucleic acid that has been produced synthetically (e.g., by serial base condensation) is considered to be isolated. Likewise, nucleic acids that are recombinantly expressed, cloned, produced by a primer extension reaction (e.g., PCR), or otherwise excised from a genome are also considered to be isolated.

An "amino acid sequence" refers to a polypeptide or protein sequence. The convention "AAwt###AAmut" is used to indicate a mutation that results in the wild-type amino acid AAwt at position ### in the polypeptide being replaced with mutant AAmut.

By "complement" is meant the complementary sequence to a nucleic acid according to standard Watson/Crick pairing rules. A complement sequence can also be a sequence of RNA complementary to the DNA sequence or its complement sequence, and can also be a cDNA.

By "substantially complementary" is meant that two sequences hybridize under stringent hybridization conditions. The skilled artisan will understand that substantially complementary sequences need not hybridize along their entire length.

As used herein the term "codon" refers to a sequence of three adjacent nucleotides (either RNA or DNA) constituting the genetic code that determines the insertion of a specific amino acid in a polypeptide chain during protein synthesis or the signal to stop protein synthesis. The term "codon" is also used to refer to the corresponding (and complementary) sequences of three nucleotides in the messenger RNA into which the original DNA is transcribed.

As used herein, the term "AHAS Protein" refers to a protein that has homology to a *Brassica* AHAS protein. In certain embodiments, the AHAS protein has 70%; 75%; 80%; 85%; 90%; 95%; 96%; 97%; 98%; 99%; or 100% identity to a specific *Brassica* AHAS protein, such as for example, the *Brassica napus* AHAS protein I or the *Brassica napus* AHAS protein III. In certain embodiments, the AHAS protein has 70%; 75%; 80%; 85%; 90%; 95%; 96%; 97%; 98%; 99%; or 100% identity to a sequence selected from the sequences in Figure 2.

The AHAS protein is modified with at least two mutations.

The AHAS protein is modified with at least two mutations shown in Table 2.

The term "wild-type" refers to a gene or a gene product that has the characteristics of that gene or gene product when isolated from a naturally occurring source. A wild-type gene is that which is most frequently observed in a population and is thus arbitrarily designated the "normal" or "wild-type" form of the gene. "Wild-type" may also refer to the sequence at a specific nucleotide position or positions, or the sequence at a particular codon position or positions, or the sequence at a particular amino acid position or positions.

As used herein, "mutant," or "modified" refers to a nucleic acid or protein which displays modifications in sequence and or functional properties (*i.e*., altered characteristics) when compared to the wild-type gene or gene product. "Mutant," or "modified" also refers to the sequence at a specific nucleotide position or positions, or the sequence at a particular codon position or positions, or the sequence at a particular amino acid position or positions which displays modifications in sequence and or functional properties (*i.e.,* altered characteristics) when compared to the wild-type gene or gene product.

A "mutation" is meant to encompass at least a single nucleotide variation in a nucleic acid sequence or a single amino acid variation in a polypeptide relative to the normal sequence or wild-type sequence. A mutation may include a substitution, a deletion, an inversion or an insertion.

As used herein, the term "homology" refers to sequence similarity among proteins and DNA. The term "homology" or "homologous" refers to a degree of identity. There may be partial homology or complete homology. A partially homologous sequence is one that has less than 100% sequence identity when compared to another sequence.

"Heterozygous" refers to having different alleles at one or more genetic loci in homologous chromosome segments. As used herein "heterozygous" may also refer to a sample, a cell, a cell population or an organism in which different alleles at one or more genetic loci may be detected. Heterozygous samples may also be determined via methods known in the art such as, for example, nucleic acid sequencing. For example, if a sequencing electropherogram shows two peaks at a single locus and both peaks are roughly the same size, the sample may be characterized as heterozygous. Or, if one peak is smaller than another, but is at least about 25% the size of the larger peak, the sample may be characterized as heterozygous. In some embodiments, the smaller peak is at least about 15% of the larger peak. In other embodiments, the smaller peak is at least about 10% of the larger peak. In other embodiments, the smaller peak is at least about 5% of the larger peak. In other embodiments, a minimal amount of the smaller peak is detected.

As used herein, "homozygous" refers to having identical alleles at one or more genetic loci in homologous chromosome segments. "Homozygous" may also refer to a sample, a cell, a cell population or an organism in which the same alleles at one or more genetic loci may be detected. Homozygous samples may be determined via methods known in the art, such as, for example, nucleic acid sequencing. For example, if a sequencing electropherogram shows a single peak at a particular locus, the sample may be termed "homozygous" with respect to that locus.

The term "hemizygous" refers to a gene or gene segment being present only once in the genotype of a cell or an organism because the second allele is deleted. As used herein "hemizygous" may also refer to a sample, a cell, a cell population or an organism in which an allele at one or more genetic loci may be detected only once in the genotype.

The term "zygosity status" as used herein refers to a sample, a cell population, or an organism as appearing heterozygous, homozygous, or hemizygous as determined by testing methods known in the art and described herein. The term "zygosity status of a nucleic acid" means determining whether the source of nucleic acid appears heterozygous, homozygous, or hemizygous. The "zygosity status" may refer to differences in a single nucleotide in a sequence. In some methods, the zygosity status of a sample with respect to a single mutation may be categorized as homozygous wild-type, heterozygous (*i.e.,* one wild-type allele and one mutant allele), homozygous mutant, or hemizygous (*i.e.,* a single copy of either the wild-type or mutant allele).

As used herein, the term "RTDS" refers to The *Rapid Trait Development System*™ (RTDS) developed by Cibus. RTDS is a site-specific gene modification system that is effective at making precise changes in a gene sequence without the incorporation of foreign genes or control sequences.

The term "about" as used herein means in quantitative terms plus or minus 10%. For example, "about 3%" would encompass 2.7-3.3% and "about 10%" would encompass 9-11%.

### BRIEF DESCRIPTION OF THE FIGURES

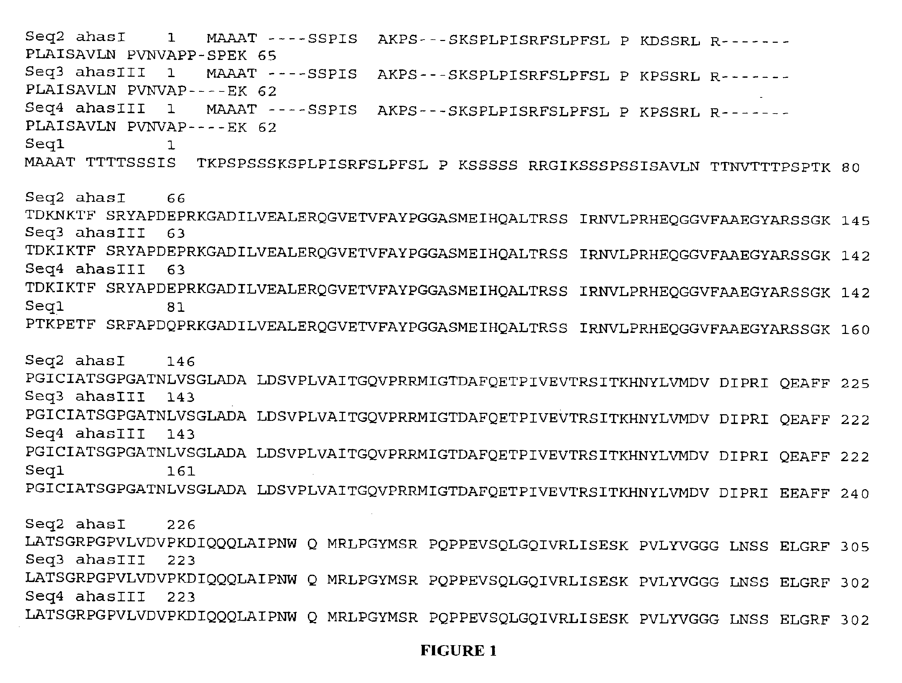

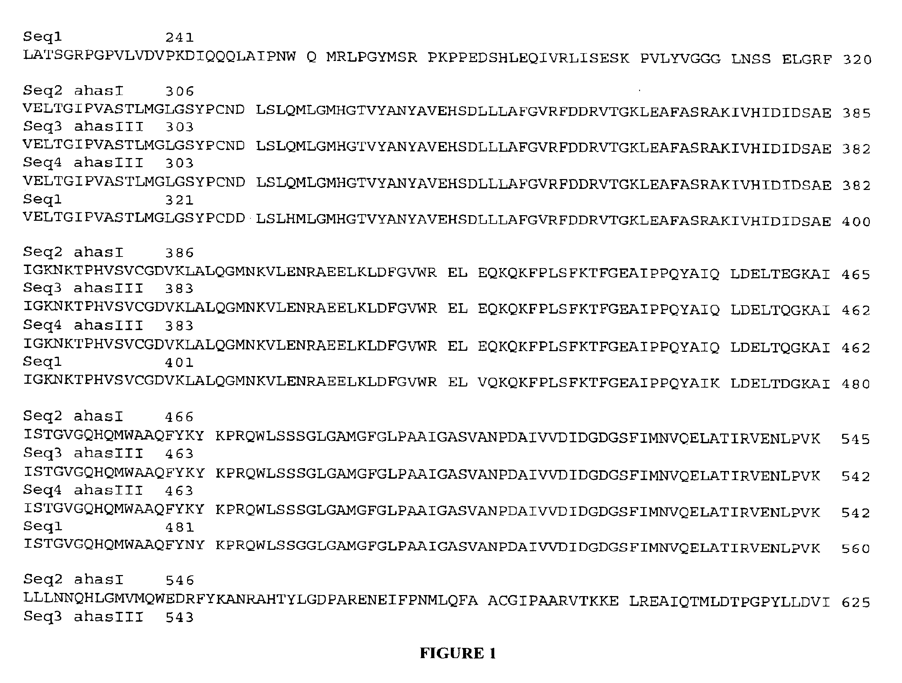

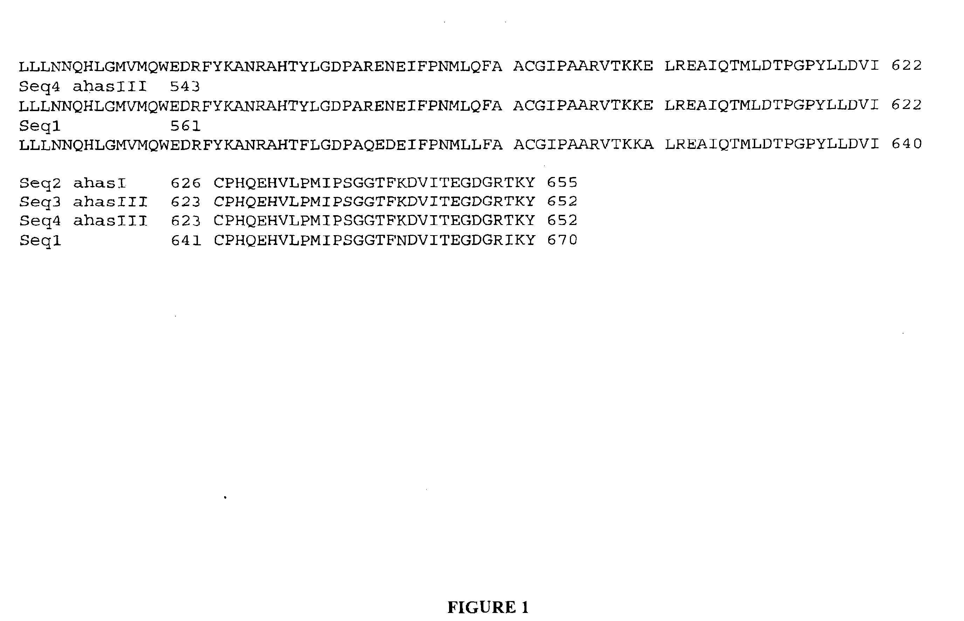

**FIGURE 1** shows an amino acid alignment of *Arabidopsis* acetohydroxyacid synthase AHAS (SEQ ID NO: 1), *Brassica napus* AHAS I (SEQ ID NO: 2), and *Brassica napus* AHAS III (SEQ ID NOs: 3 and 4). SEQ ID NO: 1 is the amino acid sequence of the *Arabidopsis* AHAS At3g48560 based on the annotated genomic DNA sequence Genbank accession number NC003074. SEQ ID NO: 2 is the amino acid sequence of *Brassica napus* AHAS I from Cibus elite lines BN-2 and BN-11. This sequence is identical to the translated product of Genbank accession Z11524. SEQ ID NO: 3 is the amino acid sequence of *Brassica napus* AHAS III from Cibus elite line BN-2. This sequence is identical to the translated product of Genbank accession Z11526, excepting a D325E substitution at amino acid 325. SEQ ID NO: 4 is the amino acid sequence of *Brassica napus* AHAS III from Cibus elite line BN-11. This sequence is identical to the translated product of SEQ ID NO: 3, excepting an E343 at amino acid 343 as in SEQ ID NO: 1.
Figure 2 shows amino acid sequences of translated genes referred to in Table 2. Amino acids indicated in bold represent the mutation.
Figure 3 shows nucleotide sequences referred to in Table 2. Nucleotides indicated in bold represent the mutation.
Figure 4 shows results of the spray trial described in Example 4.

### DETAILED DESCRIPTION OF THE INVENTION

Provided are compositions and methods related in part to the successful targeting of acetohydroxyacid synthase (AHAS) genes in *Brassica* using, for example, the Rapid Trait Development System (***RTDS***™) technology developed by Cibus. In combination or alone, plants containing any of the mutations disclosed herein can form the basis of new herbicide-resistant products. Also provided are seeds produced from the mutated plants in which the AHAS genes are either homozygous or heterozygous for the mutations. The mutations disclosed herein can be in combination with any other mutation known or with mutations discovered in the future.

*RTDS* is based on altering a targeted gene by utilizing the cell's own gene repair system to specifically modify the gene sequence in situ and not insert foreign DNA and gene expression control sequences. This procedure effects a precise change in the genetic sequence while the rest of the genome is left unaltered. In contrast to conventional transgenic GMOs, there is no integration of foreign genetic material, nor is any foreign genetic material left in the plant. The changes in the genetic sequence introduced by *RTDS* are not randomly inserted. Since affected genes remain in their native location, no random, uncontrolled or adverse pattern of expression occurs.

The *RTDS* that effects this change is a chemically synthesized oligonucleotide which may be composed of both DNA and modified RNA bases as well as other chemical moieties, and is designed to hybridize at the targeted gene location to create a mismatched base-pair(s). This mismatched base-pair acts as a signal to attract the cell's own natural gene repair system to that site and correct (replace, insert or delete) the designated nucleotide(s) within the gene. Once the correction process is complete the *RTDS* molecule is degraded and the now-modified or repaired gene is expressed under that gene's normal endogenous control mechanisms.

The subject mutations in the AHAS I and III genes were described using the *Brassica napus* AHAS genes and proteins (see SEQ ID NOs: 2, 3 and 4). The compositions and methods also encompass mutant AHAS genes of other species (paralogs). However, due to variations in the AHAS genes of different species, the number of the amino acid residue to be changed in one species may be different in another species. Nevertheless, the analogous position is readily identified by one of skill in the art by sequence homology. For example, FIGURE 1 shows the aligned amino acid sequences of the *Arabidopsis* AHAS (SEQ ID NO:1) and *Brassica napus* AHAS I (SEQ ID NO:2) and AHAS III (SEQ ID NO:3 and SEQ ID NO: 4) paralogs. Thus, analogous positions in these and other paralogs can be identified and mutated.

The compositions and methods relate in part to mutations in an AHAS gene that render a plant resistant or tolerant to an herbicide of the AHAS-inhibiting or ALS-inhibiting family of herbicides. The compositions and methods also relate to the use of a gene repair oligonucleobase to make a desired mutation in the chromosomal or episomal sequences of a plant in the gene encoding for an AHAS protein. The mutated protein, which substantially maintains the catalytic activity of the wild-type protein, allows for increased resistance or tolerance of the plant to an herbicide of the AHAS-inhibiting family, and allows for the substantially normal growth or development of the plant, its organs, tissues, or cells as compared to the wild-type plant irrespective of the presence or absence of the herbicide. The compositions and methods also relate to a non-transgenic plant cell in which an AHAS gene has been mutated, a non-transgenic plant regenerated therefrom, as well as a plant resulting from a cross using a regenerated non-transgenic plant to a plant having a mutation in a different AHAS gene or to a plant having a mutated EPSPS gene, for example.

Imidazolinones are among the five chemical families of AHAS-inhibiting herbicides. The other four families are sulfonylureas, triazolopyrimidines, pyrimidinylthiobenzoates and sulfonylamino-carbonyltriazolinones (Tan *et al.,* 2005).

Also provided is a transgenic or non-transgenic plant or plant cell having one or more mutations in the AHAS gene, for example, such as disclosed herein. In certain embodiments, the plant or plant cell having one or more mutations in the AHAS gene has increased resistance or tolerance to a member of the AHAS-inhibiting. In certain examples, the plant or plant cell having one or more mutations in the AHAS gene may exhibit substantially normal growth or development of the plant, its organs, tissues or cells, as compared to the corresponding wild-type plant or cell. Further disclosed are non-transgenic plants having a mutation in an AHAS gene, for example, such as disclosed herein, which in certain embodiments has increased resistance or tolerance to a member of the AHAS-inhibiting herbicide family and may exhibit substantially normal growth or development of the plant, its organs, tissues or cells, as compared to the corresponding wild-type plant or cell, i.e., in the presence of one or more herbicide such as for example, an imidazolinone and/or sulfonyl urea, the mutated AHAS protein has substantially the same catalytic activity as compared to the wild-type AHAS protein.

Further provided are methods for producing a plant having a mutated AHAS gene, for example, having mutations as described herein; the plant substantially maintains the catalytic activity of the wild-type protein irrespective of the presence or absence of a relevant herbicide. In certain embodiments, the methods include introducing into a plant cell a gene repair oligonucleobase with one or more targeted mutations in the AHAS gene (for example, such as disclosed herein) and identifying a cell, seed, or plant having a mutated AHAS gene.

In various embodiments, plants as disclosed herein can be of any species of dicotyledonous, monocotyledonous or gymnospermous plant, including any woody plant species that grows as a tree or shrub, any herbaceous species, or any species that produces edible fruits, seeds or vegetables, or any species that produces colorful or aromatic flowers. For example, the plant maybe selected from a species of plant from the group consisting of canola, sunflower, tobacco, sugar beet, cotton, maize, wheat, barley, rice, sorghum, tomato, mango, peach, apple, pear, strawberry, banana, melon, potato, carrot, lettuce, onion, soya spp, sugar cane, pea, field beans, poplar, grape, citrus, alfalfa, rye, oats, turf and forage grasses, flax, oilseed rape, cucumber, morning glory, balsam, pepper, eggplant, marigold, lotus, cabbage, daisy, carnation, tulip, iris, lily, and nut producing plants insofar as they are not already specifically mentioned.

The gene repair oligonucleobase can be introduced into a plant cell using any method commonly used in the art, including but not limited to, microcarriers (biolistic delivery), microfibers, polyethylene glycol (PEG)-mediated uptake, electroporation, and microinjection.

Also provided are methods and compositions related to the culture of cells mutated according to methods as disclosed herein in order to obtain a plant that produces seeds, henceforth a "fertile plant", and the production of seeds and additional plants from such a fertile plant.

Also provided are methods of selectively controlling weeds in a field, the field comprising plants with the disclosed AHAS gene alterations and weeds, the method comprising application to the field of an herbicide to which the plants have been rendered resistant.

Also provided are mutations in the AHAS gene that confer resistance or tolerance to a member of the relevant herbicide to a plant or wherein the mutated AHAS gene has substantially the same enzymatic activity as compared to wild-type AHAS.

### Gene Repair Oligonucleobases

The methods and compositions disclosed herein can be practiced or made with "gene repair oligonucleobases" having the conformations and chemistries as described in detail below. The "gene repair oligonucleobases" as contemplated herein have also been described in published scientific and patent literature using other names including "recombinagenic oligonucleobases;" "RNA/DNA chimeric oligonucleotides;" "chimeric oligonucleotides;" "mixed duplex oligonucleotides" (MDONs); "RNA DNA oligonucleotides (RDOs);" "gene targeting oligonucleotides;" "genoplasts;" "single stranded modified oligonucleotides;" "Single stranded oligodeoxynucleotide mutational vectors" (SSOMVs); "duplex mutational vectors;" and "heteroduplex mutational vectors."

Oligonucleobases having the conformations and chemistries described in U.S. Pat. No. 5,565,350 by Kmiec (Kmiec I) and U.S. Pat. No. 5,731,181 by Kmiec (Kmiec II) are suitable for use as "gene repair oligonucleobases" of the invention. The gene repair oligonucleobases in Kmiec I and/or Kmiec II contain two complementary strands, one of which contains at least one segment of RNA-type nucleotides (an "RNA segment") that are base paired to DNA-type nucleotides of the other strand.

Kmiec II discloses that purine and pyrimidine base-containing non-nucleotides can be substituted for nucleotides. Additional gene repair molecules that can be used for the present invention are described in U.S. Pat. Nos. 5,756,325; 5,871,984; 5,760,012; 5,888,983; 5,795,972; 5,780,296; 5,945,339; 6,004,804; and 6,010,907 and in International Patent No. PCT/US00/23457; and in International Patent Publication Nos. WO 98/49350; WO 99/07865; WO 99/58723; WO 99/58702; and WO 99/40789.

In one example, the gene repair oligonucleobase is a mixed duplex oligonucleotides (MDON) in which the RNA-type nucleotides of the mixed duplex oligonucleotide are made RNase resistant by replacing the 2'-hydroxyl with a fluoro, chloro or bromo functionality or by placing a substituent on the 2'-O. Suitable substituents include the substituents taught by the Kmiec II. Alternative substituents include the substituents taught by U.S. Pat. No. 5,334,711 (Sproat) and the substituents taught by patent publications EP 629 387 and EP 679 657 (collectively, the Martin Applications). As used herein, a 2'-fluoro, chloro or bromo derivative of a ribonucleotide or a ribonucleotide having a 2'-OH substituted with a substituent described in the Martin Applications or Sproat is termed a "2'-Substituted Ribonucleotide." As used herein the term "RNA-type nucleotide" means a 2'-hydroxyl or 2'-Substituted Nucleotide that is linked to other nucleotides of a mixed duplex oligonucleotide by an unsubstituted phosphodiester linkage or any of the non-natural linkages taught by Kmiec I or Kmiec II. As used herein the term "deoxyribo-type nucleotide" means a nucleotide having a 2'-H, which can be linked to other nucleotides of a gene repair oligonucleobase by an unsubstituted phosphodiester linkage or any of the non-natural linkages taught by Kmiec I or Kmiec II.

In a particular example of the present invention, the gene repair oligonucleobase is a mixed duplex oligonucleotides (MDON) that is linked solely by unsubstituted phosphodiester bonds. In alternative examples, the linkage is by substituted phosphodiesters, phosphodiester derivatives and non-phosphorus-based linkages as taught by Kmiec II. In yet another example, each RNA-type nucleotide in the mixed duplex oligonucleotide is a 2'-Substituted Nucleotide. Particular preferred examples of 2'-Substituted Ribonucleotides are 2'-fluoro, 2'-methoxy, 2'-propyloxy, 2'-allyloxy, 2'-hydroxylethyloxy, 2'-methoxyethyloxy, 2'-fluoropropyloxy and 2'-trifluoropropyloxy substituted ribonucleotides. More preferred embodiments of 2'-Substituted Ribonucleotides are 2'-fluoro, 2'-methoxy, 2'-methoxyethyloxy, and 2'-allyloxy substituted nucleotides. In another example the mixed duplex oligonucleotide is linked by unsubstituted phosphodiester bonds.

Although mixed duplex oligonucleotides (MDONs) having only a single type of 2'-substituted RNA-type nucleotide are more conveniently synthesized, the methods can be practiced with mixed duplex oligonucleotides having two or more types of RNA-type nucleotides. The function of an RNA segment may not be affected by an interruption caused by the introduction of a deoxynucleotide between two RNA-type trinucleotides, accordingly, the term RNA segment encompasses terms such as "interrupted RNA segment." An uninterrupted RNA segment is termed a contiguous RNA segment. In an alternative example an RNA segment can contain alternating RNase-resistant and unsubstituted 2'-OH nucleotides. The mixed duplex oligonucleotides preferably have fewer than 100 nucleotides and more preferably fewer than 85 nucleotides, but more than 50 nucleotides. The first and second strands are Watson-Crick base paired. In one example the strands of the mixed duplex oligonucleotide are covalently bonded by a linker, such as a single stranded hexa, penta or tetranucleotide so that the first and second strands are segments of a single oligonucleotide chain having a single 3' and a single 5' end. The 3' and 5' ends can be protected by the addition of a "hairpin cap" whereby the 3' and 5' terminal nucleotides are Watson-Crick paired to adjacent nucleotides. A second hairpin cap can, additionally, be placed at the junction between the first and second strands distant from the 3' and 5' ends, so that the Watson-Crick pairing between the first and second strands is stabilized.

The first and second strands contain two regions that are homologous with two fragments of the target gene, i.e., have the same sequence as the target gene. A homologous region contains the nucleotides of an RNA segment and may contain one or more DNA-type nucleotides of connecting DNA segment and may also contain DNA-type nucleotides that are not within the intervening DNA segment. The two regions of homology are separated by, and each is adjacent to, a region having a sequence that differs from the sequence of the target gene, termed a "heterologous region." The heterologous region can contain one, two or three mismatched nucleotides. The mismatched nucleotides can be contiguous or alternatively can be separated by one or two nucleotides that are homologous with the target gene. Alternatively, the heterologous region can also contain an insertion or one, two, three or of five or fewer nucleotides. Alternatively, the sequence of the mixed duplex oligonucleotide may differ from the sequence of the target gene only by the deletion of one, two, three, or five or fewer nucleotides from the mixed duplex oligonucleotide. The length and position of the heterologous region is, in this case, deemed to be the length of the deletion, even though no nucleotides of the mixed duplex oligonucleotide are within the heterologous region. The distance between the fragments of the target gene that are complementary to the two homologous regions is identical to the length of the heterologous region where a substitution or substitutions is intended. When the heterologous region contains an insertion, the homologous regions are thereby separated in the mixed duplex oligonucleotide farther than their complementary homologous fragments are in the gene, and the converse is applicable when the heterologous region encodes a deletion.

The RNA segments of the mixed duplex oligonucleotides are each a part of a homologous region, i.e., a region that is identical in sequence to a fragment of the target gene, which segments together preferably contain at least 13 RNA-type nucleotides and preferably from 16 to 25 RNA-type nucleotides or yet more preferably 18-22 RNA-type nucleotides or most preferably 20 nucleotides. In one example, RNA segments of the homology regions are separated by and adjacent to, i.e., "connected by" an intervening DNA segment. In one example, each nucleotide of the heterologous region is a nucleotide of the intervening DNA segment. An intervening DNA segment that contains the heterologous region of a mixed duplex oligonucleotide is termed a "mutator segment."

According to the present invention, the gene repair oligonucleobase (GRON) is a single stranded oligodeoxynucleotide mutational vector (SSOMV), which is disclosed in International Patent Application PCT/US00/23457, U.S. Pat. Nos. 6,271,360, 6,479,292, and 7,060,500. The sequence of the SSOMV is based on the same principles as the mutational vectors described in U.S. Pat. Nos. 5,756,325; 5,871,984; 5,760,012; 5,888,983; 5,795,972; 5,780,296; 5,945,339; 6,004,804; and 6,010,907 and in International Publication Nos. WO 98/49350; WO 99/07865; WO 99/58723; WO 99/58702; and WO 99/40789. The sequence of the SSOMV contains two regions that are homologous with the target sequence separated by a region that contains the desired genetic alteration termed the mutator region. The mutator region can have a sequence that is the same length as the sequence that separates the homologous regions in the target sequence, but having a different sequence. Such a mutator region can cause a substitution. Alternatively, the homologous regions in the SSOMV can be contiguous to each other, while the regions in the target gene having the same sequence are separated by one, two or more nucleotides. Such an SSOMV causes a deletion from the target gene of the nucleotides that are absent from the SSOMV. Lastly, the sequence of the target gene that is identical to the homologous regions may be adjacent in the target gene but separated by one, two, or more nucleotides in the sequence of the SSOMV. Such an SSOMV causes an insertion in the sequence of the target gene.

The nucleotides of the SSOMV are deoxyribonucleotides that are linked by unmodified phosphodiester bonds except that the 3' terminal and/or 5' terminal intemucleotide linkage or alternatively the two 3' terminal and/or 5' terminal internucleotide linkages can be a phosphorothioate or phosphoamidate. As used herein an internucleotide linkage is the linkage between nucleotides of the SSOMV and does not include the linkage between the 3' end nucleotide or 5' end nucleotide and a blocking substituent. In a specific embodiment the length of the SSOMV is between 21 and 55 deoxynucleotides and the lengths of the homology regions are, accordingly, a total length of at least 20 deoxynucleotides and at least two homology regions should each have lengths of at least 8 deoxynucleotides.

The SSOMV can be designed to be complementary to either the coding or the non-coding strand of the target gene. When the desired mutation is a substitution of a single base, it is preferred that both the mutator nucleotide and the targeted nucleotide be a pyrimidine. To the extent that is consistent with achieving the desired functional result, it is preferred that both the mutator nucleotide and the targeted nucleotide in the complementary strand be pyrimidines. Particularly preferred are SSOMVs that encode transversion mutations, i.e., a C or T mutator nucleotide is mismatched, respectively, with a C or T nucleotide in the complementary strand.

In addition to the oligodeoxynucleotide, the SSOMV can contain a 5' blocking substituent that is attached to the 5' terminal carbons through a linker. The chemistry of the linker is not critical other than its length, which should preferably be at least 6 atoms long and that the linker should be flexible. A variety of non-toxic substituents such as biotin, cholesterol or other steroids or a non-intercalating cationic fluorescent dye can be used. Particularly preferred reagents to make SSOMVs are the reagents sold as Cy3™ and Cy5™ by Glen Research, Sterling Va. (now GE Healthcare), which are blocked phosphoroamidites that upon incorporation into an oligonucleotide yield 3,3,3',3'-tetramethyl N,N'-isopropyl substituted indomonocarbocyanine and indodicarbocyanine dyes, respectively. Cy3 is particularly preferred. When the indocarbocyanine is N-oxyalkyl substituted it can be conveniently linked to the 5' terminal of the oligodeoxynucleotide as a phosphodiester with a 5' terminal phosphate. The chemistry of the dye linker between the dye and the oligodeoxynucleotide is not critical and is chosen for synthetic convenience. When the commercially available Cy3 phosphoramidite is used as directed, the resulting 5' modification consists of a blocking substituent and linker together which are a N-hydroxypropyl, N'-phosphatidylpropyl 3,3,3',3'-tetramethyl indomonocarbocyanine.

In a preferred embodiment the indocarbocyanine dye is tetra substituted at the 3 and 3' positions of the indole rings. Without limitations as to theory these substitutions prevent the dye from being an intercalating dye. The identity of the substituents at these positions is not critical. The SSOMV can in addition have a 3' blocking substituent. Again the chemistry of the 3' blocking substituent is not critical.

The mutations herein described might also be obtained by mutagenesis (random, somatic or directed) and other DNA editing or recombination technologies including, but not limited to, gene targeting using site-specific homologous recombination by zinc finger nucleases.

### Delivery of Gene Repair Oligonucleobases into Plant Cells

Any commonly known method used to transform a plant cell can be used for delivering the gene repair oligonucleobases. Illustrative methods are listed below.

### Microcarriers and Microfibers

The use of metallic microcarriers (microspheres) for introducing large fragments of DNA into plant cells having cellulose cell walls by projectile penetration is well known to those skilled in the relevant art (henceforth biolistic delivery). U.S. Pat. Nos. 4,945,050; 5,100,792 and 5,204,253 describe general techniques for selecting microcarriers and devices for projecting them.

Specific conditions for using microcarriers in the methods of the present invention are described in International Publication WO 99/07865. In an illustrative technique, ice cold microcarriers (60 mg/mL), mixed duplex oligonucleotide (60 mg/mL) 2.5 M CaCl₂ and 0.1 M spermidine are added in that order; the mixture gently agitated, e.g., by vortexing, for 10 minutes and then left at room temperature for 10 minutes, whereupon the microcarriers are diluted in 5 volumes of ethanol, centrifuged and resuspended in 100% ethanol. Good results can be obtained with a concentration in the adhering solution of 8-10 µg/µL microcarriers, 14-17 µg/mL mixed duplex oligonucleotide, 1.1-1.4 M CaCl₂ and 18-22 mM spermidine. Optimal results were observed under the conditions of 8 µg/µL microcarriers, 16.5 µg/mL mixed duplex oligonucleotide, 1.3 M CaCl₂ and 21 mM spermidine.

Gene repair oligonucleobases can also be introduced into plant cells for the practice of the present invention using microfibers to penetrate the cell wall and cell membrane. U.S. Pat. No. 5,302,523 to Coffee et al. describes the use of 30.times.0.5 µm and 10.times.0.3 µm silicon carbide fibers to facilitate transformation of suspension maize cultures of Black Mexican Sweet. Any mechanical technique that can be used to introduce DNA for transformation of a plant cell using microfibers can be used to deliver gene repair oligonucleobases for transmutation.

An illustrative technique for microfiber delivery of a gene repair oligonucleobase is as follows: Sterile microfibers (2 µg) are suspended in 150 µL of plant culture medium containing about 10 µg of a mixed duplex oligonucleotide. A suspension culture is allowed to settle and equal volumes of packed cells and the sterile fiber/nucleotide suspension are vortexed for 10 minutes and plated. Selective media are applied immediately or with a delay of up to about 120 h as is appropriate for the particular trait.

### Protoplast Electroporation

In an alternative embodiment, the gene repair oligonucleobases can be delivered to the plant cell by electroporation of a protoplast derived from a plant part. The protoplasts are formed by enzymatic treatment of a plant part, particularly a leaf, according to techniques well known to those skilled in the art. See, e.g., Gallois et al., 1996, in Methods in Molecular Biology 55:89-107, Humana Press, Totowa, N.J.; Kipp et al., 1999, in Methods in Molecular Biology 133:213-221, Humana Press, Totowa, N.J. The protoplasts need not be cultured in growth media prior to electroporation. Illustrative conditions for electroporation are 3.times.10.sup.5 protoplasts in a total volume of 0.3 mL with a concentration of gene repair oligonucleobase of between 0.6-4 µg/mL.

### Protoplast PEG-mediated DNA uptake

In an alternative embodiment, nucleic acids are taken up by plant protoplasts in the presence of the membrane-modifying agent polyethylene glycol, according to techniques well known to those skilled in the art (see, e.g., Gharti-Chhetri *et al.,* 1992; Datta *et al.,* 1992).

### Microinjection

In an alternative embodiment, the gene repair oligonucleobases can be delivered by injecting it with a microcapillary into plant cells or into protoplasts (see, e.g., *Miki et al.,* 1989; Schnorf *et al.,* 1991).

### Selection of Herbicide Resistant Plants and Application of Herbicide

Plants and plant cells can be tested for resistance or tolerance to an herbicide using commonly known methods in the art, e.g., by growing the plant or plant cell in the presence of an herbicide and measuring the rate of growth as compared to the growth rate in the absence of the herbicide.

As used herein, substantially normal growth of a plant, plant organ, plant tissue or plant cell is defined as a growth rate or rate of cell division of the plant, plant organ, plant tissue, or plant cell that is at least 35%, at least 50%, at least 60%, or at least 75% of the growth rate or rate of cell division in a corresponding plant, plant organ, plant tissue or plant cell expressing the wild-type AHAS protein.

As used herein, substantially normal development of a plant, plant organ, plant tissue or plant cell is defined as the occurrence of one or more development events in the plant, plant organ, plant tissue or plant cell that are substantially the same as those occurring in a corresponding plant, plant organ, plant tissue or plant cell expressing the wild-type AHAS protein.

In certain embodiments plant organs provided herein include, but are not limited to, leaves, stems, roots, vegetative buds, floral buds, meristems, embryos, cotyledons, endosperm, sepals, petals, pistils, carpels, stamens, anthers, microspores, pollen, pollen tubes, ovules, ovaries and fruits, or sections, slices or discs taken therefrom. Plant tissues include, but are not limited to, callus tissues, ground tissues, vascular tissues, storage tissues, meristematic tissues, leaf tissues, shoot tissues, root tissues, gall tissues, plant tumor tissues, and reproductive tissues. Plant cells include, but are not limited to, isolated cells with cell walls, variously sized aggregates thereof, and protoplasts.

Plants are substantially "tolerant" to a relevant herbicide when they are subjected to it and provide a dose/response curve which is shifted to the right when compared with that provided by similarly subjected non-tolerant like plant. Such dose/response curves have "dose" plotted on the X-axis and "percentage kill", "herbicidal effect", etc., plotted on the y-axis. Tolerant plants will require more herbicide than non-tolerant like plants in order to produce a given herbicidal effect. Plants that are substantially "resistant" to the herbicide exhibit few, if any, necrotic, lytic, chlorotic or other lesions, when subjected to herbicide at concentrations and rates which are typically employed by the agrochemical community to kill weeds in the field. Plants which are resistant to an herbicide are also tolerant of the herbicide.

### EXAMPLES

Following are examples, which illustrate procedures for practicing the invention. These examples should not be construed as limiting. All percentages are by weight and all solvent mixture proportions are by volume unless otherwise noted.

### Example 1: Preparation of Herbicide-Resistant Brassica Samples

Unless otherwise specified, as used herein, numbering of the gene(s) is based on the amino acid sequence of the *Arabidopsis* acetolactate synthase (ALS) or acetohydroxyacid synthase (AHAS) At3g48560 (SEQ ID NO:1). In laboratory notebook references prior to October 2005, the S653 position (based on the *Arabidopsis* amino acid sequence) is referred to as S621 based on the corn amino acid sequences ZmAHAS108 and ZmAHAS109 (Fang *et al.,* 1992).

One goal was to make the imazethapyr (Imi) resistant amino acid substitution S653N in either or both BnAHAS I and III of spring Canola (*Brassica napus,* Spring Oilseed Rape-designated BN-2) and Winter Oilseed Rape (WOSR, also *Brassica napus -* designated BN-11).

In order to amplify the target regions of BnAHAS I and III from *Brassica napus* (initially elite Canola line BN-2), the oligo pair BnALS1 and BnALS2 was designed (SEQ ID NOs: 9 and 10). Since BnAHAS I and III do not contain introns, BnALS1 and BnALS2 amplify the target regions of 284 bp from both genomic DNA and cDNA flanking the S653 site. This primer pair was also designed to enable amplification of the ALS target region from *Arabidopsis*. The primers were received and resuspended in sterile water.

Initially the BnALS1/BnALS2 primer pair was used to PCR amplify the BnAHAS I and III C-terminal target regions from BN-2. Later, the ALS target regions were amplified from additional BN-2 samples and cloned into pGEM-T easy. Cloned inserts from 12 colonies from each of 3 genomic DNA and cDNA samples grown in overnight cultures were plasmid prepped and sequenced, confirming the target sequence. Glycerol stocks were prepared for BN-2 ALS 2c-21 as the BnAHAS I representative and BN-2 ALS YB10-2g-14 as the BnAHAS III representative for these 284 bp target regions.

The genomic and cDNA target region BnALS sequences from line BN-2 were analyzed and PCR errors recorded. Based on the sequence of BnAHAS I and III from BN-2 a single GRON (Gene Repair Oligonucleotide) BnALS1621/C/41/5'Cy3/3'idC (SEQ ID NO: 5) was designed to make a serine to asparagine amino acid substitution (AGT -> AAT) at position 653 (See Table 1). The initial syntheses were resuspended and their concentrations determined. The resuspended oligos were stored frozen at -70°C. The non-coding version of this GRON was called BnALS1621/NC/41/5'Cy3/3'idC (SEQ ID NO: 6). At a later date, the BnALS1621/NC was compared with all Genbank sequences where the only sequences with >16 nucleotides of 41 hybridizing were BnAHAS I and III and where the oligo had the single mismatch G->A designed to introduce the S653N amino acid substitution.

**TABLE 1. GRON SEQUENCES**

| GRON | Sequence |
|---|---|
| BnALS1621/C/41/5'Cy3/3'idC | VTGTGTTACCGATGATCCCAAATGGTGGCACTTTC AAAGATGH (SEQ ID NO: 5) |
| BnALS1621/NC/41/5'Cy3/3'idC | VCATCTTTGAAAGTGCCACCATTTGGGATCATCGG TAACACAH (SEQ ID NO: 6) |
| BnALS1574/C/41/5'Cy3/3'idC | VCTTGGGATGGTCATGCAATTGGAAGATCGGTTCT ACAAAGCH (SEQ ID NO: 7) |
| BnALS1574/NC/41/5'Cy3/3'idC | VGCTTTGTAGAACCGATCTTCCAATTGCATGACCA TCCCAAGH (SEQ ID NO: 8) |

| | |
|---|---|
| The converting base is shown in bold. V=CY3; H=3'DMT dC CPG | |

BnALS target regions, from a Cibus elite Winter Oil Seed Rape (WOSR) line BN-11, were amplified from genomic DNA and cDNA of single plants known to represent line BN-11. The BN-11 BnALS sequences were analyzed and PCR errors recorded.

In addition to characterizing the BnALS target regions from BN-2 and BN-11, the same target regions were also characterized in the commercial variety Clearfield Canola (BN-15). The Clearfield BnAHAS sequences were analyzed and PCR errors recorded showing the expected amino acid changes, S653N (AGT -> AAT) in BnAHAS I and W574L (TGG -> TTG) in BnAHAS III. Ultimately full coding sequences for BnAHAS I and III were amplified using BnALS3/BnALSOR2 (SEQ ID NOs: 11 and 12) and BnALS3/BnALSOR3 (SEQ ID NOs: 11 and 13) respectively, cloned and sequenced from lines BN-2, BN-11 and BN-15 to serve as reference sequences for comparative purposes

Imi resistant BN-2 (Canola) callus samples BnALS1621N-44 to 53, arising from various treatments, were extracted using the Edwards *et al.* (1991) method. Genomic DNA from this material was screened using an allele specific PCR (AS-PCR) mix (MM#23 composed of oligos BnALSOF1, BnALSOR2, BnALSOR3 and BnALSIR1A) (SEQ ID NOs: 14, 12, 13, and 15, respectively) to specifically detect the S653N change and allele specific PCR mix (MM#29 composed of oligos BnALSOF2, BnALSOR4 and BnALSIF1T) (SEQ ID NOs: 16, 17, and 18, respectively) to specifically detect the W574L change in either BnAHAS I or III. These initial AS-PCR results were inconclusive, therefore, additional samples BnALS1621N-54 to 58 for most of samples BnALS1621N-44 to 53 were received, and extracted using the Edwards *et al.* (1991) method with additional purification steps to obtain more pure genomic DNA.

At this point, samples BnALS1621N-54 to 58 were amplified with a BnALSOF2, BnALSOR2 and BnALSOR3 primer combination, and a small amount of amplified product was obtained. These products were cloned into pGEM-T easy and 12 white colonies per line setup as overnight cultures, plasmid prepped (using the Qiagen plasmid miniprep kit), and sequenced using BigDye 3.1 sequencing chemistry.

Preliminary sequence analysis determined that BnALS1621N-55-13 had an S653N mutation in BnAHAS III (AGT -> AAT) and two other clones from this line, BnALS1621N-55-15 and 19, were wildtype in BnAHAS III. Full sequence analysis was recorded. To confirm that this single positive did not result from a PCR error, an additional 38 colonies from this line were screened by AS-PCR. The strongest 8 positives from this AS-PCR screen BnALS1621N-55-1 to 8 were setup as overnight cultures, and plasmid DNA was isolated and sequenced. Seven of the 8 positive colonies, BnALS1621N-55-2 to 8 by AS-PCR were positive by sequencing for the S653N mutation in BnAHAS III; the other BnALS1621N-55-1 was a wildtype BnAHAS I sequence. Together these results indicated that line BnALS1621N-55 is heterozygous for the S653N mutation in BnAHAS III.

Sample BnALS1621N-55 was a parallel callus sample from the same initial Imi resistant callus as BnALS1621N-49. Genomic DNA from these samples was amplified with the BnALSOF2, BnALSOR2 and BnALSOR3 primer combination, and the fragments cloned into pGEM-T easy and transformed. MM#23 was used to screen 38 white colonies for the S653N mutation, with 4 positive colonies being identified (BnALS1621N-49-9 to 12). These colonies were sequenced. Three of the four colonies (BnALS1621N-49-9, 10 and 12) had the S653N mutation in BnAHAS III.

A similar method of target region cloning followed by AS-PCR with sequence confirmation was used to identify other lines with the S653N polymorphism. Among these was line BnALS-97. BnALS-97 was regenerated into a plant and allowed to set seed. Since this line was a prototype (as a plant), the full coding sequences of both BnAHAS I and III were cloned and sequenced. In this line, the only polymorphism compared to the BN-2 wildtype sequence was the AGT -> AAT codon change which results in the S653N amino acid substitution in BnAHAS III.

Preliminary sequence analysis of random cloned BnALSOF2/BnALSOR2 and BnALSOF2/BnALSOR3 amplicons from line BnALS1621N-57 indicated that both clones BnALS1621N-57-43 and 46 had the W574L mutation (TGG -> TTG) in BnAHAS I. Full sequence analysis was recorded, and indicated line 57 is heterozygous, since clones 38 and 41 were W574. Therefore, the BnALSOF2, BnALSOR2 and BnALSOR3 primer combination was used to amplify this fragment from BnALS1621N-57 and BnALS1621N-45, a parallel callus sample from the same initial Imi resistant callus as BnALS1621N-57, ligated into pGEM-T easy, transformed and plated with blue/white selection.

MM#29 was used to screen 19 white colonies for the W574L from each of the plates for BnALS1621N-57 and BnALS1621N-45, with 4 positive colonies for each (BnALS1621N-45-1, 2, 9 and 18, and BnALS1621N-57-1, 7, 13 and 16) being plasmid prepped and sequenced. Sequence analysis showed that all 8 colonies had the W574L mutation in BnAHAS I. The annealing temperature for MM#29 was optimized, and an additional 19 colonies screened using these new conditions. Three positive colonies (BnALS1621N-57-17, 18 and 19) were plasmid prepped and sequenced.

Additional Imi resistant Canola samples BnALS-68 to 91 were received and genomic DNA extracted using the Edwards *et al.* (1991) method. From each sample, the BnALSOF2, BnALSOR2 and BnALSOR3 primer combinations were used to amplify fragments of BnAHAS I and III and clone them into pGEM-T easy.

Using MM#23 in AS-PCR reactions to detect the S653N mutation, 12 colonies per line were screened, and results showed 2 positives from line BnALS-81 (BnALS-81-203 and 208) and 4 from BnALS-76 (BnALS-76-153, 154, 156 and 162) to contain the S653N mutation in BnAHAS III by sequencing. Full sequence analysis was recorded. By AS-PCR screening with MM#23, both PCR-amplified target regions or of bacterial colonies with single cloned inserts, line BnALS-159 (molecular biology sample 102; colony 655) was identified as having the S653N mutation in BnAHAS I, Cibus' initial S653N event in this gene.

Finally, in line BnALS-83 (molecular biology sample 91), wildtype (colony 408) and mutant (colonies 403, 405 and 406) were identified to indicate this line was heterozygous for the S653T mutation (AGT -> ACT) in BnAHAS I. As plants, line BnALS-83 was confirmed to be heterozygous in BnAHAS I for the S653T mutation. Further screening with MM#23 (S653N) and MM#29 (W574L) of new Imi resistance BN-2 Canola lines BnALS-76 and BnALS-123 was performed. Since the aforementioned expected mutations were not found in these samples, 12 BnALSOF2/OR2/3 were cloned and sequenced. All 7 clones of BnAHAS III for BnALS-123 (Colonies 5, 17-19, 21, 22, 26 and 28) were positive for the S653T mutation. However, seed from the R1 plants was genotyped as heterozygous.

Additionally, N-terminal PCR fragments of each of BnAHAS I and III were cloned and sequenced for BnALS-58, 68 and 69 using BnALS3/BnALS8 (SEQ ID NOs: 11 and 19 respectively) and BnALS3/BnALS9 (SEQ ID NOs: 11 and 20, respectively) oligo combinations respectively. A new tissue sample for BnALS- 96 was obtained and N-terminal PCR fragments of BnAHAS I and III were cloned and sequenced to show line BnALS-96 had an A205V mutation (GCC -> GTC) in BnAHAS I. All four clones of the full length coding sequence BnALS3/OR2 amplicon from plant material derived from callus line BnALS-68 had an A205V change (GCG -> GTG) in BnAHAS III, identical to the change in BnALS-69.

Lines determined to have a mutation, the experiment they were derived from and the treatment provided are summarized in Table 2. Line BnALS-159 died as a callus line and did not regenerate shoots. Table 3 shows exemplary oligos used in the experiments disclosed herein.

**TABLE 2. MUTATIONS IN IMI RESISTANT TISSUE SAMPLES OF BN CANOLA.**

| Line (CS#) | Mutation | SNP | Gene | Line |
|---|---|---|---|---|
| BnALS-96 | A205V | GCC → GTC | I | BN2 |
| BnALS-68 | A205V | GCG → GTG | III | BN2 |
| BnALS-69 | A205V | GCG → GTG | III | BN2 |
| BnALS-58 | A205D | GCC → GAC | I | BN2 |
| BnALS-63 | W574C | TGG → TGT | III | BN2 |
| BnALS-57 | W574L | TGG → TTG | I | BN2 |
| BnALS-67 | W574L | TGG → TTG | I | BN2 |
| BN02-204-A01 | W574L;R577W | TGG → TTG; CGG → TGG | III | BN2 |
| BN02-224-C01 | W574L (HET) | TGG → TTG | III | BN2 |
| BN02-224-B01 | W574M (HET) | TGG → ATG | III | BN2 |
| BnALS-76 | W574S | TGG → TCG | III | BN2 |
| BnALS-159* | S653N | AGT → AAT | I | BN2 |
| BnALS-55 | S653N | AGT → AAT | III | BN2 |
| BnALS-97 | S653N | AGT → AAT | III | BN2 |
| BnALS-61 | S653N | AGT → AAT | III | BN2 |
| BnALS-84 | S653N | AGT → AAT | III | BN2 |
| BnALS-83 | S653T | AGT → ACT | I | BN2 |
| BnALS-123 | S653T | AGT → ACT | III | BN2 |
| BN02-139-E07 | W574C(het);S653N | TGG → TGC; AGT → AAT | III | BN2 |
| BN02-139-D05 | A205V;S653N | GCG → GTG;AGT → AAT | III | BN2 |
| BN02-139-F08 | A205V;S653N | GCG → GTG;AGT → AAT | III | BN2 |
| BN02-139-C03 | A205V(het);S653N | GCC → GTC;AGT → AAT | I;III | BN2 |
| BN02-139-D06 | W574C;S653N | TGG → TGC;AGT → AAT | I;III | BN2 |
| BN02-139-E10 | W574L;S653N | TGG → TTG;AGT → AAT | I;III | BN2 |
| BN02-139-A13 | W574L(het);S653N | TGG → TTG;AGT → AAT | III | BN2 |
| BN02-139-A12 | D376E;S653N | GAC → GAG;AGT → AAT | III | BN2 |
| BN02-139-F09 | A122V;S653N | GCT→GTT;AGT → AAT | I;III | BN2 |
| BN02-139-A01 | A205D, S653N | GCG → GAC;AGT → AAT | I;III | BN2 |
| BN02-139-D-04 | W574C; S653N | TGG → TGC;AGT → AAT | I;III | BN2 |
| BN02-139-B11 | W574C; S653N | TGG → TGC;AGT → AAT | I;III | BN2 |

**TABLE 3: EXEMPLARY OLIGOS**

| Name | Length | Oligo Sequence |
|---|---|---|
| BnALS1 (SEQ ID NO: 9) | 24 | ATGCAATGGGAAGATCGGTTCTAC |
| BnALS2 (SEQ ID NO: 10) | 29 | CCATCYCCTTCKGTTATKACATCKTTGAA |
| BnALS3 (SEQ ID NO: 11) | 20 | CTAACCATGGCGGCGGCAAC |
| BnALSOR2 (SEQ ID NO: 12) | 28 | AGTCTGGGAACAAACCAAAAGCAGTACA |
| BnALSOR3 (SEQ ID NO: 13) | 28 | CGTCTGGGAACAACCAAAAGTAGTACAA |
| BnALSOF1 (SEQ ID NO: 14) | 28 | AGTGACGAAGAAAGAAGAACTCCGAGAA |
| BnALSIR1A (SEQ ID NO: 15) | 30 | CTGTTATTACATCTTTGAAAGTGCCACAAT |
| BnALSOF2 (SEQ ID NO: 16) | 28 | TGACGGTGATGGAAGCTTCATAATGAAC |
| BnALSOR4 (SEQ ID NO: 17) | 28 | GTCCWGGTGTATCCAGCATTGTCTGAAT |
| BnALSIF1T (SEQ ID NO: 18) | 26 | CAGCATCTTGGGATGGTCATGCAGTT |
| BnALS8 (SEQ ID NO: 19) | 21 | CCCATCAAAGTACTCGCACCG |
| BnALS9 (SEQ ID NO: 20) | 21 | CCCATCAACGTACTCGCACCA |

As shoots become available, crosses are being made in all permutations and combinations. Line BnALS-97 is the reference S653N in BnAHAS III. Line BnALS-57 is the reference W574L in BnAHAS I. Both BnALS-68 and 69 are being advanced as reference lines for A205V in BnAHAS III. Line BnALS-83 is the first line that was heterozygous for S653T in BnAHAS I as callus to also be heterozygous as a plant.

### Example 2: Materials and Methods

**Cell Culture Work Description.** Shoots derived both from seeds and from microspore-derived embryos were propagated under sterile conditions *in vitro*. Cuttings were subcultured every 2 - 4 weeks and cultured in petri dishes (25 mm x 90 mm) in a volume of 40 - 45 mL RS medium (Dovzhenko, 2001). The dishes were sealed with Micropore tape (3M Company). Young leaves were used for protoplast isolation.

**Protoplast isolation and purification.** About 600 mg of leaf tissue of 2 - 3 week-old *in vitro* shoots were cut into small strips with a scalpel in a petri dish with 5 mL medium B (Pelletier *et al.,* 1983), pH adjusted to 5.8. After approximately 1 h, medium B was replaced with enzyme solution, consisting of medium B in which 0.5% (w/v) Cellulase YC and 0.75% (w/v) Macerozyme R10 (both from Karlan Research Products, Cottonwood, Arizona), 1 g/L bovine serum albumin, and 1 g/L 2-morpholinoethanesulfonic acid were dissolved. The enzyme solution was vacuum-infiltrated into the leaf tissue, and the dish with leaf pieces in enzyme solution was incubated for at 25°C in darkness. Protoplast purification was performed using an iodixanol density gradient (adapted from Optiprep Application Sheet C18; Purification of Intact Plant Protoplasts; Axis-Shield USA, 10 Commerce Way, Norton, MA 02776). After the density gradient centrifugation, the band with purified protoplasts was removed together with about 5 mL W5 medium (Frigerio *et al.,* 1998). The protoplast yield was determined with a hemocytometer, and the protoplasts were stored for 2 h at 4°C.

**Gene Repair Oligonucleotide GRON introduction.** The protoplast suspension was mixed with an equal volume of W5 medium, transferred to a 50 mL centrifuge tube, and centrifuged for 5 min at the lowest setting of a clinical centrifuge (about 50 x g). The supernatant was removed and replaced with TM medium (Klaus, 2001), adjusting the protoplast density to 5 x 10⁶/mL. Aliquots of 100 µL containing 5 x 10⁶ protoplasts each were distributed into 12 mL round bottom centrifuge tubes. GRONs targeted at a mutation in one or both AHAS genes were then introduced into the protoplasts using a PEG treatment. To introduce the GRONs into the protoplasts, 12.5 µg GRON dissolved in 25 µL purified water and 125 µL of a polyethylene glycol solution (5 g PEG MW 1500, 638 mg mannitol, 207 mg CaNO₃ x 4H₂O and 8.75 mL purified water; pH adjusted to about 9.0) was added. After a 30 min incubation on ice, the protoplast-PEG suspension was washed with W5 medium and resuspended in medium B. The suspension was kept overnight in a refrigerator at about 4°C.

**Embedding of protoplasts in calcium alginate.** One day after the GRON introduction, protoplasts were embedded in calcium alginate. The embedding of protoplasts in gel substrates (e.g., agarose, alginate) has been shown to enhance protoplast survival and to increase division frequencies of protoplast-derived cells. The method applied was based on that described in Dovzhenko (2001).

**Protoplast culture and selection of imazethapyr-resistant calli.** The selection of imazethapyr-resistant calli was carried out using sequential subcultures of the alginates in media according to Pelletier *et al.* (1983). Selection was started one week after the PEG/GRON treatment at a concentration of 0.5 µM imazethapyr. The herbicide did not have an immediate effect. Initially, all microcolonies that had formed during the early culture phase without imazethapyr continued to grow, but slower than controls without added herbicide. One to two weeks after the onset of selection, the colonies slowed down in growth or stopped growing.

Before the end of the selection phase in liquid medium, cells and colonies were released from the alginate by treating them for 30 - 45 min with culture medium containing 50 mM sodium citrate. At the moment of transferring released colonies from liquid to solid medium, the majority of colonies were either dead, or consisted of a greenish center, covered with outer layers of dead cells. On the solidified selection medium E the majority of microcalli that still contained living cells stopped growing and turned brownish. Limited growth of individual calli continued occasionally, but all non-resistant calli eventually turned brown and died. Two to three weeks after the transfer to solidified selection medium (occasionally earlier), actively growing calli appeared among a background of brownish cells and microcalli.

Regeneration of plants from protoplast-derived, herbicide-tolerant calli with a confirmed mutation in an AHAS gene. Imi-tolerant calli that had developed on solidified selection medium and whose DNA upon analysis had shown the presence of a mutation were transferred to herbicide-free medium E (Pelletier *et al.,* 1983) to accelerate development. Individual callus lines varied in their growth rates and morphologies. In general, the development towards shoot regeneration followed these steps: Undifferentiated, green callus -> callus with dark green areas -> development of roots -> development of shoot initials -> development of stunted shoots with hyperhydric (vitrified) leaves.

The development of individual callus line was variable, but through continuous subculture and multiplication on medium E or modifications of medium E with lower concentrations of alpha-naphthalene acetic acid (NAA) eventually many callus lines produced shoots.

Once shoots with three to four leaves had formed on medium E, they were transferred to RS medium (Dovzhenko, 2001). On this medium, over time shoot and leaf tissue developed that was morphologically 'normal' (i.e., non-hyperhydric). After *in vitro* plantlets had produced roots, standard protocols were used for the adaptation to greenhouse conditions.

### Example 4: Herbicide Spray Data

*B. napus* plants at the 5-6 leaf stage were sprayed with various AHAS inhibiting herbicides. *B. napus* plants sprayed included the parental line BN02 (or BN11 as required), BN15 (Clearfield, a commercial two gene check) and mutants as detailed below. Herbicides were sprayed in the presence of 0.25% AU391 surfactant at the following rates:
Imazamox (Beyond™) 0, 2, 4, 6, 8, 12, 16, 32 and 48 oz active ingredient/Acre (ai/A)
Thifensulfuron 0.028, 0.056, 0.112, 0.168 lb ai/A
Tribenuron 0.015, 0.03, 0.06, 0.12, 0.18 lb ai/A
Nicosulfuron lb 0.06, 0.120, 0.24, 0.36 ai/A
Rimsulfuron 0.015, 0.03, 0.06, 0.12, 0.18 lb ai/A
2:1 weight:weight Thifensulfuron/Tribenuron 0.056, 0.112, 0.224, 0.336 lb ai/A
2.22:1 Thifensulfuron/Nicosulfuron 0.058, 0.116, 0.232 lb ai/A
Primisulfuron 0.035, 0.070, 0.140 lb ai/A
Flumetsulam0.040, 0.080, 0.16 lb ai/A
Cloransulam0.039, 0.078, 0.156 lb ai/A

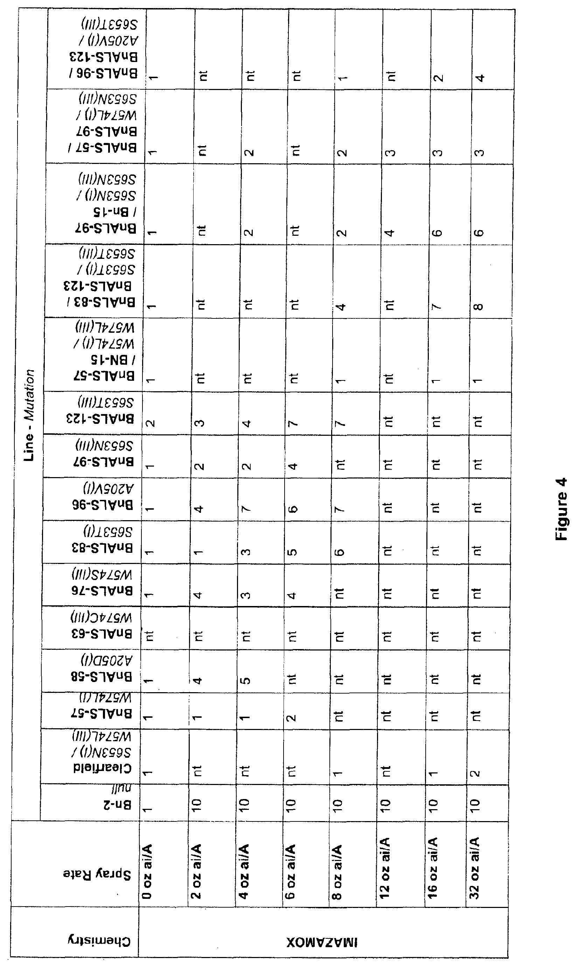

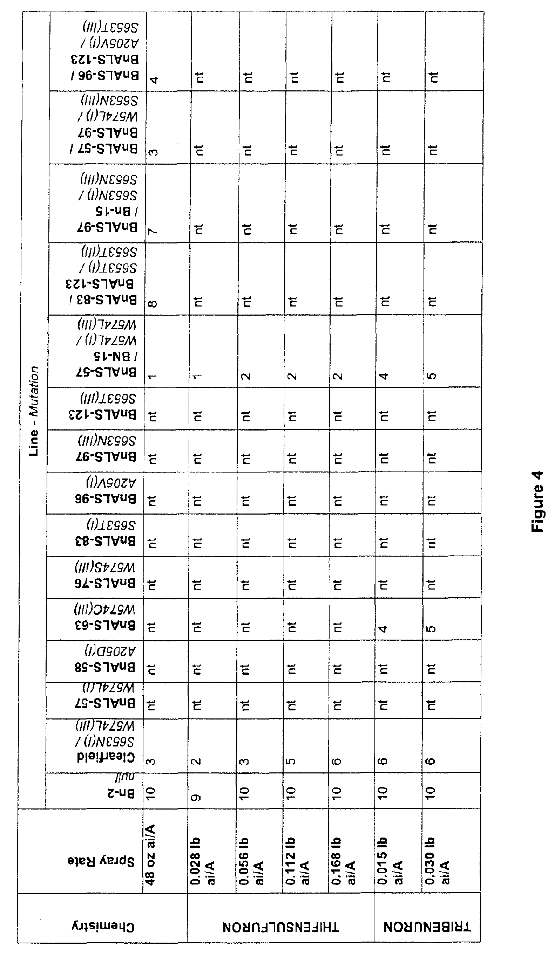

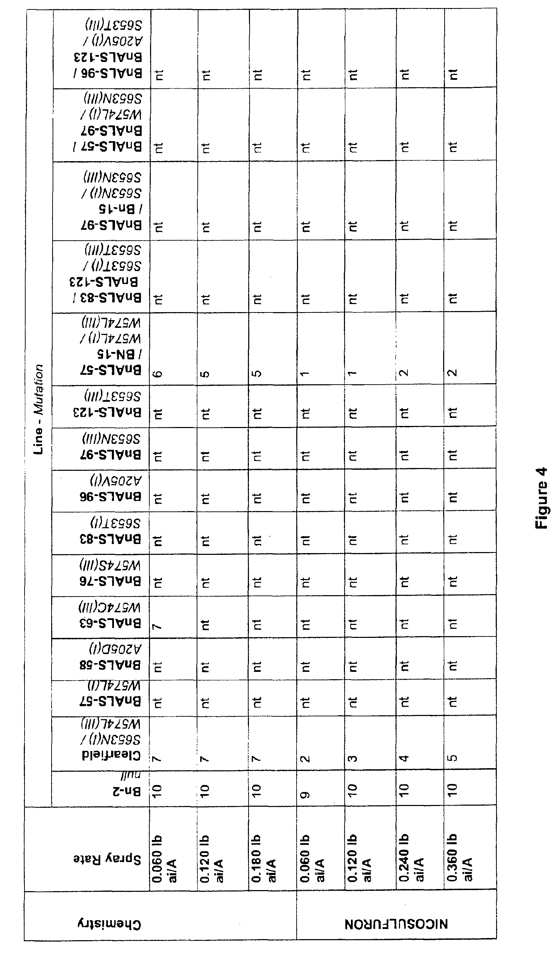

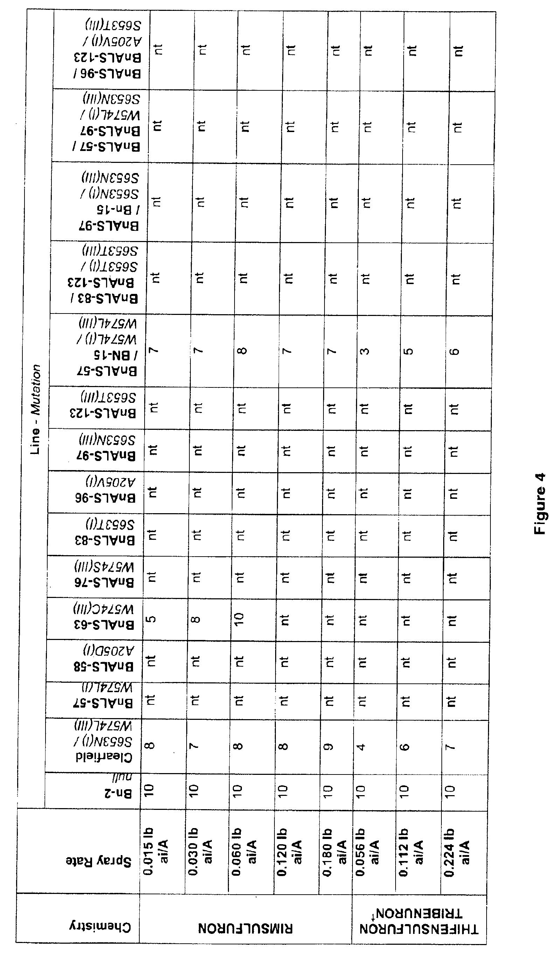

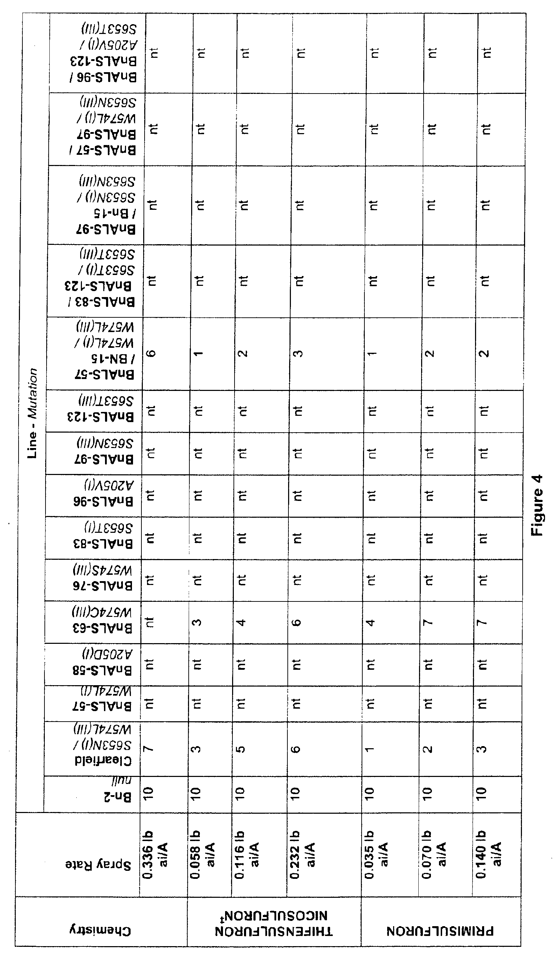

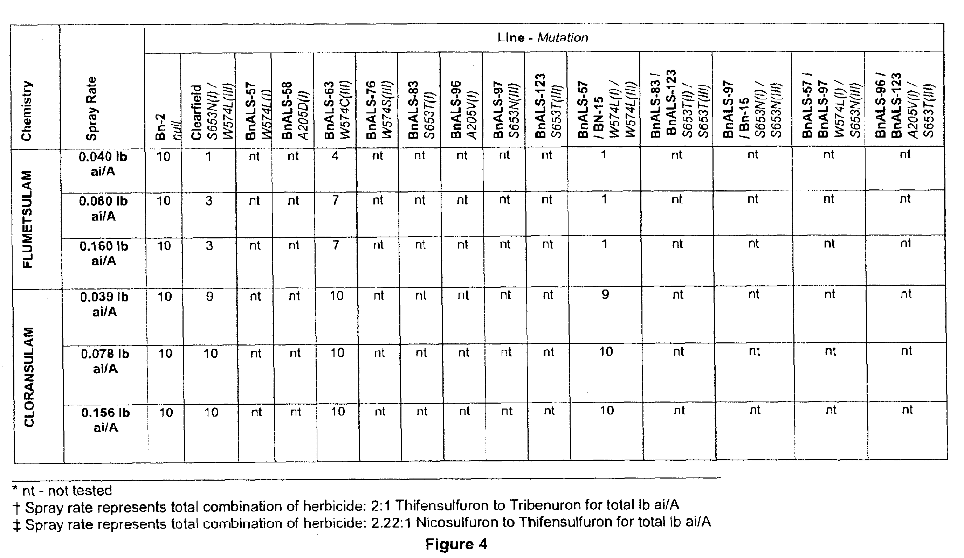

Herbicides were applied by foliar spray with control plants being left unsprayed. Excepting Imazamox, all AHAS inhibiting herbicide trials were evaluated 14 days post spraying using a damage scale of 1-10 with 1 being dead, and 10 being the undamaged unsprayed controls. Individual plant lines were scored at each spray rate compared to the performance of the controls at that particular rate. Results of the spray trial are presented in Figure 4.

Chemistry tested: Genotype (# of seedlings for all rates)
Thifensulfuron: BN2 (6), BN15 (6), BN15xBnALS-57 (18)
Tribenuron: BN2 (6), BN15 (6), BN15xBnALS-57 (18), 63 (9)
Nicosulfuron: BN2 (6), BN15 (6), BN15xBnALS-57 (18)
THI / TRI: BN2 (6), BN 15 (6), BN15xBnALS-57 (18)
Rimsulfuron: BN2 (6), BN15 (6), BN15xBnALS-57 (18), 63 (9)
THI / Nic: BN2 (18), 63 (9), BN15xBnALS-57 (36), BN15 (18)
Primisulfuron: BN2 (9), 63 (9), BN15 (9), BN15xBnALS-57 (18)
Flumetsulam: BN2 (9), 63 (9), BN 15 (9), BN15xBnALS-57 (18)
Cloransulam: BN2 (9), 63 (9), BN15 (9), BN15xBnALS-57 (18)

Trials with Imazamox were evaluated as follows:
10d score for: 8, 16, 32, and 48 oz/A of BnALS-83xBnALS-123, BnALS-96xBnALS-123, BnALS-97xBnALS-57, BN15xBnALS-57, BN2, BN 15, BnALS-97xBN15 17d score for: 4 and 12 oz/A of BnALS-97xBnALS-57, BN15xBnALS-97, BN2 28d score for: 2, 4, 6, 8 oz/A for all the single factor mutations.

### Imazamox:

2 oz/A: BN2 (18), BnALS-123 (9), BnALS-96 (9), BnALS-97 (18), BnALS-83 (6), BnALS-76 (18), BnALS-58 (9), BnALS-57 (12)
4 oz/A: BN2 (18), BnALS-123 (9), BnALS-96 (9), BnALS-97 (18), BnALS-83 (9), BnALS-76 (18), BnALS-58 (9), BnALS-57 (12), BnALS-97xBN15 (15), BnALS-97xBnALS-57 (14)
6 oz/A: BN2 (9), BnALS-123 (9), BnALS-96 (12), BnALS-97 (9), BnALS-83 (12), BnALS-76 (9), BnALS-57 (12)
8 oz/A: BnALS-123 (9), BnALS-96 (12), BnALS-83 (12), BnALS-83xBnALS-123 (18), BnALS-96xBnALS-123 (18), BN2 (18), BN 15 (18), BN15xBnALS-57 (15), BnALS-97xBnALS-57 (18), BN15xBnALS-97 (18)
12 oz/A: BnALS-97xBN 15 (15), BnALS-97xBnALS-57 (14), BN2 (12)
16 oz/A: BnALS-83xBnALS-123 (18), BnALS-96xBnALS-123 (18), BN2 (15), BN15 (18), BN15xBnALS-57 (18), BnALS-97xBnALS-57 (18), BN15xBnALS-97 (18)
32 oz/A: BnALS-83xBnALS-123 (18), BnALS-96xBnALS-123 (18), BN2 (13), BN15 (18), BN15xBnALS-57 (18), BnALS-97xBnALS-57 (18), BN15xBnALS-97 (18)
48 oz/A: BnALS-83xBnALS-123 (18), BnALS-96xBnALS-123 (18), BN2 (6), BN15 (18), BN15xBnALS-57 (18), BnALS-97xBnALS-57 (18), BN15xBnALS-97 (18)

### REFERENCES

Datta SK, Datta K, Soltanifar N, Donn G, Potrykus I (1992) Herbicide-resistant Indica rice plants from IRRI breeding line IR72 after PEG-mediated transformation of protoplasts. Plant Molec. Biol. 20:619-629
Dovzhenko A (2001) Towards plastid transformation in rapeseed (Brassica napus L.) and sugarbeet (Beta vulgaris L.). PhD Dissertation, LMU Munich, Faculty of Biology Edwards K, Johnstone C, Thompson C (1991) A simple and rapid method for the preparation of plant genomic DNA for PCR analysis. Nucleic Acids Res. 19:1349.
Frigerio L, Vitale A, Lord JM, Ceriotti A, Roberts LM (1998) Free ricin A chain, proricin, and native toxin have different cellular fates when expressed in tobacco protoplasts. J Biol Chem 273:14194-14199
Fang LY, Gross PR, Chen CH, Lillis M (1992) Sequence of two acetohydroxyacid synthase genes from Zea mays. Plant Mol Biol. 18(6):1185-7
Gharti-Chhetri GB, Cherdshewasart W, Dewulf J, Jacobs M, Negrutiu I (1992) Polyethylene glycol-mediated direct gene transfer in Nicotiana spp. Physiol. Plant. 85:345-351
Klaus S (2003) Markerfreie transplastome Tabakpflanzen (Marker-free transplastomic tobacco plants). PhD Dissertation, LMU Munich, Faculty of Biology
Miki B, Huang B, Bird S, Kemble R, Simmonds D, Keller W (1989) A procedure for the microinjection of plant cells and protoplasts. Meth. Cell Science 12:139-144
Pelletier G, Primard C, Vedel F, Chetrit P, Remy R, Rouselle P, Renard M (1983) Intergeneric cytoplasm hybridization in Cruciferae by protoplast fusion. Mol. Gen. Genet. 191: 244-250
Schnorf M, Neuhaus-Url G, Galli A, Iida S, Potrykus I, Neuhaus G (1991) An improved approach for transformation of plant cells by microinjection: molecular and genetic analysis. Transgen. Res. 1:23- 30
Tan S, Evans RR, Dahmer ML, Singh BK, Shaner DL (2005) Imidazolinone-tolerant crops: history, current status and future. Pest Manag Sci. 61(3):246-57.

Unless otherwise defined, all technical and scientific terms used herein have the same meaning as commonly understood by one of ordinary skill in the art to which this invention belongs.

The inventions illustratively described herein may suitably be practiced in the absence of any element or elements, limitation or limitations, not specifically disclosed herein. Thus, for example, the terms "comprising," "including," "containing," etc. shall be read expansively and without limitation. Additionally, the terms and expressions employed herein have been used as terms of description and not of limitation, and there is no intention in the use of such terms and expressions of excluding any equivalents of the features shown and described or portions thereof.

Thus, it should be understood that although the present invention has been specifically disclosed by preferred embodiments and optional features, modification, improvement, and variation of the inventions disclosed may be resorted to by those skilled in the art, and that such modifications, improvements and variations are considered to be within the scope of this invention. The materials, methods, and examples provided here are representative of preferred embodiments, are exemplary, and are not intended as limitations on the scope of the invention.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

## Claims

1. A method for producing an herbicide-resistant plant, said method comprising:
(a) introducing into a plant cell a single stranded gene repair oligonucleobase (GRON) with a targeted mutation in an acetohydroxyacid synthase (AHAS) gene to produce a plant cell with:
(i) an AHAS gene that expresses an AHAS protein comprising an alanine to valine or aspartic acid substitution at a position corresponding to position 205 of SEQ ID NO: 1, and
(ii) an AHAS gene that expresses an AHAS protein comprising a serine to asparagine substitution at a position corresponding to position 653 of SEQ ID NO: 1;
(b) identifying a plant cell having substantially normal growth and catalytic activity as compared to a corresponding wild-type plant cell in the presence of an AHAS-inhibiting herbicide; and
(c) regenerating a non-transgenic herbicide-resistant plant having a mutated AHAS gene from said plant cell.

2. The method of claim 1, wherein said AHAS-inhibiting herbicide is selected from the group consisting of herbicides of: imidazolinone, sulfonylurea, triazolopyrimidine, pyrimidinylthiobenzoate, sulfonylamino-carbonyltriazolinone, and mixtures thereof.

3. The method of claim 1 or 2, wherein said AHAS gene encodes a protein comprising 70% or more identity to one or more of the amino acid sequences of figure 2.

4. The method of claim 1, 2 or 3, wherein said herbicide-resistant plant is selected from the group consisting of sugar beet, oilseed rape, and canola; and wherein said herbicide is a sulfonylurea herbicide.

## Patentansprüche

1. Verfahren zum Erzeugen einer Herbizid-resistenten Pflanze, wobei das Verfahren umfasst:
(a) das Einbringen in eine Pflanzenzelle einer einzelsträngigen Gen-Reparatur-Oligonukleobase (GRON) mit einer zielgerichteten Mutation in einem Acetohydroxysäure-Synthasegen (AHAS-Gen), um eine Pflanzenzelle zu erzeugen mit:
(i) einem AHAS-Gen, welches ein AHAS-Protein exprimiert, umfassend eine Substitution von Alanin nach Valin oder Asparaginsäure an einer Position entsprechend Position 205 von SEQ ID NO: 1, und
(ii) einem AHAS-Gen, welches ein AHAS-Protein exprimiert, umfassend eine Substitution von Serin nach Asparagin an einer Position entsprechend Position 653 von SEQ ID NO: 1;
(b) das Identifizieren einer Pflanzenzelle mit im Wesentlichen normalem Wachstum und katalytischer Aktivität im Vergleich zu einer entsprechenden Wildtyp-Pflanzenzelle in der Gegenwart eines AHAS-inhibierenden Herbizids; und
(c) das Regenerieren einer nicht-transgenen Herbizid-resistenten Pflanze mit einem mutierten AHAS-Gen aus der Pflanzenzelle.

2. Verfahren nach Anspruch 1, wobei das AHAS-inhibierende Herbizid ausgewählt ist aus der Gruppe, bestehend aus den Herbiziden: Imidazolinon, Sulfonylharnstoff, Triazolopyrimidin, Pyrimidinylthiobenzoat, Sulfonylamino-carbonyltriazolinon, und Gemischen davon.

3. Verfahren nach Anspruch 1 oder 2, wobei das AHAS-Gen für ein Protein kodiert, welches eine Identität von 70% oder darüber zu einer oder mehreren der Aminosäuresequenzen von Figur 2 aufweist.

4. Verfahren nach Anspruch 1, 2 oder 3, wobei die Herbizid-resistente Pflanze ausgewählt ist aus der Gruppe, bestehend aus Zuckerrübe, Raps, und Canola; und wobei das Herbizid ein Sulfonylharnstoff-Herbizid ist.

## Revendications

1. Procédé de production d'une plante résistant aux herbicides, ledit procédé comprenant le fait de:
(a) introduire dans une cellule de plante une oligonucléobase de réparation de gènes à un seul brin (GRON) avec une mutation ciblée dans un gène d'acétohydroxyacide synthase (AHAS), pour produire une cellule de plante avec:
(i) un gène d'AHAS qui exprime une protéine d'AHAS comprenant une substitution d'alanine par valine ou acide aspartique en une position correspondant à la position 205 de SEQ ID NO: 1, et
(ii) un gène d'AHAS qui exprime une protéine d'AHAS comprenant une substitution de sérine par asparagine en une position correspondant à la position 653 de SEQ ID NO: 1;
(b) identifier une cellule de plante présentant une croissance et une activité catalytique sensiblement normales, comparé à une cellule de plante de type sauvage correspondante en présence d'un herbicide inhibant l'AHAS-; et
(c) régénérer une plante résistant aux herbicides non transgénique présentant un gène d'AHAS muté de ladite cellule de plante.

2. Procédé selon la revendication 1, dans lequel ledit herbicide inhibant l'AHAS est choisi parmi le groupe composé d'herbicides de: imidazolinone, sulfonylurée, triazolopyrimidine, pyrimidinylthio-benzoate, sulfonylamino-carbonyltriazolinone, et des mélanges de ceux-ci.

3. Procédé selon la revendication 1 ou 2, dans lequel ledit gène d'AHAS code une protéine comprenant 70% ou plus d'identité avec une ou plusieurs des séquences d'acides aminés de la figure 2.

4. Procédé selon la revendication 1, 2 ou 3, dans lequel ladite plante résistant à l'herbicide est choisie parmi le groupe composé de la betterave sucrière, du colza et du canola; et dans lequel ledit herbicide est un herbicide à base de sulfonylurée.
